# EUROPEAN PATENT SPECIFICATION

(11) **EP 2 102 215 B1**
(45) Date of publication and mention of the grant of the patent: **01.09.2010**
(21) Application number: 07857234.4
(22) Date of filing: 28.11.2007
(51) Int. Cl.: C07D 495/04, A61K 31/4365, A61P 3/04, A61P 3/10

(54) **4,5,6,7-TETRAHYDRO-THIENO [2,3-C]PYRIDINES AS H3 MODULATORS**
4,5,6,7-TETRAHYDROTHIENO[2,3-C]PYRIDINE ALS H3-MODULATOREN
4,5,6,7-TÉTRAHYDRO-THIÉNO [2,3-C]PYRIDINES EN TANT QUE MODULATEURS DE H3

(30) Priority: 08.12.2006 EP 06125715
(43) Date of publication of application: 23.09.2009
(73) Proprietor: F. Hoffmann-La Roche AG, 4070 Basel (CH)
(72) Inventor: CHEN, Li, Shanghai 201203 (CN); LAI, Wayne Wen, Shanghai 201203 (CN); NETTEKOVEN, Matthias, 79639 Grenzach-Wyhlen (DE); ROCHE, Olivier, 68220 Folgensbourg (FR); ZHENG, Deye, Shanghai 201204 (CN)
(74) Representative: Klostermeyer-Rauber, Dörte
(86) International application number: PCT/EP2007/062930
(87) International publication number: WO 2008/068174

(56) References cited:
- EP-A- 0 421 861
- STARK H: "Recent advances in histamine H3/H4 receptor ligands" EXPERT OPINION ON THERAPEUTIC PATENTS, ASHLEY PUBLICATIONS, GB, vol. 13, no. 6, 2003, pages 851-865, XP002353598 ISSN: 1354-3776
- BERLIN ET AL: "Recent advances in the development of histamine H3 antagonists" EXPERT OPINION ON THERAPEUTIC PATENTS, vol. 17, no. 6, June 2007 (2007-06), pages 675-687, XP002468038

## Description

The present invention is concerned with new 4,5,6,7-tetrahydro-thieno[2,3-c] pyridine derivatives, their manufacture, pharmaceutical compositions containing them and their use as medicaments. The active compounds of the present invention are useful in treating obesity and other disorders.

In particular, the present invention relates to compounds of the general formula 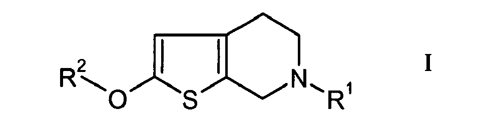 wherein
- R¹: is selected from the group consisting of
-CO-R³, wherein
R³ is selected from the group consisting of C₁₋₇-alkyl,
C₃₋₇-cycloalkyl-C₁₋₇-alkyl, wherein the cycloalkyl ring may be unsubstituted or substituted with one or two groups independently selected from C₁₋₇-alkyl; and phenyl, wherein the phenyl ring may be unsubstituted or substituted with one or two groups independently selected from C₁₋₇-alkyl, cyano, halogen, halogen-C₁₋₇-alkyl and C₁₋₇-alkoxy,
-CO-NR⁴R⁵, wherein
R⁴ and R⁵ independently from each other are selected from the group consisting of hydrogen, C₁₋₇-alkyl, C₁₋₇-alkoxy-C₁₋₇-alkyl, phenyl unsubstituted or substituted with one or two groups independently selected from C₁₋₇-alkyl, halogen, C₁₋₇-alkoxy and C₁₋₇-halogen-C₁₋₇-alkyl, and phenyl-C₁₋₇-alkyl, wherein phenyl is unsubstituted or substituted with one or two groups independently selected from C₁₋₇-alkyl, halogen, C₁₋₇-alkoxy and halogen-C₁₋₇-alkyl,
or R⁴ and R⁵ together with the nitrogen atom to which they are attached form a 4-, 5-, 6- or 7-membered heterocyclic ring optionally containing a further heteroatom selected from nitrogen, oxygen or sulfur, a sulfinyl group or a sulfonyl group, said heterocyclic ring being unsubstituted or substituted with one or two groups independently selected from C₁₋₇-alkyl, halogen, cyano, C₁₋₇-alkoxy and phenyl, wherein phenyl is unsubstituted or substituted with one or two groups independently selected from C₁₋₇-alkyl, halogen and C₁₋₇- alkoxy, and
-SO₂-R⁶, wherein
R⁶ is selected from the group consisting of C₁₋₇-alkyl, C₁₋₇-alkoxy-C₁₋₇-alkyl, cycloalkyl-C₁₋₇-alkyl, wherein the cycloalkyl ring may be unsubstituted or substituted with one or two groups independently selected from C₁₋₇-alkyl, halogen and C₁₋₇-alkoxy; and
phenyl, wherein the phenyl ring may be unsubstituted or substituted with one or two groups independently selected from C₁₋₇-alkyl, cyano, halogen, halogen-C₁₋₇-alkyl and C₁₋₇-alkoxy;
- R²: is a group selected from 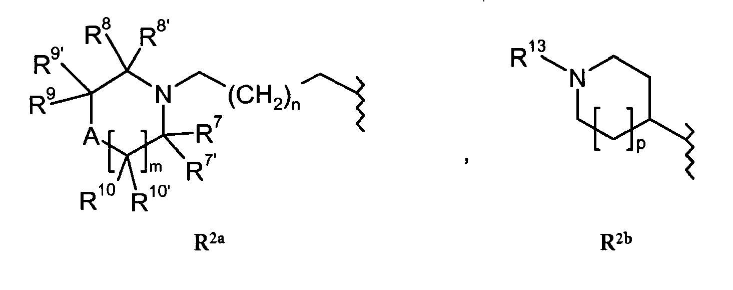 and 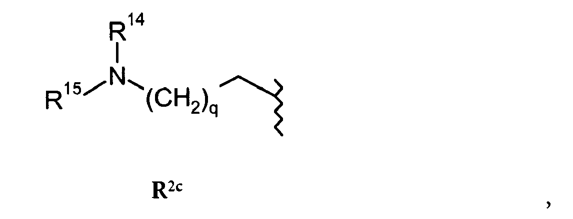 wherein
m is 0, 1 or 2;
n is 0, 1 or 2;
A is selected from -CR¹¹R^{11'}-, O, S or -NR¹²;
R⁷, R^{7'}, R⁸, R^{8'}, R⁹, R^{9'}, R¹⁰, R^{10'}, R¹¹ and R^{11'} independently from each other are hydrogen or C₁₋₇-alkyl;
R¹² is hydrogen or C₁₋₇-alkyl;
p is 0, 1 or 2;
R¹³ is C₁₋₇-alkyl or C₃₋₇-cycloalkyl;
q is 1, 2 or 3; and
R¹⁴ and R¹⁵ independently from each other are C₁₋₇-alkyl;
and pharmaceutically acceptable salts thereof.

The compounds of formula I are antagonists and/or inverse agonists at the histamine 3 receptor (H3 receptor).

Histamine (2-(4-imidazolyl) ethylamine) is one of the aminergic neurotransmitters which is widely distributed throughout the body, e. g. the gastrointestinal tract (Burks 1994 in Johnson L.R. ed., Physiology of the Gastrointestinal Tract, Raven Press, NY, pp. 211- 242). Histamine regulates a variety of digestive pathophysiological events like gastric acid secretion, intestinal motility (Leurs et al., Br J. Pharmacol. 1991, 102, pp 179-185), vasomotor responses, intestinal inflammatory responses and allergic reactions (Raithel et al., Int. Arch. Allergy Immunol. 1995, 108, 127-133). In the mammalian brain, histamine is synthesized in histaminergic cell bodies which are found centrally in the tuberomammillary nucleus of the posterior basal hypothalamus. From there, the histaminergic cell bodies project to various brain regions (Panula et al., Proc. Natl. Acad. Sci. USA 1984, 81, 2572-2576; Inagaki et al., J. Comp. Neurol 1988, 273, 283 - 300).

According to current knowledge, histamine mediates all its actions in both the CNS and the periphery through four distinct histamine receptors, the histamine H1, H2 H3 and H4 receptors.

H3 receptors are predominantly localized in the central nervous system (CNS). As an autoreceptor H3 receptors constitutively inhibit the synthesis and secretion of histamine from histaminergic neurons (Arrang et al., Nature 1983, 302, 832-837; Arrang et al., Neuroscience 1987, 23, 149-157). As heteroreceptors, H3 receptors also modulate the release of other neurotransmitters such as acetylcholine, dopamine, serotonin and norepinephrine among others in both the central nervous system and in peripheral organs, such as lungs, cardiovascular system and gastrointestinal tract (Clapham & Kilpatrik, Br. J. Pharmacol. 1982, 107, 919- 923; Blandina et al. in The Histamine H3 Receptor (Leurs RL and Timmermann H eds, 1998, pp 27-40, Elsevier, Amsterdam, The Netherlands). H3 receptors are constitutively active, meaning that even without exogenous histamine, the receptor is tonically activated. In the case of an inhibitory receptor such as the H3 receptor, this inherent activity causes tonic inhibition of neurotransmitter release. Therefore it may be important that a H3R antagonist would also have inverse agonist activity to both block exogenous histamine effects and to shift the receptor from its constitutively active (inhibitory) form to a neutral state.

The wide distribution of H3 receptors in the mammalian CNS indicates the physiological role of this receptor. Therefore the therapeutic potential as a novel drug development target in various indications has been proposed.

The administration of H3R ligands - as antagonists, inverse agonists, agonists or partial agonists - may influence the histamine levels or the secretion of neurotransmitters in the brain and the periphery and thus may be useful in the treatment of several disorders. Such disorders include obesity, (Masaki et al; Endocrinol. 2003, 144, 2741-2748; Hancock et al., European J. of Pharmacol. 2004, 487, 183-197), cardiovascular disorders such as acute myocardial infarction, dementia and cognitive disorders such as attention deficit hyperactivity disorder (ADHD) and Alzheimer's disease, neurological disorders such as schizophrenia, depression, epilepsy, Parkinson's disease, and seizures or convulsions, sleep disorders, narcolepsy, pain, gastrointestinal disorders, vestibular dysfunction such as Morbus Meniere, drug abuse and motion sickness (Timmermann, J. Med. Chem. 1990, 33, 4-11).

It is therefore an object of the present invention to provide selective, directly acting H3 receptor antagonists respectively inverse agonists. Such antagonists / inverse agonists are useful as therapeutically active substances, particularly in the treatment and/or prevention of diseases which are associated with the modulation of H3 receptors.

EP 0 421 861 Al refers to the preparation of tetrahydrothienopyridines as elastase and platelet aggregation inhibitors. Stark, Expert Opinon on Therapeutic Patents 2003, Vol. 13(6), 851-865, reports on recent advances in histamine H3/H4 receptor ligands. Berlin et al., Expert Opinon on Therapeutic Patents 2007, Vol. 17(6), 675-687, describes recent advances in the development of histamine H3 antagonists.

In the present description the term "alkyl", alone or in combination with other groups, refers to a branched or straight-chain monovalent saturated aliphatic hydrocarbon radical of one to twenty carbon atoms, preferably one to sixteen carbon atoms, more preferably one to ten carbon atoms.

The term "lower alkyl" or "C₁-C₇-alkyl", alone or in combination, signifies a straight-chain or branched-chain alkyl group with 1 to 7 carbon atoms, preferably a straight or branched-chain alkyl group with 1 to 6 carbon atoms and particularly preferred a straight or branched-chain alkyl group with 1 to 4 carbon atoms Examples of straight-chain and branched C₁-C₇ alkyl groups are methyl, ethyl, propyl, isopropyl, butyl, isobutyl, tert.-butyl, the isomeric pentyls, the isomeric hexyls, the isomeric heptyls and the isomeric octyls, preferably methyl and ethyl and most preferred methyl.

The term "cycloalkyl" or "C₃₋₇-cycloalkyl" denotes a saturated carbocyclic group containing from 3 to 7 carbon atoms, such as cyclopropyl, cyclobutyl, cyclopentyl, cyclohexyl or cycloheptyl. Especially preferred are cyclopropyl, cyclopentyl and cyclohexyl.

The term "lower cycloalkylalkyl" or "C₃₋₇-cycloalkyl-C₁₋₇-alkyl" refers to lower alkyl groups as defined above wherein at least one of the hydrogen atoms of the lower alkyl group is replaced by cycloalkyl. A preferred example is cyclohexylmethyl.

The term "lower alkoxy" refers to the group R'-O-, wherein R' is lower alkyl and the term "lower alkyl" has the previously given significance. Examples of lower alkoxy groups are e.g. methoxy, ethoxy, n-propoxy, isopropoxy, n-butoxy, isobutoxy, sec. butoxy and tert.-butoxy, preferably methoxy and ethoxy and most preferred methoxy.

The term "lower alkoxyalkyl" or "C₁₋₇-alkoxy-C₁₋₇-alkyl" refers to lower alkyl groups as defined above wherein at least one of the hydrogen atoms of the lower alkyl groups is replaced by an alkoxy group, preferably methoxy or ethoxy. Among the preferred lower alkoxyalkyl groups are 2-methoxyethyl or 3-methoxypropyl.

The term "halogen" refers to fluorine, chlorine, bromine and iodine, with fluorine, chlorine and bromine being preferred.

The term "lower halogenalkyl" or "halogen-C₁₋₇-alkyl" refers to lower alkyl groups as defined above wherein at least one of the hydrogen atoms of the lower alkyl group is replaced by a halogen atom, preferably fluoro or chloro, most preferably fluoro. Among the preferred halogenated lower alkyl groups are trifluoromethyl, difluoromethyl, fluoromethyl and chloromethyl, with trifluoromethyl being especially preferred.

The term "carbamoyl" refers to the group -CO-NH₂.

The term "lower phenylalkyl" or "phenyl-C₁₋₇-alkyl" to lower alkyl groups as defined above wherein at least one of the hydrogen atoms of the lower alkyl group is replaced by a phenyl group. Preferred lower phenylalkyl groups are benzyl or phenethyl.

The term "form a 4-, 5-, 6- or 7-membered heterocyclic ring optionally containing a further heteroatom selected from nitrogen, oxygen or sulfur" refers to a saturated N-heterocyclic ring, which may optionally contain a further nitrogen, oxygen or sulfur atom, such as azetidinyl, pyrrolidinyl, imidazolidinyl, pyrazolidinyl, oxazolidinyl, isoxazolidinyl, thiazolidinyl, isothiazolidinyl, piperidinyl, piperazinyl, morpholinyl, thiomorpholinyl, or azepanyl. A "4-, 5-, 6- or 7-membered heterocyclic ring containing a sulfinyl group or a sulfonyl group" means a N-heterocyclic ring that contains a -S(O)-group or a -SO₂- group, for example 1-oxothiomorpholinyl or 1,1-dioxothiomorpholinyl. The heterocyclic ring may be unsubstituted or substituted by one, two or three groups independently selected from lower alkyl, halogen, cyano, lower alkoxy and phenyl, wherein phenyl is unsubstituted or substituted with one or two groups independently selected from lower alkyl, halogen and lower alkoxy.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, preferably hydrochloric acid, and organic acids such as acetic acid, propionic acid, glycolic acid, pyruvic acid, oxylic acid, maleic acid, malonic acid, salicylic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared form addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins and the like. The compound of formula I can also be present in the form of zwitterions. Particularly preferred pharmaceutically acceptable salts of compounds of formula I are the hydrochloride salts.

The compounds of formula I can also be solvated, e.g. hydrated. The solvation can be effected in the course of the manufacturing process or can take place e.g. as a consequence of hygroscopic properties of an initially anhydrous compound of formula I (hydration). The term "pharmaceutically acceptable salts" also includes physiologically acceptable solvates.

"Isomers" are compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers. A carbon atom bonded to four nonidentical substituents is termed a "chiral center".

In detail, the present invention relates to compounds of the general formula 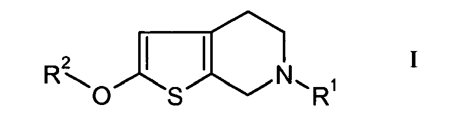 wherein
- R¹: is selected from the group consisting of
-CO-R³, wherein
R³ is selected from the group consisting of
C₁₋₇-alkyl,
C₃₋₇-cycloalkylalkyl, wherein the cycloalkyl ring may be unsubstituted or substituted with one or two groups independently selected from C₁₋₇-alkyl; phenyl, wherein the phenyl ring may be unsubstituted or substituted with one or two groups independently selected from C₁₋₇-alkyl, cyano, halogen, halogen-C₁₋₇-alkyl and C₁₋₇-alkoxy,
-CO-NR⁴R⁵, wherein
R⁴ and R⁵ independently from each other are selected from the group consisting of hydrogen, C₁₋₇-alkyl, C₁₋₇-alkoxy-C₁₋₇-alkyl, phenyl unsubstituted or substituted with one or two groups independently selected from C₁₋₇-alkyl, halogen, C₁₋₇-alkoxy and halogen-C₁₋₇-alkyl, and phenyl-C₁₋₇-alkyl, wherein phenyl is unsubstituted or substituted with one or two groups independently selected from C₁₋₇-alkyl, halogen, C₁₋₇-alkoxy and halogen-C₁₋₇-alkyl,
or R⁴ and R⁵ together with the nitrogen atom to which they are attached form a 4-, 5-, 6- or 7-membered heterocyclic ring optionally containing a further heteroatom selected from nitrogen, oxygen or sulfur, a sulfinyl group or a sulfonyl group, said heterocyclic ring being unsubstituted or substituted with one or two groups independently selected from C₁₋₇-alkyl, halogen, cyano, C₁₋₇-alkoxy and phenyl, wherein phenyl is unsubstituted or substituted with one or two groups independently selected from C₁₋₇-alkyl, halogen and C₁₋₇- alkoxy, and
-SO₂-R⁶, wherein R⁶ is selected from the group consisting of C₁₋₇-alkyl, C₁₋₇-alkoxy-C₁₋₇-alkyl,
C₃₋₇-cycloalkyl-C₁₋₇-alkyl, wherein the cycloalkyl ring may be unsubstituted or substituted with one or two groups independently selected from C₁₋₇-alkyl, halogen and C₁₋₇-alkoxy;
phenyl, wherein the phenyl ring may be unsubstituted or substituted with one or two groups independently selected from C₁₋₇-alkyl, cyano, halogen, halogen-C₁₋₇-alkyl and C₁₋₇-alkoxy;
- R²: is a group selected from 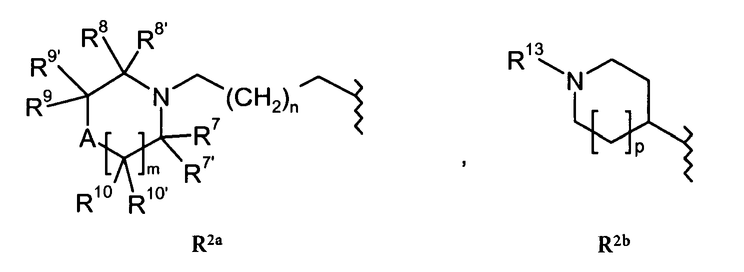 and 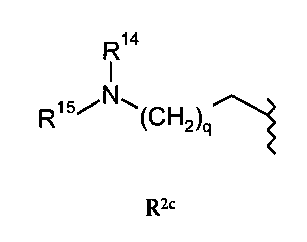
wherein
- m: is 0, 1 or 2;
- n: is 0, 1 or 2;
- A: is selected from -CR¹¹R^{11'}-, O, S or -NR¹²;
- R⁷, R^{7'}, R⁸, R^{8'}, R⁹, R^{9'}, R¹⁰, R^{10'}, R¹¹ and R^{11'}: independently from each other are hydrogen or C₁₋₇-alkyl;
- R¹²: is hydrogen or C₁₋₇- alkyl;
- p: is 0, 1 or 2;
- R¹³: is C₁₋₇-alkyl or C₃₋₇-cycloalkyl;
- q: is 1, 2 or 3; and
- R¹⁴ and R¹⁵: independently from each other are C₁₋₇-alkyl;
and pharmaceutically acceptable salts thereof.

A group of preferred compounds of the present invention are compounds of formula I, wherein R¹ is -CO-R³ and wherein R³ is selected from the group consisting of C₁₋₇-alkyl, C₃₋₇-cycloalkyl-C₁₋₇-alkyl, wherein the cycloalkyl ring may be unsubstituted or substituted with one or two groups independently selected from C₁₋₇-alkyl, and phenyl, wherein the phenyl ring may be unsubstituted or substituted with one or two groups independently selected from C₁₋₇-alkyl, cyano, halogen, halogen-C₁₋₇-alkyl and C₁₋₇-alkoxy.

Compounds of formula I, wherein R¹ is -CO-R³ and wherein R³ is C₁₋₇-alkyl or C₃₋₇-cycloalkylalkyl, wherein the cycloalkyl ring may be unsubstituted or substituted with one or two groups independently selected from C₁₋₇-alkyl, are especially preferred, with those compounds, wherein R³ is C₁₋₇-alkyl, being more preferred, and with those compounds of formula I, wherein R³ is C₄₋₅-alkyl such as isobutyl or 2,2-dimethylpropyl, being most preferred.

Also preferred are compounds of formula I according to the invention, wherein R¹ is -CO-R³ and wherein R³ is phenyl, wherein the phenyl ring may be unsubstituted or substituted with one or two groups independently selected from C₁₋₇-alkyl, cyano, halogen, halogen-C₁₋₇-alkyl and C₁₋₇-alkoxy, with those compounds wherein R³ is phenyl substituted with one or two groups independently selected from halogen, halogen-C₁₋₇-alkyl and C₁₋₇-alkoxy, being most preferred.

Also preferred are compounds of formula I of the present invention are compounds of formula I, wherein R¹ is -CO-NR⁴R⁵ and wherein R⁴ and R⁵ independently from each other are selected from the group consisting of hydrogen, C₁₋₇-alkyl, C₁₋₇-alkoxy-C₁₋₇-alkyl, phenyl unsubstituted or substituted with one or two groups independently selected from C₁₋₇-alkyl, halogen, C₁₋₇-alkoxy and halogen-C₁₋₇-alkyl, and phenyl-C₁₋₇-alkyl, wherein phenyl is unsubstituted or substituted with one or two groups independently selected from C₁₋₇-alkyl, halogen, C₁₋₇-alkoxy and halogen-C₁₋₇-alkyl, or R⁴ and R⁵ together with the nitrogen atom to which they are attached form a 4-, 5-, 6-or 7-membered heterocyclic ring optionally containing a further heteroatom selected from nitrogen, oxygen or sulfur, a sulfinyl group or a sulfonyl group, said heterocyclic ring being unsubstituted or substituted with one or two groups independently selected from C₁₋₇-alkyl, halogen, cyano, C₁₋₇-alkoxy and phenyl, wherein phenyl is unsubstituted or substituted with one or two groups independently selected from C₁₋₇-alkyl, halogen and C₁₋₇-alkoxy.

More preferred are compounds of formula I, wherein R¹ is -CO-NR⁴R⁵ and wherein R⁴ and R⁵ independently from each other are selected from the group consisting of hydrogen, C₁₋₇-alkyl, C₁₋₇-alkoxy-C₁₋₇-alkyl, phenyl unsubstituted or substituted with one or two groups independently selected from C₁₋₇-alkyl, halogen, C₁₋₇-alkoxy and halogen-C₁₋₇-alkyl, and phenyl-C₁₋₇-alkyl, wherein phenyl is unsubstituted or substituted with one or two groups independently selected from C₁₋₇-alkyl, halogen, C₁₋₇-alkoxy and halogen-C₁₋₇-alkyl, with those compounds, wherein R⁴ and R⁵ independently from each other are hydrogen or C₁₋₇-alkyl, being especially preferred.

More preferably, at least one of R⁴ and R⁵ is not hydrogen.

Also more preferred are those compounds of formula I according to the invention, wherein R¹ is -CO-NR⁴R⁵ and wherein R⁴ and R⁵ together with the nitrogen atom to which they are attached form a 4-, 5-, 6- or 7-membered heterocyclic ring optionally containing a further heteroatom selected from nitrogen, oxygen or sulfur, a sulfinyl group or a sulfonyl group, said heterocyclic ring being unsubstituted or substituted with one or two groups independently selected from C₁₋₇-alkyl, halogen, cyano, C₁₋₇-alkoxy and phenyl, wherein phenyl is unsubstituted or substituted with one or two groups independently selected from C₁₋₇-alkyl, halogen and C₁₋₇-alkoxy.

Within this group, those compounds are preferred, wherein R¹ is -CO-NR⁴R⁵ and wherein R⁴ and R⁵ together with the nitrogen atom to which they are attached form a heterocyclic ring selected from the group consisting of pyrrolidinyl, piperidinyl, piperazinyl and morpholinyl, said heterocyclic ring being unsubstituted or substituted with one or two groups independently selected from C₁₋₇-alkyl, halogen, cyano, C₁₋₇-alkoxy and phenyl, wherein phenyl is unsubstituted or substituted with one or two groups independently selected from C₁₋₇-alkyl, halogen and C₁₋₇-alkoxy.

Further preferred are compounds of formula I according to the present invention, wherein R¹ is SO₂-R⁶ and wherein R⁶ is selected from the group consisting of C₁₋₇-alkyl, C₁₋₇-alkoxy-C₁₋₇-alkyl,
C₃₋₇-cycloalkyl-C₁₋₇-alkyl, wherein the cycloalkyl ring may be unsubstituted or substituted with one or two groups independently selected from C₁₋₇-alkyl, halogen
and C₁₋₇-alkoxy, and
phenyl, wherein the phenyl ring may be unsubstituted or substituted with one or two groups independently selected from C₁₋₇-alkyl, cyano, halogen, halogen-C₁₋₇-alkyl and C₁₋₇-alkoxy.

Within this group, compounds of formula I are preferred, wherein R¹ is SO₂-R⁶ and wherein R⁶ is C₁₋₇-alkyl or phenyl, wherein the phenyl ring may be unsubstituted or substituted with one or two groups selected from C₁₋₇-alkyl and halogen.

Further preferred compounds of formula I according to the present invention are those compounds, wherein R² signifies 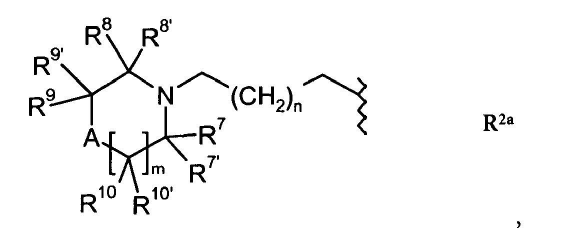 wherein
- m: is 0 or 1;
- n: is 1;
- A: is selected from -CR¹¹R^{11'}-, O, S or -NR¹²;
- R⁷, R^{7'}, R⁸, R^{8'}, R⁹, R^{9'}, R¹⁰, R^{11'}, R¹¹ and R^{11'}: independently from each other are hydrogen or C₁₋₇-alkyl; and
- R¹²: is hydrogen or C₁₋₇-alkyl.

Within this group, those compounds of formula I are preferred, wherein A is CR¹¹R^{11'} and R⁷, R^{7'}, R⁸, R⁸', R⁹, R^{9'}, R¹⁰, R^{10'}, R¹¹ and R^{11'} independently from each other are hydrogen or C₁₋₇-alkyl, with those compounds of formula I, wherein R⁷, R^{7'}, R⁸, R^{8'}, R⁹, R^{9'}, R¹⁰, R^{10'}, R¹¹ and R^{11'} are hydrogen, being especially preferred.

Also preferred are compounds of formula I, wherein A is O, with those compounds of formula I, wherein A is O and R⁷, R^{7'}, R⁸, R^{8'}, R⁹, R^{9'}, R¹⁰ and R^{10'} are hydrogen, being especially preferred.

Further preferred are compounds of formula I according to the invention, wherein R² signifies 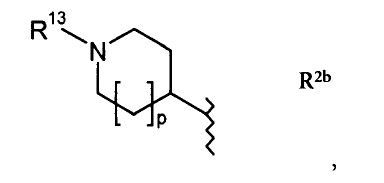 wherein p is 0 or 1; and R¹³ is C₁₋₇-alkyl or C₃₋₇-cycloalkyl.

Within this group, those compounds are especially preferred, wherein R¹³ is isopropyl or isobutyl.

Further preferred are compounds of formula I according to the invention, wherein R² signifies 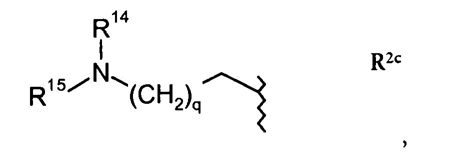 wherein q is 1, 2 or 3; and R¹⁴ and R¹⁵ independently from each other are C₁₋₇-alkyl.

Within this group, those compounds are especially preferred, wherein q is 1 and R¹⁴ and R¹⁵ are methyl.

Examples of preferred compounds of formula I are the following:
morpholin-4-yl-[2-(3-piperidin-1-yl-propoxy)-4,7-dihydro-5*H*-thieno[*2,3-c*]pyridin-6-yl] -methanone,
piperidin-1-yl-[2-(3-piperidin-1-yl-propoxy)-4,7-dihydro-5*H*-thieno[*2,3-c*]pyridin-6-yl]-methanone,
(4-methyl-piperazin-1-yl)-[2-(3-piperidin-1-yl-propoxy)-4,7-dihydro-5*H*-thieno[*2,3-c*]pyridin-6-yl]-methanone,
[2-(3-piperidin-1-yl-propoxy)-4,7-dihydro-5*H*-thieno[*2,3-c*]pyridin-6-yl]-pyrrolidin-1-yl-methanone,
(4-methoxy-piperidin-1-yl)-[2-(3-piperidin-1-yl-propoxy)-4,7-dihydro-5*H*-thieno[*2,3-c*]pyridin-6-yl]-methanone,
[2-(3-piperidin-1-yl-propoxy)-4,7-dihydro-5*H*-thieno[*2,3-c*]pyridin-6-yl]-(4-o-tolyl-piperazin-1-yl)-methanone,
3-methyl-1-[2-(3-piperidin-1-yl-propoxy)-4,7-dihydro-*5H*-thieno[*2*,*3*-*c*]pyridin-6-yl]-butan-1-one,
3,3-dimethyl-1-[2-(3-piperidin-1-yl-propoxy)-4,7-dihydro-*5H*-thieno[*2*,*3*-*c*]pyridin-6-yl]-butan-1-one,
(4-methoxy-phenyl)-[2-(3-piperidin-1-yl-propoxy)-4,7-dihydro-*5H*-thieno[*2*,*3-c*]pyridin-6-yl]-methanone,
(3-fluoro-phenyl)-[2-(3-piperidin-1-yl-propoxy)-4,7-dihydro-*5H*-thieno[*2*,*3*-*c*]pyridin-6-yl]-methanone,
(3-chloro-phenyl)-[2-(3-piperidin-1-yl-propoxy)-4,7-dihydro-*5H*-thieno[*2*,*3*-*c*]pyridin-6-yl]-methanone,
6-methanesulfonyl-2-(3-piperidin-1-yl-propoxy)-4,5,6,7-tetrahydro-thieno[*2*,*3-c*]pyridine,
6-(3-fluoro-benzenesulfonyl)-2-(3-piperidin-1-yl-propoxy)-4,5,6,7-tetrahydro-thieno[*2*,*3*-*c*]pyridine,
2-(3-piperidin-1-yl-propoxy)-6-(toluene-4-sulfonyl)-4,5,6,7-tetrahydro-thieno[*2*,*3-c*]pyridine,
2-(3-piperidin-1-yl-propoxy)-6-(toluene-2-sulfonyl)-4,5,6,7-tetrahydro-thieno[*2*,*3-c*]pyridine,
6-(4-ethyl-benzenesulfonyl)-2-(3-piperidin-1-yl-propoxy)-4,5,6,7-tetrahydro-thieno[*2*,*3-c*]pyridine,
6-(4-tert-butyl-benzenesulfonyl)-2-(3-piperidin-1-yl-propoxy)-4,5,6,7-tetrahydro-thieno[*2*,*3*-*c*]pyridine,
6-benzenesulfonyl-2-(3-piperidin-1-yl-propoxy)-4,5,6,7-tetrahydro-thieno[*2*,*3-c*]pyridine,
6-(4-isopropyl-benzenesulfonyl)-2-(3-piperidin-1-yl-propoxy)-4,5,6,7-tetrahydro-thieno[*2*,*3*-*c*]pyridine,
3,3-dimethyl-1-[2-(3-pyrrolidin-1-yl-propoxy)-4,7-dihydro-*5H*-thieno[*2,3-c*]pyridin-6-yl]-butan-1-one,
(3-chloro-phenyl)-[2-(3-pyrrolidin-1-yl-propoxy)-4,7-dihydro-*5H*-thieno[*2*,*3*-*c*]pyridin-6-yl]-methanone,
(2-chloro-phenyl)-[2-(3-pyrrolidin-1-yl-propoxy)-4,7-dihydro-*5H*-thieno[*2*,*3*-*c*]pyridin-6-yl]-methanone,
[2-(3-pyrrolidin-1-yl-propoxy)-4,7-dihydro-*5H*-thieno[*2*,*3*-*c*]pyridin-6-yl]-(4-trifluoromethyl-phenyl)-methanone,
phenyl-[2-(3-pyrrolidin-1-yl-propoxy)-4,7-dihydro-*5H*-thieno[*2*,*3*-*c*]pyridin-6-yl]-methanone,
(3-methoxy-phenyl)-[2-(3-pyrrolidin-1-yl-propoxy)-4,7-dihydro-*5H*-thieno[*2*,*3-c*]pyridin-6-yl]-methanone,
morpholin-4-yl-[2-(3-pyrrolidin-1-yl-propoxy)-4,7-dihydro-*5H*-thieno[*2,3-c*]pyridin-6-yl]-methanone,
pyrrolidin-1-yl-[2-(3-pyrrolidin-1-yl-propoxy)-4,7-dihydro-*5H*-thieno[*2,3-c*]pyridin-6-yl]-methanone,
piperidin-1-yl-[2-(3-pyrrolidin-1-yl-propoxy)-4,7-dihydro-*5H*-thieno[*2*,*3-c*]pyridin-6-yl]-methanone,
(4-methyl-piperazin-1-yl)-[2-(3-pyrrolidin-1-yl-propoxy)-4,7-dihydro-*5H*-thieno[*2*,*3-c*]pyridin-6-yl]-methanone,
2-(3-pyrrolidin-1-yl-propoxy)-4,7-dihydro-*5H*-thieno[*2*,*3-c*]pyridin-6-yl]-(4-o-tolyl-piperazin-1-yl)-methanone,
6-methanesulfonyl-2-(3-pyrrolidin-1-yl-propoxy)-4,5,6,7-tetrahydro-thieno[*2,3-c*]pyridine,
6-benzenesulfonyl-2-(3-pyrrolidin-1-yl-propoxy)-4,5,6,7-tetrahydro-thieno[*2*,*3-c*]pyridine,
2-(3-pyrrolidin-1-yl-propoxy)-6-(toluene-4-sulfonyl)-4,5,6,7-tetrahydro-thieno[*2*,*3-c*] pyridine,
2-(3-pyrrolidin-1-yl-propoxy)-6-(toluene-2-sulfonyl)-4,5,6,7-tetrahydro-thieno[*2*,*3-c*]pyridine,
2-(3-pyrrolidin-1-yl-propoxy)-6-(toluene-3-sulfonyl)-4,5,6,7-tetrahydro-thieno[*2*,*3-c*]pyridine,
6-(4-ethyl-benzenesulfonyl)-2-(3-pyrrolidin-1-yl-propoxy)-4,5,6,7-tetrahydro-thieno[*2*,*3*-*c*]pyridine,
6-(4-isopropyl-benzenesulfonyl)-2-(3-pyrrolidin-1-yl-propoxy)-4,5,6,7-tetrahydro-thieno[*2*,*3*-*c*]pyridine,
6-(4-tert-butyl-benzenesulfonyl)-2-(3-pyrrolidin-1-yl-propoxy)-4,5,6,7-tetrahydro-thieno[*2*,*3*-*c*]pyridine,
6-(3-fluoro-benzenesulfonyl)-2-(3-pyrrolidin-1-yl-propoxy)-4,5,6,7-tetrahydro-thieno[*2*,*3*-*c*]pyridine,
6-(propane-2-sulfonyl)-2-3-pyrrolidin-1-yl-propoxy)-4,5,6,7-tetrahydro-thieno[*2*,*3-c*]pyridine,
[2-(1-isobutyl-piperidin-4-yloxy)-4,7-dihydro-*5H*-thieno[*2*,*3-c*]pyridin-6-yl]-phenyl-methanone,
1-[2-(1-isobutyl-piperidin-4-yloxy)-4,7-dihydro-*5H*-thieno[*2,3-c*]pyridin-6-yl-butan-1-one,
1-[2-(1-isobutyl-piperidin-4-yloxy)-4,7-dihydro-*5H*-thieno[*2*,*3*-*c*]pyridin-6-yl]-pentan-1-one,
1-[2-(1-isobutyl-piperidin-4-yloxy)-4,7-dihydro-*5H*-thieno[*2,3*-*c*]pyridin-6-yl]-3,3-dimethyl-butan-1-one,
[2-(1-isobutyl-piperidin-4-yloxy)-4,7-dihydro-*5H*-thieno[*2*,*3*-*c*]pyridin-6-yl]-(3-methoxy-phenyl)-methanone,
[2-(1-isobutyl-piperidin-4-yloxy)-4,7-dihydro-*5H*-thieno[*2,3-c*]pyridin-6-yl]-(4-methoxy-phenyl)-methanone,
(4-fluoro-phenyl)-[2-(1-isobutyl-piperidin-4-yloxy)-4,7-dihydro-*5H*-thieno[*2,3-c*]pyridin-6-yl]-methanone,
(3-fluoro-phenyl)-[2-(1-isobutyl-piperidin-4-yloxy)-4,7-dihydro-*5H*-thieno[*2,3-c*]pyridin-6-yl]-methanone,
[2-(1-isobutyl-piperidin-4-yloxy)-4,7-dihydro-*5*H-thieno[*2,3-c*]pyridin-6-yl](4-trifluoromethyl-phenyl)-methanone,
(3-chloro-phenyl)-[2-(1-isobutyl-piperidin-4-yloxy)-4,7-dihydro-*5H*-thieno[*2*,*3-c*]pyridin-6-yl]-methanone,
(2-chloro-phenyl)-[2-(1-isobutyl-piperidin-4-yloxy)-4,7-dihydro-5*H*-thieno[*2*,*3-c*]pyridin-6-yl]-methanone,
[2-(1-isobutyl-piperidin-4-yloxy)-4,7-dihydro-*5H*-thieno[*2*,*3*-*c*]pyridin-6-yl]-morpholin-4-yl-methanone,
2-(1-isobutyl-piperidin-4-yloxy)-4,7-dihydro-*5H*-thieno[*2*,*3*-*c*]pyridine-6-carboxcylic acid diethylamide,
[2-(1-isobutyl-piperidin-4-yloxy)-4,7-dihydro-*5H*-thieno[*2*,*3*-*c*]pyridin-6-yl]-piperidin-1-yl-methanone,
[2-(1-isobutyl-piperidin-4-yloxy)-4,7-dihydro-*5H*-thieno[*2*,*3*-*c*]pyridin-6-yl]-pyrrolidin-1-yl-methanone,
2-(1-isobutyl-piperidin-4-yloxy)-6-methanesulfonyl-4,5,6,7-tetrahydro-thieno[*2,3-c*]pyridine,
6-benzenesulfonyl-2-(1-isobutyl-piperidin-4-yloxy)-4,5,6,7-tetrahydro-thieno[*2,3-c*]pyridine,
2-(1-isobutyl-piperidin-4-yloxy)-6-(toluene-4-sulfonyl)-4,5,6,7-tetrahydro-thieno[*2,3-c*]pyridine,
2-(1-isobutyl-piperidin-4-yloxy)-6-(toluene-2-sulfonyl)-4,5,6,7-tetrahydro-thieno[*2,3-c*]pyridine,
2-(1-isobutyl-piperidin-4-yloxy)-6-(toluene-3-sulfonyl)-4,5,6,7-tetrahydro-thieno[*2,3-c*]pyridine,
6-(4-ethyl-benzenesulfonyl)-2-(1-isobutyl-piperidin-4-yloxy)-4,5,6,7-tetrahydro-thieno[*2,3-c*]pyridine,
6-(3-fluoro-benzenesulfonyl)-2-(1-isobutyl-piperidin-4-yloxy)-4,5,6,7-tetrahydro-thieno[*2,3-c*]pyridine,
1-[2-(1-isopropyl-piperidin-4-yloxy)-4,7-dihydro-*5H*-thieno[*2*,*3*-*c*]pyridin-6-yl]-butan-1-one,
1-[2-(1-isopropyl-piperidin-4-yloxy)-4,7-dihydro-*5H*-thieno[*2,3-c*]pyridin-6-yl]-3-methyl-butan-1-one,
1-[2-(1-isopropyl-piperidin-4-yloxy)-4,7-dihydro-*5H*-thieno[*2*,*3-c*]pyridin-6-yl]-3,3-dimethyl-butan-1-one,
[2-(1-isopropyl-piperidin-4-yloxy)-4,7-dihydro-*5H*-thieno[*2,3-c*]pyridin-6-yl]-(4-trifluoromethyl-phenyl)-methanone,
(3-chloro-phenyl)-[2-(1-isopropyl-piperidin-4-yloxy)-4,7-dihydro-*5H*-thieno[*2,3-c*]pyridin-6-yl]-methanone,
[2-(1-isopropyl-piperidin-4-yloxy)-4,7-dihydro-*5H*-thieno[*2,3-c*]pyridin-6-yl]-(3-methoxy-phenyl)-methanone,
[2-(1-isopropyl-piperidin-4-yloxy)-4,7-dihydro-*5H*-thieno[*2*,*3*-*c*]pyridin-6-yl]-(4-methoxy-phenyl)-methanone,
(4-fluoro-phenyl)-[2-(1-isopropyl-piperidin-4-yloxy)-4,7-dihydro-*5H*-thieno[*2*,*3-c*]pyridin-6-yl]-methanone,
(3-fluoro-phenyl)-[2-(1-isopropyl-piperidin-4-yloxy)-4,7-dihydro-*5H*-thieno[*2*,*3-c*]pyridin-6-yl]-methanone,
(2-chloro-phenyl)-[2-(1-isopropyl-piperidin-4-yloxy)-4,7-dihydro-*5H*-thieno[*2*,*3-c*]pyridin-6-yl]-methanone,
[2-(1-isopropyl-piperidin-4-yloxy)-4,7-dihydro-*5H*-thieno[*2*,*3*-*c*]pyridin-6-yl]-(2-methoxy-phenyl)-methanone,
(2-fluoro-phenyl)-[2-(1-isopropyl-piperidin-4-yloxy)-4,7-dihydro-*5H*-thieno[*2,3-c*]pyridin-6-yl]-methanone,
[2-(1-isopropyl-piperidin-4-yloxy)-4,7-dihydro-*5H*-thieno[*2*,*3*-*c*]pyridin-6-yl]-morpholin-4-yl-methanone,
2-(1-isopropyl-piperidin-4-yloxy)-4,7-dihydro-*5H*-thieno[*2,3-c*]pyridine-6-carboxylic acid diethylamide,
[2-(1-isopropyl-piperidin-4-yloxy)-4,7-dihydro-*5H*-thieno[*2*,*3*-*c*]pyridin-6-yl]-piperidin-1-yl-methanone,
2-(1-isopropyl-piperidin-4-yloxy)-4,7-dihydro-*5H*-thieno[*2,3-c*]pyridine-6-carboxylic acid methylamide,
[2-(1-isopropyl-piperidin-4-yloxy)-4,7-dihydro-*5H*-thieno[*2*,*3*-*c*]pyridin-6-yl]-(4-methyl-piperazin-1-yl)-methanone,
2-(1-isopropyl-piperidin-4-yloxy)-4,7-dihydro-*5H*-thieno[*2,3-c*]pyridine-6-carboxylic acid ethylamide,
2-(1-isopropyl-piperidin-4-yloxy)-4,7-dihydro-*5H*-thieno[*2*,*3*-*c*]pyridine-6-carboxylic acid isopropylamide,
2-(1-isopropyl-piperidin-4-yloxy)-4,7-dihydro-*5H*-thieno[*2*,*3*-*c*]pyridine-6-carboxylic acid butylamide,
[2-(1-isopropyl-piperidin-4-yloxy)-4,7-dihydro-*5H*-thieno[*2,3-c*]pyridin-6-yl]-(4-o-tolyl-piperazin-1-yl)-methanone,
2-(1-isopropyl-piperidin-4-yloxy)-6-methanesulfonyl-4,5,6,7-tetrahydro-thieno[*2,3-c*]pyridine,
6-benzenesulfonyl-2-(1-isopropyl-piperidin-4-yloxy)-4,5,6,7-tetrahydro-thieno[*2,3-c*]pyridine,
2-(1-isopropyl-piperidin-4-yloxy)-6-(toluene-4-sulfonyl)-4,5,6,7-tetrahydro-thieno[*2,3-c*]pyridine,
2-(1-isopropyl-piperidin-4-yloxy)-6-(toluene-2-sulfonyl)-4,5,6,7-tetrahydro-thieno[*2,3-c*]pyridine,
2-(1-isopropyl-piperidin-4-yloxy)-6-(toluene-3-sulfonyl)-4,5,6,7-tetrahydro-thieno[*2,3-c*]pyridine,
6-(4-ethyl-benzenesulfonyl)-2-(1-isopropyl-piperidin-4-yloxy)-4,5,6,7-tetrahydro-thieno[*2,3-c*]pyridine,
6-(4-isopropyl-benzenesulfonyl)-2-(1-isopropyl-piperidin-4-yloxy)-4,5,6,7-tetrahydro-thieno[*2,3-c*]pyridine,
6-(4-tert-butyl-benzenesulfonyl)-2-(1-isopropyl-piperidin-4-yloxy)-4,5,6,7-tetrahydro-thieno[*2,3-c*]pyridine,
6-(3-fluoro-benzenesulfonyl)-2-(1-isopropyl-piperidin-4-yloxy)-4,5,6,7-tetrahydro-thieno[*2,3-c*]pyridine,
2-(1-isopropyl-piperidin-4-yloxy)-6-(propane-2-sulfonyl)-4,5,6,7-tetrahydro-thieno[*2,3-c*]pyridine,
2-(1-isopropyl-pyrrolidin-3-yloxy)-6-(toluene-4-sulfonyl)-4,5,6,7-tetrahydro-thieno[*2,3-c*]pyridine,
6-(4-tert-butyl-benzenesulfonyl)-2-(1-isopropyl-pyrrolidin-3-yloxy)-4,5,6,7-tetrahydro-thieno[*2*,*3*-*c*]pyridine,
6-(4-isopropyl-benzenesulfonyl)-2-(1-isopropyl-pyrrolidin-3-yloxy)-4,5,6,7-tetrahydro-thieno[*2*,*3*-c]pyridine,
(4-chloro-phenyl)-[2-(3-morpholin-4-yl-propoxy)-4,7-dihydro-*5H*-thieno[*2,3-c*]pyridin-6-yl]-methanone,
(3-chloro-phenyl)-[2-(3-morpholin-4-yl-propoxy)-4,7-dihydro-*5H*-thieno[*2,3-c*]pyridin-6-yl]-methanone,
dimethyl-{2-[6-(toluene-2-sulfonyl)-4,5,6,7-tetrahydro-thieno[*2*,*3*-*c*]pyridin-2-yloxy]-ethyl}-amine,
(2-chloro-phenyl)-[2-(2-dimethylamino-ethoxy)-4,7-dihydro-*5H*-thieno[*2*,*3*-*c*]pyridin-6-yl]-methanone,
{2-[6-(4-isopropyl-benzenesulfonyl)-4,5,6,7-tetrahydro-thieno[*2*,*3*-c]pyridin-2-yloxy]-ethyl}-dimethyl-amine,
and pharmaceutically acceptable salts thereof.

Particularly preferred compounds of formula I of the present invention are the following:
3-methyl-1-[2-(3-piperidin-1-yl-propoxy)-4,7-dihydro-*5H*-thieno[*2*,*3*-*c*]pyridin-6-yl]-butan-1-one,
3,3-dimethyl-1-[2-(3-piperidin-1-yl-propoxy)-4,7-dihydro-*5H*-thieno[*2,3-c*]pyridin-6-yl]-butan-1-one,
6-benzenesulfonyl-2-(3-pyrrolidin-1-yl-propoxy)-4,5,6,7-tetrahydro-thieno[*2,3-c*]pyridine,
1-[2-(1-isopropyl-piperidin-4-yloxy)-4,7-dihydro-5*H*-thieno[*2*,*3*-*c*]pyridin-6-yl]-butan-1-one,
1-[2-(1-isopropyl-piperidin-4-yloxy)-4,7-dihydro-*5H*-thieno[*2*,*3*-c]pyridin-6-yl]-3-methyl-butan-1-one,
1-[2-(1-isopropyl-piperidin-4-yloxy)-4,7-dihydro-*5H*-thieno[*2*,*3*-*c*]pyridin-6-yl]-3,3-dimethyl-butan-1-one,
(3-chloro-phenyl)-[2-(1-isopropyl-piperidin-4-yloxy)-4,7-dihydro-*5H*-thieno[*2*,*3-*c]pyridin-6-yl]-methanone,
[2-(1-isopropyl-piperidin-4-yloxy)-4,7-dihydro-*5H*-thieno[*2*,*3*-*c*]pyridin-6-yl]-(3-methoxy-phenyl) -methanone,
(4-fluoro-phenyl)-[2-(1-isopropyl-piperidin-4-yloxy)-4,7-dihydro-*5H*-thieno[*2,3-c*]pyridin-6-yl]-methanone,
(3-fluoro-phenyl)-[2-(1-isopropyl-piperidin-4-yloxy)-4,7-dihydro-*5H*-thieno[*2,3-c*]pyridin-6-yl]-methanone,
(2-chloro-phenyl)-[2-(1-isopropyl-piperidin-4-yloxy)-4,7-dihydro-*5H*-thieno[*2,3-*c]pyridin-6-yl]-methanone,
(2-fluoro-phenyl)-[2-(1-isopropyl-piperidin-4-yloxy)-4,7-dihydro-*5H*-thieno[*2*,*3-c*]pyridin-6-yl]-methanone,
[2-(1-isopropyl-piperidin-4-yloxy)-4,7-dihydro-*5H*-thieno[*2*,*3*-*c*]pyridin-6-yl]-morpholin-4-yl-methanone,
[2-(1-isopropyl-piperidin-4-yloxy)-4,7-dihydro-*5H*-thieno[*2,3-c*]pyridin-6-yl]-piperidin-1-yl-methanone,
[2-(1-isopropyl-piperidin-4-yloxy)-4,7-dihydro-*5H*-thieno[*2*,*3*-*c*]pyridin-6-yl]-(4-methyl-piperazin-1-yl) -methanone,
2-(1-isopropyl-piperidin-4-yloxy)-6-(toluene-2-sulfonyl)-4,5,6,7-tetrahydro-thieno[*2*,*3-c*]pyridine,
6-(3-fluoro-benzenesulfonyl)-2-(1-isopropyl-piperidin-4-yloxy)-4,5,6,7-tetrahydro-thieno[*2,3-c*]pyridine,
and pharmaceutically acceptable salts thereof.

Furthermore, the pharmaceutically acceptable salts of the compounds of formula I and the pharmaceutically acceptable esters of the compounds of formula I individually constitute preferred embodiments of the present invention.

Compounds of formula I may form acid addition salts with acids, such as conventional pharmaceutically acceptable acids, for example hydrochloride, hydrobromide, phosphate, acetate, fumarate, maleate, salicylate, sulphate, pyruvate, citrate, lactate, mandelate, tartarate, and methanesulphonate. Preferred are the hydrochloride salts. Also solvates and hydrates of compounds of formula I and their salts form part of the present invention.

Compounds of formula I can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates or mixtures of diastereoisomeric racemates. The optically active forms can be obtained for example by resolution of the racemates, by asymmetric synthesis or asymmetric chromatography (chromatography with a chiral adsorbens or eluant). The invention embraces all of these forms.

It will be appreciated, that the compounds of general formula I in this invention may be derivatised at functional groups to provide derivatives which are capable of conversion back to the parent compound in vivo. Physiologically acceptable and metabolically labile derivatives, which are capable of producing the parent compounds of general formula I in vivo are also within the scope of this invention.

A further aspect of the present invention is the process for the manufacture of compounds of formula I as defined above, which process comprises
coupling an amine of the formula II 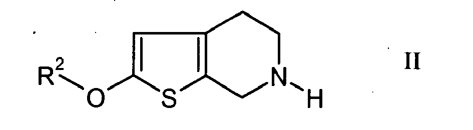 wherein R² is as defined herein before, with an halogenide of the formula III

R¹-X **III**

wherein R¹ is as defined herein before and X is halogen,
in the presence of a base to obtain a compound of the formula I 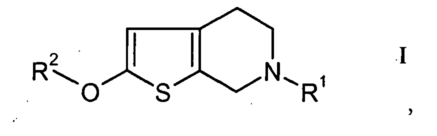 and if desired,
converting the compound obtained into a pharmaceutically acceptable salt.

The compounds of formula R¹-X (III) are acid halogenides, carbamoyl halogenides or sulfonyl halogenides wherein X signifies halogen such as e.g. chloride, iodide or bromide, preferably chloride. The base to be used is preferably a tertiary amine, with triethylamine (TEA) and diisopropylamine (DIPEA) being especially preferred.

The preparation of compounds of formula I of the present invention may be carried out in sequential or convergent synthetic routes. Syntheses of the compounds of the invention are shown in the following scheme. The skills required for carrying out the reaction and purification of the resulting products are known to those skilled in the art. The substituents and indices used in the following description of the processes have the significance given herein before unless indicated to the contrary.

In more detail, the compounds of formula I can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art. 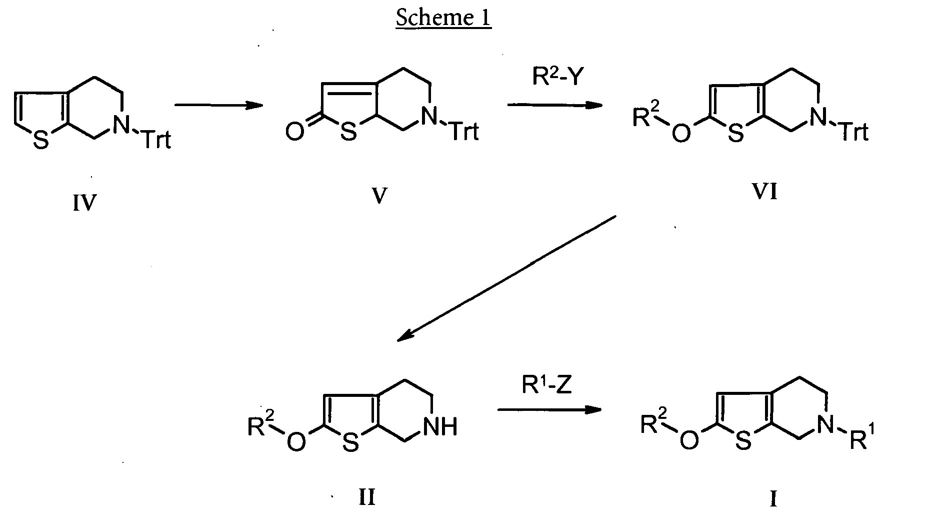
a) IV is described in literature (W02001/032655) and can serve as a potential starting material for the synthesis of compounds of formula I. 6-Trityl-4,5,6,7-tetrahydro-thieno[2,3-c]pyridine IV can be converted to the respective 6-trityl-5,6,7,7a-tetrahydro-4H-thieno[2,3-c]pyridin-2-one V under various conditions and reactions such as those described in: Comprehensive Organic Transformations: A Guide to Functional Group Preparations, 2nd Edition, Richard C. Larock. John Wiley & Sons, New York, N.Y. 1999). However, it is convenient to deprotonate IV with a base in the presence of a solvent and react the intermediate with tri-n-butyl borate. Subsequent oxidation can be achieved with oxidizing agents. There is no particular restriction on the nature of the solvent to be employed, provided that it has no adverse effect on the reaction or the reagents involved and that it can dissolve the reagents, at least to some extent. Examples for suitable solvents include tetrahydrofurane (THF) and the like. There is no particular restriction on the nature of the base used in this stage, and any base commonly used in this type of reaction may equally be employed here. Examples of such bases include n-butyl lithium, and the like. The reaction can take place over a wide range of temperatures, and the precise reaction temperature is not critical to the invention. It is convenient to carry out the reaction with heating from -78 °C to reflux temperature. There is no particular restriction on the nature of the oxidizing agent used in this stage, and any reagent commonly used in this type of reaction may equally be employed here. Examples of such oxidizing agents include H₂O₂, and the like. The time required for the reaction may also vary widely, depending on many factors, notably the reaction temperature and the nature of the reagents. However, a period of from 0.5 h to several days will usually suffice to yield compounds of formula V.
b) Nucleophilic substitutions of aminoalkyl halides are widely described in literature. For reaction conditions described in literature affecting such reactions see for example: Comprehensive Organic Transformations: A Guide to Functional Group Preparations, 2nd Edition, Richard C. Larock. John Wiley & Sons, New York, N.Y. 1999). However, we find it convenient to transform aminoalkyl halides of the formula R²-Y with 6-trityl-5,6,7,7a-tetrahydro-4H-thieno[2,3-c]pyridin-2-one V to the respective thieno[2,3-c]pyridine derivative VI in the presence of a base and a solvent. There is no particular restriction on the nature of the solvent to be employed, provided that it has no adverse effect on the reaction or the reagents involved and that it can dissolve the reagents, at least to some extent. Examples for suitable solvents include dimethylformamide (DMF) and the like. There is no particular restriction on the nature of the base used in this stage, and any base commonly used in this type of reaction may equally be employed here. Examples of such bases include Cs₂CO₃, and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield compounds VI.
c) Protecting group manipulations are widely described in literature. For reaction conditions described in literature affecting such reactions see for example: Handbook of Reagents for Organic Synthesis: Activating and Agents and Protecting Groups, Anthony J. Pearson.; William R: Roush, Editors. John Wiley, Chichester, UK, 1999 or Protecting Groups, Philip J. Kocienski, Georg Thieme 1994, Stuttgart, New York. The trityl protected nitrogen in VI can be liberated in many ways and under varying reaction. However, it is convenient to cleave the protecting group from VI under acidic conditions in the presence or the absence of a solvent to access thieno[2,3-c]pyridine derivatives II. There is no particular restriction on the nature of the solvent to be employed, provided that it has no adverse effect on the reaction or the reagents involved and that it can dissolve the reagents, at least to some extent. Even the acid itself can serve as the solvent. There is no particular restriction on the nature of the acid used in this stage, and any acid commonly used in this type of reaction may equally be employed here. Examples of such acids include trifluoroacetic acid (TFA), and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield compounds of formula II.
d) The coupling of the amine functionality with electrophiles is widely described in literature and the procedures are known to those in the art (For reaction conditions described in literature affecting such reactions see for example: Comprehensive Organic Transformations: A Guide to Functional Group Preparations, 2nd Edition, Richard C. Larock. John Wiley & Sons, New York, N.Y. 1999). The respective amines II can conveniently be transformed to the respective sulfonamides, amides or ureas through coupling of V with sulfonyl halogenides, acid halogenides or carbamoyl halogenides of the formula R¹-X wherein X is halogenide, preferably chloride (either commercially available or accessible by methods described in references or by methods known in the art; as appropriate). Alternatively, the amines II can also be added to isocyanates of the formula R⁴-N=C=O to obtain the respective compounds of formula I, wherein R¹ is - CO-NHR⁴. It is convenient to carry out the reaction in a solvent and in the presence of a base. There is no particular restriction on the nature of the solvent to be employed, provided that it has no adverse effect on the reaction or the reagents involved and that it can dissolve the reagents, at least to some extent. Examples for suitable solvents include dichloromethane (DCM), dioxane, THF, and the like. There is no particular restriction on the nature of the base used in this stage, and any base commonly used in this type of reaction may equally be employed here. Examples of such bases include triethylamine and diisopropylethyl-amine (DIPEA), and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield thieno[2,3-c]pyridine derivatives of formula I.

As described above, the compounds of formula I of the present invention can be used as medicaments for the treatment and/or prevention of diseases which are associated with the modulation of H3 receptors.

In this context, the expression 'diseases associated with the modulation of H3 receptors' means diseases which can be treated and/or prevented by modulation of H3 receptors. Such diseases encompass, but are not limited to, obesity, metabolic syndrome (syndrome X), neurological diseases including Alzheimer's disease, dementia, age-related memory dysfunction, mild cognitive impairment, cognitive deficit, attention deficit hyperactivity disorder, epilepsy, neuropathic pain, inflammatory pain, migraine, Parkinson's disease, multiple sclerosis, stroke, dizziness, schizophrenia, depression, addiction, motion sickness and sleep disorders including narcolepsy, and other diseases including asthma, allergy, allergy-induced airway responses, congestion, chronic obstructive pulmonary disease and gastro-intestinal disorders.

In a preferable aspect, the expression 'diseases associated with modulation of H3 receptors' relates to obesity, metabolic syndrome (syndrome X), and other eating disorders, with obesity being especially preferred.

The invention therefore also relates to pharmaceutical compositions comprising a compound as defined above and a pharmaceutically acceptable carrier and/or adjuvant.

Further, the invention relates to compounds as defined above for use as therapeutically active substances, particularly as therapeutic active substances for the treatment and/or prevention of diseases which are associated with the modulation of H3 receptors.

In addition, the invention relates to the use of compounds of formula I as defined above for the preparation of medicaments for the treatment and/or prevention of diseases which are associated with the modulation of H3 receptors. The use of compounds of formula I as defined above for the preparation of medicaments for the treatment and/or prevention of obesity is preferred.

Furthermore, the present invention relates to the use of a compound of formula I for the manufacture of a medicament for the treatment and prevention of obesity in a patient who is also receiving treatment with a lipase inhibitor and particularly, wherein the lipase inhibitor is orlistat.

It is a further preferred object of the present invention to provide a therapeutically effective amount of a compound according to formula I for use in the treatment or prevention of obesity and obesity related disorders in combination or association with a therapeutically effective amount of other drugs for the treatment of obesity or eating disorders so that together they give effective relief. Suitable other drugs include, but are not limited to, anorectic agents, lipase inhibitors, selective serotonin reuptake inhibitors (SSRI) and agents that stimulate metabolism of body fat. Combinations or associations of the above agents may be encompassing separate, sequential or simultaneous administration.

The term "lipase inhibitor" refers to compounds which are capable of inhibiting the action of lipases, for example gastric and pancreatic lipases. For example orlistat and lipstatin as described in U.S. Patent No. 4,598,089 are potent inhibitor of lipases. Lipstatin is a natural product of microbial origin, and orlistat is the result of a hydrogenation of lipstatin. Other lipase inhibitors include a class of compound commonly referred to as panclicins. Panclicins are analogues of orlistat (Mutoh et al, 1994). The term "lipase inhibitor" refers also to polymer bound lipase inhibitors for example described in International Patent Application WO 99/34786 (Geltex Pharmaceuticals Inc.). These polymers are **characterized in that** they have been substituted with one or more groups that inhibit lipases. The term "lipase inhibitor" also comprises pharmaceutically acceptable salts of these compounds. The term "lipase inhibitor" preferably refers to tetrahydrolipstatin. Administration of a therapeutically effective amount of a compound according to formula I in combination or association with a therapeutically effective amount of tetrahydrolipstatin is especially preferred.

Tetrahydrolipstatin (orlistat) is a known compound useful for the control or prevention of obesity and hyperlipidemia. See, U.S. Patent No. 4,598,089, issued July 1, 1986, which also discloses processes for making orlistat and U.S. Patent No. 6,004,996, which discloses appropriate pharmaceutical compositions. Further suitable pharmaceutical compositions are described for example in International Patent Applications WO 00/09122 and WO 00/09123. Additional processes for the preparation of orlistat are disclosed in European Patent Applications Publication Nos. 0 185 359, 0 189 577, 0 443 449, and 0 524 495.

Suitable anorectic agents of use in combination with a compound of the present invention include, but are not limited to, APD356, aminorex, amphechloral, amphetamine, axokine, benzphetamine, bupropion, chlorphentermine, clobenzorex, cloforex, clominorex, clortermine, CP945598, cyclexedrine, CYT009-GhrQb, dexfenfluramine, dextroamphetamine, diethylpropion, diphemethoxidine, N-ethylamphetamine, fenbutrazate, fenfluramine, fenisorex, fenproporex, fludorex, fluminorex, furfurylmethylamphetamine, levamfetamine, levophacetoperane, mazindol, mefenorex, metamfepramone, methamphetamine, metreleptin, norpseudoephedrine, pentorex, phendimetrazine, phenmetrazine, phentermine, phenylpropanolamine, picilorex, rimonabant, sibutramine, SLV319, SNAP 7941, SR147778 (Surinabant), steroidal plant extract (e.g. P57) and TM30338 and pharmaceutically acceptable salts thereof.

Most preferable anorectic agents are sibutramine, rimonabant and phentermine.

Suitable selective serotonin reuptake inhibitors of use in combination with a compound of the present invention include: fluoxetine, fluvoxamine, paroxetine and sertraline, and pharmaceutically acceptable salts thereof.

Suitable agents that stimulate metabolism of body fat include, but are not limited to, growth hormone agonist (e.g. AOD-9604).

The use of a compound of formula I in the manufacture of a medicament for the treatment and prevention of obesity in a patient who is also receiving treatment with a compound selected from the group consisting of a lipase inhibitor, an anorectic agent, a selective serotonin reuptake inhibitor, and an agent that stimulates metabolism of body fat, is also an object of the present invention.

The use of a compound of formula I in the manufacture of a medicament for the treatment and prevention of obesity in a patient who is also receiving treatment with a a lipase inhibitor, preferably with tetrahydrolipstatin, is also an object of the present invention.

It is a further preferred object to provide a therapeutically effective amount of a compound according to formula I for use in treatment or prevention of Type II diabetes (non-insulin dependent diabetes mellitus (NIDDM)) in combination or association with a therapeutically effective amount of a lipase inhibitor, particularly, wherein the lipase inhibitor is tetrahydrolipstatin. Also an object of the invention is the simultaneous, separate or sequential administration of a compound according to formula I and a lipase inhibitor, particularly tetrahydrolipstatin.

It is a further preferred object to provide a therapeutically effective amount of a compound according to formula I for use in treatment or prevention of Type II diabetes (non-insulin dependent diabetes mellitus (NIDDM)) in combination or association with a therapeutically effective amount of an anti-diabetic agent.

The term "anti-diabetic agent" refers to compounds selected from the group consisting of 1) PPARγ agonists such as pioglitazone (actos) or rosiglitazone (avandia), and the like; 2) biguanides such as metformin (glucophage), and the like; 3) sulfonylureas such as glibenclamide, glimepiride (amaryl), glipizide (glucotrol), glyburide (DiaBeta), and the like; 4) nonsulfonylureas such as nateglinide (starlix), repaglimide (prandin), and the like; 5) PPARα/γ agonists such as GW-2331, and the like 6) DPP-IV- inhibitors such as LAF-237 (vildagliptin), MK-0431, BMS-477118 (saxagliptin) or GSK23A and the like; 7) Glucokinase activators such as the compounds disclosed in e.g. WO 00/58293 Al, and the like; 8) α-Glucosidase inhibitors such as acarbose (precose) or miglitol (glyset), and the like.

Also an object of the invention is the simultaneous, separate or sequential administration of a compound according to formula I and a therapeutically effective amount of an anti-diabetic agent.

The use of a compound of formula I in the manufacture of a medicament for the treatment and prevention of Type II diabetes in a patient who is also receiving treatment with an anti-diabetic agent is also an object of the present invention.

It is a further preferred object to provide a therapeutically effective amount of a compound according to formula I for use in treatment or prevention of dyslipidemias in combination or association with a therapeutically effective amount of a lipid lowering agent.

The term "lipid lowering agent" refers to compounds selected from the group consisting of 1) bile acid sequestrants such as cholestyramine (questran), colestipol (colestid), and the like; 2) HMG-CoA reductase inhibitors such as atorvastatin (lipitor), cerivastatin (baycol), fluvastatin (lescol), pravastatin (pravachol), simvastatin (zocor) and the like; 3) cholesterol absorption inhibitors such as ezetimibe, and the like; 4) CETP inhibitors such as torcetrapib, JTT 705, and the like; 5) PPARα-agonists such as beclofibrate, gemfibrozil (lopid), fenofibrate (lipidil), bezafibrate (bezalip), and the like; 6) lipoprotein synthesis inhibitors such as niacin, and the like; and 7) niacin receptor agonists such as nicotinic acid, and the like.

Also an object of the invention is the simultaneous, separate or sequential administration of a compound according to formula I and a therapeutically effective amount of a lipid lowering agent.

The use of a compound of formula I in the manufacture of a medicament for the treatment and prevention of dyslipidemias in a patient who is also receiving treatment with a lipid lowering agent, is also an object of the present invention.

It is a further preferred object to provide a therapeutically effective amount of a compound according to formula I for use in treatment or prevention of hypertension in combination or association with a therapeutically effective amount of an anti-hypertensive agent.

The term "anti-hypertensive agent" or "blood-pressure lowering agent" refers to compounds selected from the group consisting of 1) Angiotensin-converting Enzyme (ACE) Inhibitors including benazepril (lotensin), captopril (capoten), enalapril (vasotec), fosinopril (monopril), lisinopril (prinivil, zestril), moexipril (univasc), perindopril (coversum), quinapril (accupril), ramipril (altace), trandolapril (mavik), and the like; 2) Angiotensin II Receptor Antagonists including candesartan (atacand), eprosartan (teveten), irbesartan (avapro), losartan (cozaar), telmisartan (micadisc), valsartan (diovan), and the like; 3) Adrenergic Blockers (peripheral or central) such as the beta-adrenergic blockers including acebutolol (sectrol), atenolol (tenormin), betaxolol (kerlone), bisoprolol (zebeta), carteolol (cartrol), metoprolol (lopressor; toprol-XL), nadolol (corgard), penbutolol (levatol), pindolol (visken), propranolol (inderal), timolol (blockadren) and the like; alpha/beta adrenergic blockers including carvedilol (coreg), labetalol (normodyne), and the like; alpha-1 adrenergic blockers including prazosin (minipress), doxazosin (cardura), terazosin (hytrin), phenoxybenzamine (dibenzyline), and the like; peripheral adrenergic-neuronal blockers including guanadrel (hylorel), guanethidine (ismelin), reserpine (serpasil), and the like; alpha-2 adrenergic blockers including α-methyldopa (aldomet), clonidine (catapres), guanabenz (wytensin), guanfacine (tenex), and the like; 4) Blood Vessel Dilators (Vasodilators) including hydralazine (apresoline), minoxidil (lonitren), clonidine (catapres), and the like; 5) Calcium Channel Blockers including amlodipine (norvasc), felodipine (plendil), isradipine (dynacirc), nicardipine (cardine sr), nifedipine (procardia, adalat), nisoldipine (sular), diltiazem (cardizem), verapamil (isoptil), and the like; 6) Diuretics such as thiazides and thiazides-like agents, including hydrochlorothiazide (hydrodiuril, microzide), chlorothiazide (diuril), chlorthalidone (hygroton), indapamide (lozol), metolazone (mykrox), and the like; loop diuretics, such as bumetanide (bumex) and furosemide (lasix), ethacrynic acid (edecrin), torsemide (demadex), and the like; potassium-sparing diuretics including amiloride (midamor), triamterene (dyrenium), spironolactone (aldactone), and the tiamenidine (symcor) and the like; 7) Tyrosine Hydroxylase Inhibitors, including metyrosine (demser), and the like; 8) Neutral Endopeptidase Inhibitors, including BMS-186716 (omapatrilat), UK-79300 (candoxatril), ecadotril (sinorphan), BP-1137 (fasidotril), UK-79300 (sampatrilat) and the like; and 9) Endothelin Antagonists including tezosentan (RO0610612), A308165, and the like.

Also an object of the invention is the simultaneous, separate or sequential administration of a compound according to formula I and a therapeutically effective amount of a anti-hypertensive agent.

The use of a compound of formula I in the manufacture of a medicament for the treatment and prevention of hypertension in a patient who is also receiving treatment with an anti-hypertensive agent, is also an object of the present invention.

As described above, the compounds of formula I and their pharmaceutically acceptable salts possess valuable pharmacological properties. Specifically, it has been found that the compounds of the present invention are good histamine 3 receptor (H3R) antagonists and/or inverse agonists.

The following test was carried out in order to determine the activity of the compounds of formula (I).

### Binding assay with ³H-(R)α-methylhistamine

Saturation binding experiments were performed using HR3-CHO membranes prepared as described in Takahashi, K, Tokita, S., Kotani, H. (2003) J. Pharmacol. Exp. Therapeutics 307, 213-218.

An appropriate amount of membrane (60 to 80 µg protein/well) was incubated with increasing concentrations of ³H(R)α-Methylhistamine di-hydrochloride (0.10 to 10 nM). Non specific binding was determined using a 200 fold excess of cold (R)α-Methylhistamine dihydrobromide (500 nM final concentration). The incubation was carried out at room temperature (in deep-well plates shaking for three hours). The final volume in each well was 250 µl. The incubation was followed by rapid filtration on GF/B filters (pre-soaked with 100 µl of 0.5% PEI in Tris 50 mM shaking at 200 rpm for two hours). The filtration was made using a cell-harvester and the filter plates were then washed five times with ice cold washing buffer containing 0.5 M NaCl. After harvesting, the plates were dried at 55 °C for 60 min, then we added scintillation fluid (Microscint 40, 40 microl in each well) and the amount of radioactivity on the filter was determined in Packard top-counter after shaking the plates for two hours at 200 rpm at room temperature.

Binding Buffer: 50 mM Tris-HCl pH 7.4 and 5 mM MgCl₂x6H₂O pH 7.4. Washing Buffer: 50 mM Tris-HCl pH 7.4 and 5 mM MgCl₂x6H₂O and 0.5 M NaCl pH 7.4.

Indirect measurement of affinity of H3R inverse agonists: twelve increasing concentrations (ranging from 10 µM to 0.3 nM) of the selected compounds were always tested in competition binding experiments using membrane of the human H3R-CHO cell line. An appropriate amount of protein, e.g. approximately 500cpm binding of RAMH at Kd, were incubated for 1 hour at room temperature in 250 µl final volume in 96-well plates in presence of ³H(R)α-methylhistamine (1 nM final concentration = Kd). Non-specific binding was determined using a 200 fold excess of cold (R)α - methylhistamine dihydrobromide.

All compounds were tested at a single concentration in duplicate. Compounds that showed an inhibition of [³H]-RAMH by more than 50% were tested again to determine IC₅₀ in a serial dilution experiment, meaning concentrations were spanning 10 points starting from 4.6 x 10⁻⁶ M to 1.0 x 10⁻⁹ M. The dilution factor was 1/2.15 for the whole series. The concentration at which 50% inhibition of the radioligand ³H(R)α-methylhistamine is obtained (the IC₅₀) is determined from the linear regression of a plot of the logarithm of the concentration versus percent inhibition measured for the different concentrations. Ki's were calculated from IC₅₀ based on Cheng-Prusoff equation (Cheng, Y, Prusoff, WH (1973) Biochem Pharmacol 22, 3099-3108): Ki = IC50 / [1 + D/Kd] wherein D is the concentration of the radioligand and Kd is the binding constant for the radioligand binding to the receptor under the conditions used in the competition experiment.

The compounds of the present invention exhibit Kᵢ values within the range of about 1 nM to about 1000 nM, preferably of about 1 nM to about 100 nM, and more preferably of about 1 nM to about 50 nM. The following table shows measured values for some selected compounds of the present invention.

| | Kᵢ (nM) |
|---|---|
| Example 10 | 31.3 |
| Example 20 | 40.9 |
| Example 32 | 33.6 |
| Example 44 | 41.7 |
| Example 64 | 13.1 |
| Example 94 | 64.3 |

Demonstration of additional biological activities of the compounds of the present invention may be accomplished through in vitro, ex vivo, and in vivo assays that are well known in the art. For example, to demonstrate the efficacy of a pharmaceutical agent for the treatment of obesity-related disorders such as diabetes, Syndrome X, or atherosclerotic disease and related disorders such as hypertriglyceridemia and hypercholesteremia, the following assays may be used.

### Method for Measuring Blood Glucose Levels

db/db mice (obtained from Jackson Laboratories, Bar Harbor, ME) are bled (by either eye or tail vein) and grouped according to equivalent mean blood glucose levels. They are dosed orally (by gavage in a pharmaceutically acceptable vehicle) with the test compound once daily for 7 to 14 days. At this point, the animals are bled again by eye or tail vein and blood glucose levels are determined.

### Method for Measuring Triglyceride Levels

hApoAl mice (obtained from Jackson Laboratories, Bar Harbor, ME) are bled (by either eye or tail vein) and grouped according to equivalent mean serum triglyceride levels. They are dosed orally (by gavage in a pharmaceutically acceptable vehicle) with the test compound once daily for 7 to 14 days. The animals are then bled again by eye or tail vein, and serum triglyceride levels are determined.

### Method for Measuring HDL-Cholesterol Levels

To determine plasma HDL-cholesterol levels, hApoAl mice are bled and grouped with equivalent mean plasma HDL-cholesterol levels. The mice are orally dosed once daily with vehicle or test compound for 7 to 14 days, and then bled on the following day. Plasma is analyzes for HDL-cholesterol.

The compounds of formula I and their pharmaceutically acceptable salts and esters can be used as medicaments, e.g. in the form of pharmaceutical preparations for enteral, parenteral or topical administration. They can be administered, for example, perorally, e.g. in the form of tablets, coated tablets, dragées, hard and soft gelatine capsules, solutions, emulsions or suspensions, rectally, e.g. in the form of suppositories, parenterally, e.g. in the form of injection solutions or infusion solutions, or topically, e.g. in the form of ointments, creams or oils.

The production of the pharmaceutical preparations can be effected in a manner which will be familiar to any person skilled in the art by bringing the described compounds of formula I and their pharmaceutically acceptable, into a galenical administration form together with suitable, non-toxic, inert, therapeutically compatible solid or liquid carrier materials and, if desired, usual pharmaceutical adjuvants.

Suitable carrier materials are not only inorganic carrier materials, but also organic carrier materials. Thus, for example, lactose, corn starch or derivatives thereof, talc, stearic acid or its salts can be used as carrier materials for tablets, coated tablets, dragées and hard gelatine capsules. Suitable carrier materials for soft gelatine capsules are, for example, vegetable oils, waxes, fats and semi-solid and liquid polyols (depending on the nature of the active ingredient no carriers are, however, required in the case of soft gelatine capsules). Suitable carrier materials for the production of solutions and syrups are, for example, water, polyols, sucrose, invert sugar and the like. Suitable carrier materials for injection solutions are, for example, water, alcohols, polyols, glycerol and vegetable oils. Suitable carrier materials for suppositories are, for example, natural or hardened oils, waxes, fats and semi-liquid or liquid polyols. Suitable carrier materials for topical preparations are glycerides, semi-synthetic and synthetic glycerides, hydrogenated oils, liquid waxes, liquid paraffins, liquid fatty alcohols, sterols, polyethylene glycols and cellulose derivatives.

Usual stabilizers, preservatives, wetting and emulsifying agents, consistency-improving agents, flavour-improving agents, salts for varying the osmotic pressure, buffer substances, solubilizers, colorants and masking agents and antioxidants come into consideration as pharmaceutical adjuvants.

The dosage of the compounds of formula I can vary within wide limits depending on the disease to be controlled, the age and the individual condition of the patient and the mode of administration, and will, of course, be fitted to the individual requirements in each particular case. For adult patients a daily dosage of about 1 mg to about 1000 mg, especially about 1 mg to about 100 mg, comes into consideration. Depending on the dosage it is convenient to administer the daily dosage in several dosage units.

The pharmaceutical preparations conveniently contain about 0.1-500 mg, preferably 0.5-100 mg, of a compound of formula I.

The following examples serve to illustrate the present invention in more detail. They are, however, not intended to limit its scope in any manner.

### Examples

### Intermediates

### Intermediate 1

### 2-(3-Piperidin-1-yl-propoxy)-4,5,6,7-tetrahydro-thieno[2,3-c]pyridine

### a) Step 1: 6-Trityl-5,6,7,7a-tetrahydro-4H-thieno[2,3-c]pyridin-2-one

To a solution of 10 g (26.3 mmol) 6-trityl-4,5,6,7-tetrahydro-thieno[2,3-c]pyridine (as prepared in WO 2001/032655) in 40 mL THF was added butyl lithium (78.9 mmol, 31.56 mL) at -20 °C. The mixture was stirred for 2 h at room temperature and then cooled to -40 °C, then a solution of tri-n-butyl borate (21.29 mL, 78.9 mmol) in 20 mL THF was added dropwise. The mixture was stirred for 2 h at room temperature. After the solution was cooled to -60 °C, 30% H₂O₂ (14 mL, 118.4 mmol) was added and allowed to stir over night at room temperature. Water was added and the reaction solution was stirred for 5 minutes and then extracted with dichloromethane. After removing the solvent 9 g of crude 6-trityl-5,6,7,7a-tetrahydro-4H-thieno[2,3-c]pyridin-2-one was obtained (89% yield).

### b) Step 2: 2-(3-Piperidin-1-yl-propoxy)-6-trityl-4,5,6,7-tetrahydro-thieno[2,3-c]pyridine

A mixture of 10 g (25.2 mmol) 6-trityl-5,6,7,7a-tetrahydro-4H-thieno[2,3-c]pyridin-2-one, 5.8 g (34 mmol) 1-(3-chloropropyl)-piperidine (commercially available) and 20.5 g (63 mmol) Cs₂CO₃ in 200 mL of DMF solvent were heated to 80 °C for 10 h. After removal of the solvent, the residue was purified with column chromatograph to give 11.5 g of pure product as brown oil. (84% yield).

### c) Step 3: 2-(3-Piperidin-1-yl-propoxy)-4,5,6,7-tetrahydro-thieno[2,3-c]pyridine

A mixture of 9 g (0.172 mmol) 2-(3-piperidin-1-yl-propoxy)-6-trityl-4,5,6,7-tetrahydro-thieno[2,3-c]pyridine and 145 mL (1.89 mol) TFA in 290 mL of DCM was stirred at room temperature for 1 h. After evaporation under vacuo, the residue was dissolved in water, adjusted to basic pH with aqueous NH₃H₂O and extracted with DCM. The organic phase was dried and concentrated to give 4 g of the product as a brown oil (83% yield).

### Intermediate 2

### 2-(3-Pyrrolidin-1-yl-propoxy)-4,5,6,7-tetrahydro-thieno[2,3-c]pyridine

In analogy to the procedure described for the synthesis of 2-(3-piperidin-1-yl-propoxy)-4,5,6,7-tetrahydro-thieno[2,3-c]pyridine (intermediate 1) the title compound was prepared from 6-trityl-5,6,7,7a-tetrahydro-4H-thieno[2,3-c]pyridin-2-one (intermediate 1, step 1) and 1-(3-chloro-propyl)-pyrrolidine (commercially available). The trityl-group was subsequently cleaved with TFA.

### Intermediate 3

### 2-(1-Isobutyl-piperidin-4-yloxy)-4,5,6,7-tetrahydro-thieno[2,3-c]pyridine

In analogy to the procedure described for the synthesis of 2-(3-piperidin-1-yl-propoxy)-4,5,6,7-tetrahydro-thieno[2,3-c]pyridine (intermediate 1) the title compound was prepared from 6-trityl-5,6,7,7a-tetrahydro-4H-thieno[2,3-c]pyridin-2-one (intermediate 1, step 1) and 4-bromo-1-isobutyl-piperidine (commercially available). The trityl-group was subsequently cleaved with TFA.

### Intermediate 4

### 2-(1-Isopropyl-piperidin-4-yloxy)-4,5,6,7-tetrahydro-thieno[2,3-c]pyridine

In analogy to the procedure described for the synthesis of 2-(3-piperidin-1-yl-propoxy)-4,5,6,7-tetrahydro-thieno[2,3-c]pyridine (intermediate 1) the title compound was prepared from 6-trityl-5,6,7,7a-tetrahydro-4H-thieno[2,3-c]pyridin-2-one (intermediate 1, step 1) and 4-bromo-1-isopropyl-piperidine (commercially available). The trityl-group was subsequently cleaved with TFA.

### Intermediate 5

### 2-(1-Isopropyl-pyrrolidin-3-yloxy)-4,5,6,7-tetrahydro-thieno[2,3-c]pyridine

In analogy to the procedure described for the synthesis of 2-(3-piperidin-1-yl-propoxy)-4,5,6,7-tetrahydro-thieno[2,3-c]pyridine (intermediate 1) the title compound was prepared from 6-trityl-5,6,7,7a-tetrahydro-4H-thieno[2,3-c]pyridin-2-one (intermediate 1, step 1) and 3-bromo-1-isopropyl-pyrrolidine (commercially available). The trityl-group was subsequently cleaved with TFA.

### Intermediate 6

### 2-(3-Morpholin-4-yl-propoxy)-4,5,6,7-tetrahydro-thieno[2,3-c]pyridine

In analogy to the procedure described for the synthesis of 2-(3-piperidin-1-yl-propoxy)-4,5,6,7-tetrahydro-thieno[2,3-c]pyridine (intermediate 1) the title compound was prepared from 6-trityl-5,6,7,7a-tetrahydro-4H-thieno[2,3-c]pyridin-2-one (intermediate 1, step 1) and 4-(3-chloro-propyl)-morpholine (commercially available). The trityl-group was subsequently cleaved with TFA.

### Intermediate 7

### Dimethyl-[2-(4,5,6,7-tetrahydro-thieno[2,3-c]pyridin-2-yloxy)-ethyl]-amine

In analogy to the procedure described for the synthesis of 2-(3-piperidin-1-yl-propoxy)-4,5,6,7-tetrahydro-thieno[2,3-c]pyridine (intermediate 1) the title compound was prepared from 6-trityl-5,6,7,7a-tetrahydro-4H-thieno[2,3-c]pyridin-2-one (intermediate 1, step 1) and diethylamino ethyl chloride (commercially available). The trityl-group was subsequently cleaved with TFA.

### Intermediate 8

### 4-Methoxy-piperidine-1-carbonyl chloride

To a stirred solution of triphosgene (7.4 g, 0.025 mmol) in dichloromethane (200 mL) at 0 °C was added dropwise a solution of 4-methoxy-piperidine hydrochloride salt (10 g, 0.066 mmol) and pyridine (10.9 g, 0.139 mmol) in dichloromethane (100 mL). The mixture was stirred over night and filtered through a silica pad, eluted with dichloromethane and evaporated. After trituration with diisopropyl ether, the mixture was used without purification.

### Intermediate 9

### N-butylcarbamoyl chloride

In analogy to the procedure described for the synthesis of 4-methoxy-piperidine-1-carbonyl chloride the title compound was prepared from triphosgene and butylamine (commercially available).

### Example 1

### Morpholin-4-yl-[2-(3-piperidin-1-yl-propoxy)-4,7-dihydro-5H-thieno[2,3-c]pyridin-6-yl] -methanone

A mixture of 100 mg (0.357 mmol) 2-(3-piperidin-1-yl-propoxy)-4,5,6,7-tetrahydro-thieno[2,3-c]pyridine (intermediate 1), 64 mg (0.428 mmol) of morpholine-4-carbonyl chloride (commercially available) and 43 mg (0.428 mmol) of triethyl amine in 10 mL DCM was stirred at room temperature overnight. After concentration in vacuo, the residue was purified by HPLC to give 52.3 mg of the product as white powder (37.2% yield). MS (m/e): 394.2 (MH⁺).

In analogy to the procedure described for the synthesis of morpholin-4-yl-[2-(3-piperidin-1-yl-propoxy)-4,7-dihydro-5H-thieno[2,3-c]pyridin-6-yl] -methanone (example 1) further 6;7- thieno[2,3-c]pyridine derivatives have been synthesized from their respective starting materials mentioned in table 1. The examples are shown in table 1 and comprise example 2- example 101.

**Table 1**

| No | MW | Name | Starting materials | MW found [MH⁺] |
|---|---|---|---|---|
| 2 | 391.58 | piperidin-1-yl-[2-(3-piperidin-1-yl-propoxy)-4,7-dihydro-5*H*-thieno[2,3-c]pyridin-6-yl]-methanone | 2-(3-piperidin-1-yl-propoxy)-4,5,6,7-tetrahydro-thieno[2,3-c]pyridine (intermediate 1) and piperidinecarbonyl chloride (commercially available) | 392.3 |
| 3 | 406.59 | (4-methyl-piperazin-1-yl)-[2-(3-piperidin-1-yl-propoxy)-4,7-dihydro-5*H*-thieno[2,3-*c*]pyridin-6-yl]-methanone | 2-(3-piperidin-1-yl-propoxy)-4,5,6,7-tetrahydro-thieno[2,3-c]pyridine (intermediate 1) and 4-methylpiperazine-1-carbonyl chloride (commercially available) | 407.1 |
| 4 | 377.55 | [2-(3-piperidin-1-yl-propoxy)-4,7-dihydro-5*H*-thieno[2,3-c]pyridin-6-yl]-pyrrolidin-1-yl-methanone | 2-(3-piperidin-1-yl-propoxy)-4,5,6,7-tetrahydro-thieno[2,3-c]pyridine (intermediate 1) and 1-pyrrolidinecarbonyl chloride (commercially available) | 378.2 |
| 5 | 421.6 | (4-methoxy-piperidin-1-yl)-[2-(3-piperidin-1-yl-propoxy)-4,7-dihydro-5*H*-thieno[2,3-*c*]pyridin-6-yl]-methanone | 2-(3-piperidin-1-yl-propoxy)-4,5,6,7-tetrahydro-thieno[2,3-c]pyridine (intermediate 1) and 4-methoxy-piperidine-1-carbonyl chloride (intermediate 8) | 422.1 |
| 6 | 482.69 | [2-(3-piperidin-1-yl-propoxy)-4,7-dihydro-5*H*-thieno[2,3-*c*]pyridin-6-yl]-(4-o-tolyl-piperazin-1-yl)-methanone | 2-(3-piperidin-1-yl-propoxy)-4,5,6,7-tetrahydro-thieno[2,3-c]pyridine (intermediate 1) and 4-o-tolyl-piperazine-1-carbonyl chloride (WO 96/02525 A1) | 483.1 |
| 7 | 364.55 | 3-methyl-1-[2-(3-piperidin-1-yl-propoxy)-4,7-dihydro-5*H*-thieno[2,3-*c*]pyridin-6-yl]-butan-1-one | 2-(3-piperidin-1-yl-propoxy)-4,5,6,7-tetrahydro-thieno[2,3-c]pyridine (intermediate 1) and 3-methyl-butyryl chloride (commercially available) | 365.2 |
| 8 | 378.58 | 3,3-dimethyl-1-[2-(3-piperidin-1-yl-propoxy)-4,7-dihydro-5*H*-thieno[2,3-c]pyridin-6-yl]-butan-1-one | 2-(3-piperidin-1-yl-propoxy)-4,5,6,7-tetrahydro-thieno[2,3-c]pyridine (intermediate 1) and 3,3-dimethyl-butyryl chloride (commercially available) | 379.2 |
| 9 | 414.57 | (4-methoxy-phenyl)-[2-(3-piperidin-1-yl-propoxy)-4,7-dihydro-5*H*-thieno[2,3-*c*]pyridin-6-yl]-methanone | 2-(3-piperidin-1-yl-propoxcy)-4,5,6,7-tetrahydro-thieno[2,3-c]pyridine (intermediate 1) and 4-methoxy-benzoyl chloride (commercially available) | 415.2 |
| 10 | 402.53 | (3-fluoro-phenyl)-[2-(3-piperidin-1-yl-propoxy)-4,7-dihydro-5*H*-thieno[2,3-*c*]pyridin-6-yl]-methanone | 2-(3-piperidin-1-yl-propoxy)-4,5,6,7-tetrahydro-thieno[2,3-c]pyridine (intermediate 1) and 3-fluoro-benzoyl chloride (commercially available) | 403.1 |
| 11 | 418.99 | (3-chloro-phenyl)-[2-(3-piperidin-1-yl-propoxy)-4,7-dihydro-5*H*-thieno[2,3-*c*]pyridin-6-yl]-methanone | 2-(3-piperidin-1-yl-propoxy)-4,5,6,7-tetrahydro-thieno[2,3-c]pyridine (intermediate 1) and 3-chloro-benzoyl chloride (commercially available) | 419.0 |
| 12 | 358.52 | 6-methanesulfonyl-2-(3-piperidin-1-yl-propoxy)-4,5,6,7-tetrahydro-thieno[2,3-*c*]pyridine | 2-(3-piperidin-1-yl-propoxy)-4,5,6,7-tetrahydro-thieno[2,3-c]pyridine (intermediate 1) and methanesulfonyl chloride (commercially available) | 359.1 |
| 13 | 438.59 | 6-(3-fluoro-benzenesulfonyl)-2-(3-piperidin-1-yl-propoxy)-4,5,6,7-tetrahydro-thieno[2,3-*c*]pyridine | 2-(3-piperidin-1-yl-propoxy)-4,5,6,7-tetrahydro-thieno[2,3-c]pyridine (intermediate 1) and 3-fluoro-benzenesulfonyl chloride (commercially available) | 439.1 |
| 14 | 434.62 | 2-(3-piperidin-1-yl-propoxy)-6-(toluene-4-sulfonyl)-4,5,6,7-tetrahydro-thieno[2,3-*c*]pyridine | 2-(3-piperidin-1-yl-propoxy)-4,5,6,7-tetrahydro-thieno[2,3-c]pyridine (intermediate 1) and 4-methyl-benzenesulfonyl chloride (commercially available) | 435.1 |
| 15 | 434.62 | 2-(3-piperidin-1-yl-propoxy)-6-(toluene-2-sulfonyl)-4,5,6,7-tetrahydro-thieno[2,3-*c*]pyridine | 2-(3-piperidin-1-yl-propoxy)-4,5,6,7-tetrahydro-thieno[2,3-c]pyridine (intermediate 1) and 2-methyl-benzenesulfonyl chloride (commercially available) | 435.1 |
| 16 | 448.65 | 6-(4-ethyl-benzenesulfonyl)-2-(3-piperidin-1-yl-propoxy)-4,5,6,7-tetrahydro-thieno[2,3-*c*]pyridine | 2-(3-piperidin-1-yl-propoxy)-4,5,6,7-tetrahydro-thieno[2,3-c]pyridine (intermediate 1) and 4-ethyl-benzenesulfonyl chloride (commercially available) | 449.2 |
| 17 | 476.7 | 6-(4-tert-butyl-benzenesulfonyl)-2-(3-piperidin-1-yl-propoxy)-4,5,6,7-tetrahydro-thieno[2,3-*c*]pyridine | 2-(3-piperidin-1-yl-propoxy)-4,5,6,7-tetrahydro-thieno[2,3-c]pyridine (intermediate 1) and 4-tert-butyl-benzenesulfonyl chloride (commercially available) | 477.2 |
| 18 | 420.6 | 6-benzenesulfonyl-2-(3-piperidin-1-yl-propoxy)-4,5,6,7-tetrahydro-thieno [2,3-*c*] pyridine | 2-(3-piperidin-1-yl-propoxy)-4,5,6,7-tetrahydro-thieno[2,3-c]pyridine (intermediate 1) and benzenesulfonyl chloride (commercially available) | 421.1 |
| 19 | 462.68 | 6-(4-isopropyl-benzenesulfonyl)-2-(3-piperidin-1-yl-propoxy)-4,5,6,7-tetrahydro-thieno[2,3-*c*]pyridine | 2-(3-piperidin-1-yl-propoxy)-4,5,6,7-tetrahydro-thieno[2,3-c]pyridine (intermediate 1) and 4-isopropyl-benzenesulfonyl chloride (commercially available) | 463.1 |
| 20 | 364.55 | 3,3-dimethyl-1-[2-(3-pyrrolidin-1-yl-propoxy)-4,7-dihydro-5*H*-thieno[2,3-*c*]pyridin-6-yl]-butan-1-one | 2-(3-pyrrolidin-1-yl-propoxy)-4,5,6,7-tetrahydro-thieno[2,3-c]pyridine (intermediate 2) and 3,3-dimethyl-butyryl chloride (commercially available) | 365.2 |
| 21 | 404.96 | (3-chloro-phenyl)-[2-(3-pyrrolidin-1-yl-propoxy)-4,7-dihydro-5*H*-thieno[2,3-*c*]pyridin-6-yl]-methanone | 2-(3-pyrrolidin-1-yl-propoxy)-4,5,6,7-tetrahydro-thieno[2,3-c]pyridine (intermediate 2) and 3-chloro-benzoyl chloride (commercially available) | 405.1 |
| 22 | 404.96 | (2-chloro-phenyl)-[2-(3-pyrrolidin-1-yl-propoxy)-4,7-dihydro-5*H*-thieno[2,3-*c*]pyridin-6-yl]-methanone | 2-(3-pyrrolidin-1-yl-propoxy)-4,5,6,7-tetrahydro-thieno[2,3-c]pyridine (intermediate 2) and 2-chloro-benzoyl chloride (commercially available) | 405.2 |
| 23 | 438.51 | [2-(3-pyrrolidin-1-yl-propoxy)-4,7-dihydro-5*H*-thieno[2,3-*c*]pyridin-6-yl]-(4-trifluoromethyl-phenyl)-methanone | 2-(3-pyrrolidin-1-yl-propoxy)-4,5,6,7-tetrahydro-thieno[2,3-c]pyridine (intermediate 2) and 4-trifluoromethyl benzoyl chloride (commercially available) | 439.2 |
| 24 | 370.51 | phenyl-[2-(3-pyrrolidin-1-yl-propoxy)-4,7-dihydro-5*H*-thieno[2,3-*c*]pyridin-6-yl]-methanone | 2-(3-pyrrolidin-1-yl-propoxy)-4,5,6,7-tetrahydro-thieno[2,3-c]pyridine (intermediate 2) and benzoyl chloride (commercially available) | 371.3 |
| 25 | 400.54 | (3-methoxy-phenyl)-[2-(3-pyrrolidin-1-yl-propoxy)-4,7-dihydro-5*H*-thieno[2,3-*c*]pyridin-6-yl]-methanone | 2-(3-pyrrolidin-1-yl-propoxy)-4,5,6,7-tetrahydro-thieno[2,3-c]pyridine (intermediate 2) and 3-methoxy-benzoyl chloride (commercially available) | 401.3 |
| 26 | 379.52 | morpholin-4-yl-[2-(3-pyrrolidin-1-yl-propoxy)-4,7-dihydro-5*H*-thieno[2,3-*c*]pyridin-6-yl]-methanone | 2-(3-pyrrolidin-1-yl-propoxy)-4,5,6,7-tetrahydro-thieno[2,3-c]pyridine (intermediate 2) and 4-morpholinyl carbonyl chloride (commercially available) | 380.2 |
| 27 | 363.52 | pyrrolidin-1-yl-[2-(3-pyrrolidin-1-yl-propoxy)-4,7-dihydro-5*H*-thieno[2,3-*c*]pyridin-6-yl]-methanone | 2-(3-pyrrolidin-1-yl-propoxy)-4,5,6,7-tetrahydro-thienc[2,3-c]pyridine (intermediate 2) and 1-pyrrolidinecarbonyl chloride (commercially available) | 364.2 |
| 28 | 377.55 | piperidin-1-yl-[2-(3-pyrrolidin-1-yl-propoxy)-4,7-dihydro-5*H*-thieno[2,3-*c*]pyridin-6-yl]-methanone | 2-(3-pyrrolidin-1-yl-propoxy)-4,5,6,7-tetrahydro-thieno[2,3-c]pyridine (intermediate 2) and piperidinecarbonyl chloride (commercially available) | 378.3 |
| 29 | 392.57 | (4-methyl-piperazin-1-yl)-[2-(3-pyrrolidin-1-yl-propoxy)-4,7-dihydro-5*H*-thieno[2,3-*c*]pyridin-6-yl]-methanone | 2-(3-pyrrolidin-1-yl-propoxy)-4,5,6,7-tetrahydro-thieno[2,3-c]pyridine (intermediate 2) and 4-methylpiperazine-1-carbonyl chloride (commercially available) | 393.1 |
| 30 | 468.66 | 2-(3-pyrrolidin-1-yl-propoxy)-4,7-dihydro-5*H*-thieno[2,3-*c*]pyridin-6-yl]-(4-*o*-tolyl-piperazin-1-yl)-methanone | 2-(3-pyrrolidm-1-yl-propoxy)-4,5,6,7-tetrahydro-thieno[2,3-c]pyridine (intermediate 2) and 4-*o*-tolyl-piperazine-1-carbonyl chloride (WO 96/02525 A1) | 469.1 |
| 31 | 344.5 | 6-methanesulfonyl-2-(3-pyrrolidin-1-yl-propoxy)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and methanesulfonyl chloride (commercially available) | 345.1 |
| 32 | 406.57 | 6-benzenesulfonyl-2-(3-pyrrolidin-1-yl-propoxy)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and benzenesulfonyl chloride (commercially available) | 407.1 |
| 33 | 420.6 | 2-(3-pyrrolidin-1-yl-propoxy)-6-(toluene-4-sulfonyl)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and 4-methyl-benzenesulfonyl chloride (commercially available) | 421.1 |
| 34 | 420.6 | 2-(3-pyrrolidin-1-yl-propoxy)-6-(toluene-2-sulfonyl)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and 2-methyl-benzenesulfonyl chloride (commercially available) | 421.1 |
| 35 | 420.6 | 2-(3-pyrrolidin-1-yl-propoxy)-6-(toluene-3-sulfonyl)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and 3-methyl-benzenesulfonyl chloride (commercially available) | 421.1 |
| 36 | 434.62 | 6-(4-ethyl-benzenesulfonyl)-2-(3-pyrrolidin-1-yl-propoxy)-4,5,6,7-tetrahydro-thieno[2,3-*c*] pyridine | 2-(3-pyrrolidin-1-yl-propoxy)-2-(3-pyrrolidin-1-yl-propoxy)-4,5,6,7-tetrahydro-thieno[2,3-c]pyridine (intermediate 2) and 4-ethyl-benzenesulfonyl chloride (commercially available) | 435 |
| 37 | 448.65 | 6-(4-isopropyl-benzenesulfonyl)-2-(3-pyrrohdin-1-yl-propoxy)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and 4-isopropyl-benzenesulfonyl chloride (commercially available) | 449.2 |
| 38 | 462.68 | 6-(4-tert-butyl-benzenesulfonyl)-2-(3-pyrrolidin-1-yl-propoxy)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and 4-tert-butyl-benzenesulfonyl chloride (commercially available) | 463.1 |
| 39 | 424.56 | 6-(3-fluoro-benzenesulfonyl)-2-(3-pyrrolidin-1-yl-propoxy)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and 3-fluoro-benzenesulfonyl chloride (commercially available) | 425.1 |
| 40 | 372.55 | 6-(propane-2-sulfonyl)-2-(3-pyrrolidin-1-yl-propoxy)-4,5,6,7-tetrahydro-thieno[2,3-*c*]pyridine | 2-(3-pyrrolidin-1-yl-propoxy)-4,5,6,7-tetrahydro-thieno[2,3-c]pyridine (intermediate 2) and propane-2-sulfonyl chloride (commercially available) | 373.0 |
| 41 | 398.57 | [2-(1-isobutyl-piperidin-4-yloxy)-4,7-dihydro-5*H*-thieno[2,3-*c*]pyridin-6-yl]-phenyl-methanone | 2-(1-isobutyl-piperidin-4-yloxy)-4,5,6,7-tetrahydro-thieno[2,3-c]pyridine(Intermediate 3) and benzoyl chloride (commercially available) | 399.3 |
| 42 | 364.55 | 1-[2-(1-isobutyl-piperidin-4-yloxy)-4,7-dihydro-5*H*-thieno[2,3-*c*]pyridin-6-yl]-butan-1-one | 2-(1-isobutyl-piperidin-4-yloxy)-4,5,6,7-tetrahydro-thieno[2,3-c]pyridine (Intermediate 3) and butyryl chloride (commercially available) | 365.3 |
| 43 | 378.58 | 1-[2-(1-isobutyl-piperidin-4-yloxy)-4,7-dihydro-5*H*-thieno[2,3- *c*]pyridin-6-yl]-pentan-1-one | 2-(1-isobutyl-piperidin-4-yloxy)-4,5,6,7-tetrahydro-thieno[2,3-c]pyridine (Intermediate 3) and pentanoyl chloride (commercially available) | 379.3 |
| 44 | 392.6 | 1-[2-(1-isobutyl-piperidin-4-yloxy)-4,7-dihydro-5*H*-thieno[2,3-*c*]pyridin-6-yl]-3,3-dimethyl-butan-1-one | 2-(1-isobutyl-piperidin-4-yloxy)-4,5,6,7-tetrahydro-thieno[2,3-c]pyridine (Intermediate 3) and 3,3-dimethyl-butyryl chloride (commercially available) | 393.3 |
| 45 | 428.59 | [2-(1-isobutyl-piperidin-4-yloxy)-4,7-dihydro-5*H*-thieno[2,3-*c*]pyridin-6-yl]-(3-methoxy-phenyl)-methanone | 2-(1-isobutyl-piperidin-4-yloxy)-4,5,6,7-tetrahydro-thieno[2,3-c]pyridine (Intermediate 3) and 3-methoxy-benzoyl chloride (commercially available) | 429.2 |
| 46 | 428.59 | [2-(1-isobutyl-piperidin-4-yloxy)-4,7-dihydro-5*H*-thieno[2,3-*c*]pyridin-6-yl]-(4-methoxy-phenyl)-methanone | 2-(1-isobutyl-piperidin-4-yloxy)-4,5,6,7-tetrahydro-thieno[2,3-c]pyridine (Intermediate 3) and 4-methoxy-benzoyl chloride (commercially available) | 429.2 |
| 47 | 416.56 | (4-fluoro-phenyl)-[2-(1-isobutyl-piperidin-4-yloxy)-4,7-dihydro-5*H*-thieno[2,3-*c*]pyridin-6-yl]-methanone | 2-(1-isobutyl-piperidin-4-yloxy)-4,5,6,7-tetrahydro-thieno[2,3-c]pyridine (Intermediate 3) and 4-fluoro-benzoyl chloride (commercially available) | 417.2 |
| 48 | 416.56 | (3-fluoro-phenyl)-[2-(1-isobutyl-piperidin-4-yloxy)-4,7-dihydro-5H-thieno[2,3-*c*]pyridin-6-yl]-methanone | 2-(1-isobutyl-piperidin-4-yloxy)-4,5,6,7-tetrahydro-thieno[2,3-c]pyridine (Intermediate 3) and 3-fluoro-benzoyl chloride (commercially available) | 417.1 |
| 49 | 466.57 | [2-(1-isobutyl-piperidin-4-yloxy)-4,7-dihydro-5*H*-thieno[2,3-*c*]pyridin-6-yl]-(4-trifluoromethyl-phenyl)-methanone | 2-(1-isobutyl-piperidin-4-yloxy)-4,5,6,7-tetrahydro-thieno[2,3-c]pyridine (Intermediate 3) and 4-trifluoromethyl-benzoyl chloride (commercially available) | 467.0 |
| 50 | 433.01 | (3-chloro-phenyl)-[2-(1-isobutyl-piperidin-4-yloxy)-4,7-dihydro-5*H*-thieno[2,3-*c*]pyridin-6-yl]-methanone | 2-(1-isobutyl-piperidin-4-yloxy)-4,5,6,7-tetrahydro-thieno[2,3-c]pyridine (Intermediate 3) and 3-chloro-benzoyl chloride (commercially available) | 433.0 |
| 51 | 433.01 | (2-chloro-phenyl)-[2-(1-isobutyl-piperidin-4-yloxy)-4,7-dihydro-5*H*-thieno[2,3-*c*]pyridin-6-yl]-methanone | 2-(1-isobutyl-piperidin-4-yloxy)-4,5,6,7-tetrahydro-thieno[2,3-c]pyridine (Intermediate 3) and 2-chloro-benzoyl chloride (commercially available) | 433.0 |
| 52 | 407.58 | [2-(1-isobutyl-piperidin-4-yloxy)-4,7-dihydro-5*H*-thieno[2,3-*c*]pyridin-6-yl]-morpholin-4-yl-methanone | 2-(1-isobutyl-piperidin-4-yloxy)-4,5,6,7-tetrahydro-thieno[2,3-c]pyridine (Intermediate 3) and 4-morpholinyl carbonyl chloride (commercially available) | 408.2 |
| 53 | 393.59 | 2-(1-isobutyl-piperidin-4-yloxy)-4,7-dihydro-5*H*-thieno[2,3-*c*]pyridine-6-carboxylic acid diethylamide | 2-(1-isobutyl-piperidin-4-yloxy)-4,5,6,7-tetrahydro-thieno[2,3-c]pyridine (Intermediate 3) and diethyl carbamoyl chloride (commercially available) | 394.3 |
| 54 | 405.6 | [2-(1-isobutyl-piperidin-4-yloxy)-4,7-dihydro-5*H*-thieno[2,3-*c*]pyridin-6-yl]-piperidin-1-yl-methanone | 2-(1-isobutyl-piperidin-4-yloxy)-4,5,6,7-tetrahydro-thieno[2,3-c]pyridine (Intermediate 3) and piperidinecarbonyl chloride (commercially available) | 406.3 |
| 55 | 391.58 | [2-(1-isobutyl-piperidin-4-yloxy)-4,7-dihydro-5*H*-thieno[2,3-*c*]pyridin-6-yl] -pyrrolidin-1-yl-methanone | 2-(1-isobutyl-piperidin-4-yloxy)-4,5,6,7-tetrahydro-thieno[2,3-*c*]pyridine (Intermediate 3) and. 1-pyrrolidinecarbonyl chloride (commercially available) | 392.2 |
| 56 | 372.55 | 2-(1-isobutyl-piperidin-4-yloxy)-6-methanesulfonyl-4,5,6,7-tetrahydro-thieno[2,3-*c*]pyridine | 2-(1-isobutyl-piperidin-4-yloxy)-4,5,6,7-tetrahydro-thieno [2,3-c]pyridine (Intermediate 3) and methanesulfonyl chloride (commercially available) | 373.2 |
| 57 | 434.62 | 6-benzenesulfonyl-2-(1-isobutyl-piperidin-4-yloxy)-4,5,6,7-tetrahydro-thieno[2,3-*c*]pyridine | 2-(1-isobutyl-piperidin-4-yloxy)-4,5,6,7-tetrahydro-thieno[2,3-c]pyridine (Intermediate 3) and benzenesulfonyl chloride (commercially available) | 435.2 |
| 58 | 448.65 | 2-(1-isobutyl-piperidin-4-yloxy)-6-(toluene-4-sulfonyl)-4,5,6,7-tetrahydro-thieno[2,3- *c*]pyridine | 2-(1-isobutyl-piperidin-4-yloxy)-4,5,6,7-tetrahydro-thieno[2,3-c]pyridine (Intermediate 3) and 4-methyl-benzenesulfonyl chloride (commercially available) | 449.2 |
| 59 | 448.65 | 2-(1-isobutyl-piperidin-4-yloxy)-6-(toluene-2-sulfonyl)-4,5,6,7-tetrahydro-thieno[2,3-*c*]pyridine | 2-(1-isobutyl-piperidin-4-yloxy)-4,5,6,7-tetrahydro-thieno[2,3-c]pyridine (Intermediate 3) and 2-methyl-benzenesulfonyl chloride (commercially available) | 449.2 |
| 60 | 448.65 | 2-(1-isobutyl-piperidin-4-yloxy)-6-(toluene-3-sulfonyl)-4,5,6,7-tetrahydro-thieno[2,3-*c*]pyridine | 2-(1-isobutyl-piperidin-4-yloxy)-4,5,6,7-tetrahydro-thieno[2,3-c]pyridine (Intermediate 3) and 3-methyl-benzenesulfonyl chloride (commercially available) | 449.2 |
| 61 | 462.68 | 6-(4-ethyl-benzenesulfonyl)-2-(1-isobutyl-piperidin-4-yloxy)-4,5,6,7-tetrahydro-thieno [2,3-*c*]pyridine | 2-(1-isobutyl-piperidin-4-yloxy)-4,5,6,7-tetrahydro-thieno[2,3-c]pyridine (Intermediate 3) and 4-ethyl-benzenesulfonyl chloride (commercially available) | 463.2 |
| 62 | 452.61 | 6-(3-fluoro-benzenesulfonyl)-2-(1isobutyl-piperidin-4-yloxy)-4,5,6,7-tetrahydro-thieno[2,3-*c*]pyridine | 2-(1-isobutyl-piperidin-4-yloxy)-4,5,6,7-tetrahydro-thieno[2,3-*c*]pyridine (Intermediate 3) and 3-fluoro-benzenesulfonyl chloride (commercially available) | 453.2 |
| 63 | 350.52 | 1-[2-(1-isopropyl-piperidin-4-yloxy)-4,7-dihydro-5*H*-thieno[2,3-*c*]pyridin-6-yl]-butan-1- one | 2-(1-isopropyl-piperidin-4-yloxy)-4,5,6,7-tetrahydro-thieno[2,3-c]pyridine (intermediate 4) and butyryl chloride (commercially available) | 351.2 |
| 64 | 364.55 | 1-[2-(1-isopropyl-piperidin-4-yloxy)-4,7-dihydro-5*H*-thieno[2,3-*c*]pyridin-6-yl]-3-methyl-butan-1-one | 2-(1-isopropyl-piperidin-4-yloxy)-4,5,6,7-tetrahydro-thieno[2,3-c]pyridine (intermediate 4) and 3-methyl-butyryl chloride (commercially available) | 365.2 |
| 65 | 378.58 | 1-[2-(1-isopropyl-piperidin-4-yloxy)-4,7-dihydro-5*H*-thieno[2,3-*c*]pyridin-6-yl]-3,3-dimethyl-butan-1-one | 2-(1-isopropyl-piperidin-4-yloxy)-4,5,6,7-tetrahydro-thieno[2,3-c]pyridine thieno 2,3-c]pyndme (intermediate 4) and 3,3-dimethyl-butyryl chloride (commercially available) | 379.3 |
| 66 | 452.54 | [2-(1-isopropyl-piperidin-4-yloxy)-4,7-dihydro-5*H*-thieno[2,3-*c*]pyridin-6-yl]-(4-trifluoromethyl-phenyl)-methanone | 2-(1-isopropyl-piperidin-4-yloxy)-4,5,6,7-tetrahydro-thieno[2,3-c]pyridine (intermediate 4 and 4-trifluoromethyl benzoyl chloride (commercially available) | 453.3 |
| 67 | 418.99 | (3-chloro-phenyl)-[2-(1-isopropyl-piperidin-4-yloxy)-4,7-dihydro-5*H*-thieno[2,3-*c*]pyridin-6-yl]-methanone | 2-(1-isopropyl-piperidin-4-yloxy)-4,5,6,7-tetrahydro-thieno[2,3-c]pyridine 4) and 3-chloro-benzoyl chloride (commercially available) | 419.3 |
| 68 | 414.57 | [2-(1-isopropyl-piperidin-4-yloxy)-4,7-dihydro-5*H*-thieno[2,3-*c*]pyridin-6-yl]-(3-methoxy-phenyl)-methanone | 2-(1-isopropyl-piperidin-4-yloxy)-4,5,6,7-tetrahydro-thieno[2,3-*c*]pyridine (intermediate 4) and 3-methoxy-benzoyl chloride (commercially available) | 415.3 |
| 69 | 414.57 | [2-(1-isopropyl-piperidin-4-yloyy)-4,7-dihydro-5*H*-thieno[2,3-*c*]pyridin₋6₋yl]-(4- methoxy-phenyl)-methanone | 2-(1-isopropyl-piperidin-4-yloxy)-4,5,6,7-tetrahydro-thieno[2,3-c]pyridine (intermediate 4) and 4-methoxy-benzoyl chloride (commercially available) | 415.3 |
| 70 | 402.53 | (4-fluoro-phenyl)-[2-(1-isopropyl-piperidin-4-yloxy)-4,7-dihydro-5*H*-thieno[2,3-*c*]pyridin-6-yl]-methanone | 2-(1-isopropyl-piperidin-4-yloxy)-4,5,6,7-tetrahydro-thieno[2,3-c]pyridine (intermediate 4) and 4-fluoro-benzoyl chloride (commercially available) | 403.3 |
| 71 | 402.53 | (3-fluoro-phenyl)-[2-(1-isopropyl-piperidin-4-yloxy)-4,7-dihydro-5*H*-thieno[2,3-*c*]pyridin-6-yl]-methanone | 2-(1-isopropyl-piperidin-4-yloxy)-4,5,6,7-tetrahydro-thieno[2,3-c]pyridine thieno2,3-cpyridine (intermediate 4) and 3-fluoro-benzoyl chloride (commercially available) | 403.3 |
| 72 | 418.99 | (2-chloro-phenyl)-[2-(1-isopropyl-piperidin-4-thieno[2,3-c]pyridine yloxy)-4,7-dihydro-5*H*-thieno[2,3-*c*]pyridin-6-yl]-methanone | 2-(1-isopropyl-piperidin-4-yloxy)-4,5,6,7-tetrahydro-(intermediate 4) and 2-chloro-benzoyl chloride (commercially available) | 419.2 |
| 73 | 414.57 | [2-(1-isopropyl-piperidin-4-yloxy)-4,7-dihydro-5*H*-thieno[2,3-c]pyridin-6-yl]-(2-methoxy-phenyl)-methanone | 2-(1-isopropyl-piperidin-4-yloxy)-4,5,6,7-tetrahydro-thieno[2,3-*c*]pyridine (intermediate 4) and 2-methoxy-benzoyl chloride (commercially available) | 415.3 |
| 74 | 402.53 | (2-fluoro-phenyl)-[2-(1-isopropyl-piperidin-4-yloxy)-4,7-dihydro-5*H*-yl]-methanone | 2-(1-isopropyl-piperidin-4-yloxy)-4,5,6,7-tetrahydro-thieno[2,3-c]pyridine (intermediate 4) and (commercially available) | 403.3 |
| 75 | 393.55 | [2-(1-isopropyl-piperidin-4-yloxy)-4,7-dihydro-5*H*-thieno[2,3-*c*]pyridin-6-yl]-morpholin-4-yl-methanone | 2-(1-isopropyl-piperidin-4-yloxy)-4,5,6,7-tetrahydro-thieno[2,3-c]pyridine (intermediate 4) and 4-morpholinyl carbonyl chloride (commercially available) | 394.3 |
| 76 | 379.57 | 2-(1-isopropyl-piperidin-4-yloxy)-4,7-dihydro₋ 4-yloxy)-4,7-dihydro-5*H*-thieno[2,3-*c*]pyridine-6-carboxylic acid diethylamide | 2-(1-isopropyl-piperidin-4-yloxy) -4,5,6,7-tetrahydro-thieno[2,3-c]pyridine (intermediate 4) and diethyl carbamoyl chloride (commercially available) | 380.3 |
| 77 | 391.58 | [2-(1-isopropyl-piperidin-4-yloxy)-4,7-dihydro-5*H*-thieno[2,3-*c*]pyridin-6-yl]-piperidin-1-yl-methanone | 2-(1-isopropyl-piperidin-4-yloxy)-4,5,6,7-tetrahydro-thieno[2,3-c]pyridine (intermediate 4) and piperidinecarbonyl chloride (commercially available) | 392.3 |
| 78 | 337.49 | 2-(1-isopropyl-piperidin-4-yloxy)-4,7-dihydro-4-yloxy)-4,7-dihydro-5*H*-thieno[2,3-*c*]pyridine-6-carboacylic acid methylamide | 2-(1-isopropyl-piperidin-4-yloxy)-4,5,6,7-tetrahydro-thieno[2,3-c]pyridine (intermediate 4) and methyl isocyanate (commercially available) | 338.1 |
| 79 | 406.59 | [2-(1-isopropyl-piperidin-4-yloxy)-4,7-dihydro-5*H*-thieno[2,3-*c*]pyridin-6-yl]-(4-methyl-piperazin-1-yl)-methanone | 2-(1-isopropyl-piperidin-4-yloxy)-4,5,6,7-tetrahydro-thieno[2,3-c]pyridine (intermediate 4) and 4-methylpiperazine-1-carbonyl chloride (commercially available) | 407.1 |
| 80 | 351.51 | 2-(1-isopropyl-piperidin-4-yloxy)-4,7-dihydro-5*H*-thieno[2,3-*c*]pyridine-6-carboxylic acid ethylamide | 2-(1-isopropyl-piperidin-4-yloxy)-4,5,6,7-tetrahydro-thieno[2,3-c]pyridine (intermediate 4) and ethyl isocyanate (commercially available) | 352.1 |
| 81 | 365.54 | 2-(1-isopropyl-pipendin-4-yloxy)-4,7-dihydro-5*H*-thieno[2,3-*c*]pyridine-6-carboxylic acid isopropylamide | 2-(1-isopropyl-piperidin-4-yloxy)-4,5,6,7-tetrahydro-thieno[2,3-c]pyridine (intermediate 4) and isopropyl isocyanate (commercially available) | 366.1 |
| 82 | 379.57 | 2-(1-isopropyl-piperidin-4-yloxy)-4,7-dihydro-5*H*-thieno[2,3-*c*]pyridine-6-carboxylic acid butylamide | 2-(1-isopropyl-piperidin-4-yloacy)-4,5,6,7-tetrahydro-thieno[2,3-c]pyridine (intermediate 4) and *N*-butyl carbamoyl chloride (intermediate 9) | 380.2 |
| 83 | 482.69 | [2-(1-isopropyl-piperidin-4-yloxy)-4,7-dihydro-5*H*-thieno[2,3-*c*]pyridin-6-yl]-(4-*o*-tolyl-piperazin-1-yl)-methanone | 2-(1-isopropyl-piperidin-4-yloxy)-4,5,6,7-tetrahydro-thieno[2,3-c]pyridine (intermediate 4) and 4-*o*-tolyl-piperazine-1-carbonyl chloride (WO 96/02525 A1) | 483.1 |
| 84 | 358.52 | 2-(1-isopropyl-piperidin-4-yloxy)-6-methanesulfonyl-4,5,6,7-tetrahydro-thieno [2,3-*c*]pyridine | 2-(1-isopropyl-piperidin-4-yloxy)-4,5,6,7-tetrahydro-thieno[2,3-c]pyridine (intermediate 4) and methanesulfonyl chloride (commercially available) | 359.2 |
| 85 | 420.6 | 6-benzenesulfonyl-2-(1-isopropyl-piperidin-4-yloxy)-4,5,6,7-tetrahydro-thieno[2,3-*c*]pyridine | 2-(1-isopropyl-piperidin-4-yloxy)-4,5,6,7-tetrahydro-thieno[2,3-c]pyridine (intermediate 4) and benzenesulfonyl chloride (commercially available) | 421.2 |
| 86 | 434.62 | 2-(1-isopropyl-piperidin-4-yloxy)-6-(toluene-4-sulfonyl)-4,5,6,7-tetrahydro-thieno[2,3-*c*]pyridine | 2-(1-isopropyl-piperidin-4-yloxy)-4,5,6,7-tetrahydro-thieno[2,3-c]pyridine (intermediate 4) and 4-methyl-benzenesulfonyl chloride (commercially available) | 435.3 |
| 87 | 434.62 | 2-(1-isopropyl-piperidin-4-yloxy)-6-(toluene-2-sulfonyl)-4,5,6,7-tetrahydro-thieno[2,3-*c*]pyridine | 2-(1-isopropyl-piperidin-4-yloxy)-4,5,6,7-tetrahydro-thieno[2,3-c]pyridine (intermediate 4) and 2-methyl-benzenesulfonyl chloride (commercially available) | 435.3 |
| 88 | 434.62 | 2-(1-isopropyl-piperidin-4-yloxy)-6-(toluene-3-sulfonyl)-4,5,6,7-tetrahydro-thieno[2,3-*c*]pyridine | 2-(1-isopropyl-piperidin-4-yloxy)-4,5,6,7-tetrahydro-thieno[2,3-c]pyridine (intermediate 4) and 3-methyl-benzenesulfonyl chloride (commercially available) | 435.3 |
| 89 | 448.65 | 6-(4-ethyl-benzenesulfonyl)-2-(1-isopropyl-piperidin-4-yloxy)-4,5,6,7-tetrahydro-thieno[2,3-*c*]pyridine | 2-(1-isopropyl-piperidin-4-yloxy)-4,5,6,7-tetrahydro-thieno[2,3-c]pyridine (intermediate 4) and 4-ethyl-benzenesulfonyl chloride (commercially available) | 449.2 |
| 90 | 462.68 | 6-(4-isopropyl-benzenesulfonyl)-2-(1-isopropyl-piperidin-4-yloxy)-4,5,6,7-tetrahydro-thieno[2,3-*c*]pyridine | 2-(1-isopropyl-piperidin-4-yloxy)-4,5,6,7-tetrahydro-thieno[2,3-c]pyridine (intermediate 4) and 4-isopropyl-benzenesulfonyl chloride (commercially available) | 463.3 |
| 91 | 476.7 | 6-(4-tert-butyl-benzenesulfonyl)-2-(1-isopropyl-piperidin-4-yloxy)-4,5,6,7-tetrahydro-thieno [2,3-*c*]pyridine | 2-(1-isopropyl-piperidin-4-yloxy)-4,5,6,7-tetrahydro-thieno[2,3-c]pyridine (intermediate 4) and 4-tert-butyl-benzenesulfonyl chloride (commercially available) | 477.3 |
| 92 | 438.59 | 6-(3-fluoro-benzenesulfonyl)-2-(1-isopropyl-piperidin-4-yloxy)-4,5,6,7-tetrahydro-thieno [2,3-*c*]pyridine | 2-(1-isopropyl-piperidin-4-yloxy)-4,5,6,7-tetrahydro-thieno[2,3-c]pyridine (intermediate 4) and 3-fluoro-benzenesulfonyl chloride (commercially available) | 439.3 |
| 93 | 386.58 | 2-(1-isopropyl-piperidin-4-yloxy)-6-(propane-2-sulfonyl)-4,5,6,7-tetrahydro-thieno[2,3-*c*]pyridine | 2-(1-isopropyl-piperidin-4-yloxy)-4,5,6,7-tetrahydro-thieno[2,3-c]pyridine (intermediate 4) and (intermediate 4) and chloride propane-2-sulfonyl (commercially available) | 387.1 |
| 94 | 420.6 | 2-(1-isopropyl-pyrrolidin-3-yloacy)-6-(toluene-4-sulfonyl)-4,5,6,7-tetrahydro-thieno[2,3-*c*]pyridine | 2-(1-isopropyl-pyrrolidin-3-yloxy)-4,5,6,7-tetrahydro-thieno[2,3-c]pyridine (intermediate 5) and 4-methyl-benzenesulfonyl chloride (commercially available) | 421.0 |
| 95 | 462.68 | 6-(4-tert-butyl-benzenesulfonyl)-2-(1-isopropyl-pyrrolidin-3-yloxy)-4,5,6,7-tetrahydro-thieno[2,3-*c*]pyridine | 2-(1-isopropyl-pyrrolidin-3-yloxy)-4,5,6,7-tetrahydro-thieno[2,3-c]pyridine (intermediate 5) and 4-*tert*-butyl-benzenesulfonyl chloride (commercially available) | 463.0 |
| 96 | 448.65 | 6-(4-isopropyl-benzenesulfonyl)-2-(1-isopropyl-pyrrolidin-3-yloxy)-4,5,6,7-tetrahydro-thieno[2,3-*c*]pyridine | 2-(1-isopropyl-pyrrolidin-3-yloxy)-4,5,6,7-tetrahydro-thieno[2,3-c]pyridine (intermediate 5) and 4-isopropyl-benzenesulfonyl chloride (commercially available) | 449.0 |
| 97 | 420.96 | (4-chloro-phenyl)-[2-(3-morpholin-4-yl-propoxy)-4,7-dihydro-5*H*-thieno[2,3-*c*]pyridin-6-yl]-methanone | 2-(3-morpholin-4-yl-propoxy)-4,5,6,7-tetrahydro-thieno[2,3-c]pyridine (intermediate 6) and 4-chloro-benzoyl chloride (commercially available) | 421.1 |
| 98 | 420.96 | (3-chloro-phenyl)-[2-(3-morpholin-4-yl-propoxy)-4,7-dihydro-5*H*-thieno[2,3-*c*]pyridin-6-yl]-methanone | 2-(3-morpholin-4-yl-propoxy)-4,5,6,7-tetrahydro-thieno[2,3-c]pyridine (intermediate 6) and 3-chloro-benzoyl chloride (commercially available) | 421.1 |
| 99 | 380.53 | dimethyl-{2-[6-(toluene-2-sulfonyl)-4,5,6,7-tetrahydro-thieno[2,3-*c*]pyridin-2-yloxy] -ethyl}-amine | dimethyl-[2-(4,5,6,7-tetrahydro-thieno[2,3-c]pyridin-2-yloxy)-ethyl]-amine (intermediate 7) and 2-methyl-benzenesulfonyl chloride (commercially available) | 381.1 |
| 100 | 364.9 | (2-chloro-phenyl)-[2-(2-dimethylamino-ethoxy)-4,7-dihydro-5*H*-thieno[2,3-*c*]pyridin-6-yl]-methanone | dimethyl-[2-(4,5,6,7-tetrahydro-thieno[2,3-c]pyridin-2-yloxy)-ethyl]-amine (intermediate 7) and 2-chloro-benzoyl chloride (commercially available) | 365.1 |
| 101 | 408.58 | {2-[6-(4-isopropyl-benzenesulfonyl)-4,5,6,7-tetrahydro-thieno[2,3-c]pyridin-2-yloxy]-ethyl}-dimethyl-amine | dimethyl-[2-(4,5,6,7-tetrahydro-thieno[2,3-c]pyridin-2-yloxy)-ethyl]-amine (intermediate 7) and 4-isopropyl-benzenesulfonyl chloride (commercially available) | 409.1 |

### Example A

Film coated tablets containing the following ingredients can be manufactured in a conventional manner:

| Ingredients | Per tablet | |
|---|---|---|
| Kernel: | | |
| Compound of formula (I) | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |
| Film Coat: | | |
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxide (yellow) | 0.8 mg | 1.6 mg |
| Titanium dioxide | 0.8 mg | 1.6 mg |

The active ingredient is sieved and mixed with microcrystalline cellulose and the mixture is granulated with a solution of polyvinylpyrrolidone in water. The granulate is mixed with sodium starch glycolate and magnesiumstearate and compressed to yield kernels of 120 or 350 mg respectively. The kernels are lacquered with an aqueous solution / suspension of the above mentioned film coat.

### Example B

Capsules containing the following ingredients can be manufactured in a conventional manner:

| Ingredients | Per capsule |
|---|---|
| Compound of formula (I) | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) | 3.0 mg |
| Gelatine | 150.0 mg |
| Phenol | 4.7 mg |
| Sodium carbonate | to obtain a final pH of 7 |
| Water for injection solutions | ad 1.0 ml |

### Example D

Soft gelatin capsules containing the following ingredients can be manufactured in a conventional manner:

| **Capsule contents** | |
|---|---|
| Compound of formula (I) | 5.0 mg |
| Yellow wax | 8.0 mg |
| Hydrogenated Soya bean oil | 8.0 mg |
| Partially hydrogenated plant oils | 34.0 mg |
| Soya bean oil | 110.0 mg |
| Weight of capsule contents | 165.0 mg |

| **Gelatin capsule** | |
|---|---|
| Gelatin | 75.0 mg |
| Glycerol 85 % | 32.0 mg |
| Karion 83 | 8.0 mg (dry matter) |
| Titanium dioxide | 0.4 mg |
| Iron oxide yellow | 1.1 mg |

The active ingredient is dissolved in a warm melting of the other ingredients and the mixture is filled into soft gelatin capsules of appropriate size. The filled soft gelatin capsules are treated according to the usual procedures.

### Example E

Sachets containing the following ingredients can be manufactured in a conventional manner:

| | |
|---|---|
| Compound of formula (I) | 50.0 mg |
| Lactose, fine powder | 1015.0 mg |
| Microcrystalline cellulose (AVICEL PH 102) | 1400.0 mg |
| Sodium carboxymethyl cellulose | 14.0 mg |
| Polyvinylpyrrolidone K 30 | 10.0 mg |
| Magnesium stearate | 10.0 mg |
| Flavoring additives | 1.0 mg |

The active ingredient is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

## Claims

1. Compounds of the general formula 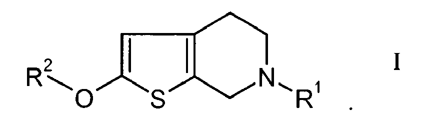 wherein
R¹ is selected from the group consisting of
-CO-R³, wherein
R³ is selected from the group consisting of
C₁₋₇-alkyl,
C₃₋₇-cycloalkyl-C₁₋₇-alkyl, wherein the cycloalkyl ring may be unsubstituted or substituted with one or two groups independently selected from C₁₋₇-alkyl, and
phenyl, wherein the phenyl ring may be unsubstituted or substituted with one or two groups independently selected from C₁₋₇-alkyl, cyano, halogen, halogen-C₁₋₇-alkyl and C₁₋₇-alkoxy,
-CO-NR⁴R⁵, wherein
R⁴ and R⁵ independently from each other are selected from the group consisting of hydrogen, C₁₋₇-alkyl, C₁₋₇-alkoxy-C₁₋₇-alkyl, phenyl unsubstituted or substituted with one or two groups independently selected from C₁₋₇-alkyl, halogen, C₁₋₇-alkoxy and halogen-C₁₋₇-alkyl, and phenyl-C₁₋₇-alkyl, wherein phenyl is unsubstituted or substituted with one or two groups independently selected from C₁₋₇-alkyl, halogen, C₁₋₇-alkoxy and halogen-C₁₋₇-alkyl,
or R⁴ and R⁵ together with the nitrogen atom to which they are attached form a 4-, 5-, 6- or 7-membered heterocyclic ring optionally containing a further heteroatom selected from nitrogen, oxygen or sulfur, a sulfinyl group or a sulfonyl group, said heterocyclic ring being unsubstituted or substituted with one or two groups independently selected from C₁₋₇-alkyl, halogen, cyano, C₁₋₇- alkoxy and phenyl, wherein phenyl is unsubstituted or substituted with one or two groups independently selected from C₁₋₇-alkyl, halogen and C₁₋₇- alkoxy, and
-SO₂-R⁶, wherein
R⁶ is selected from the group consisting of C₁₋₇-alkyl, C₁₋₇-alkoxy-C₁₋₇-alkyl, cycloalkyl-C₁₋₇-alkyl, wherein the cycloalkyl ring may be unsubstituted or substituted with one or two groups independently selected from C₁₋₇-alkyl, halogen and C₁₋₇-alkoxy, and
phenyl, wherein the phenyl ring may be unsubstituted or substituted with one or two groups independently selected from C₁₋₇-alkyl, cyano, halogen, halogen-C₁₋₇-alkyl and C₁₋₇-alkoxy;
R² is a group selected from 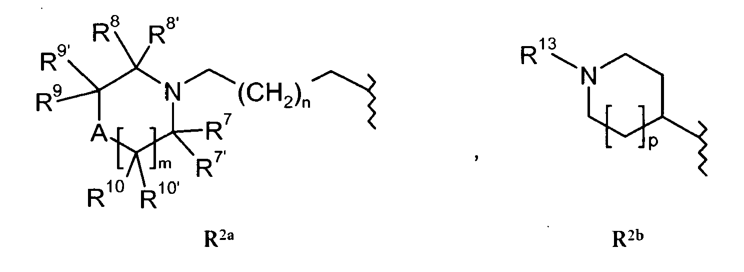 and 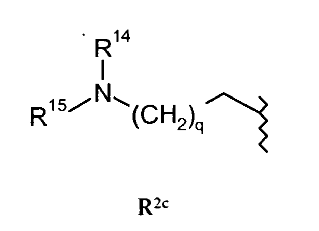
wherein
m is 0, 1 or 2;
n is 0, 1 or 2;
A is selected from -CR¹¹R^{11'}-, O, S or -NR¹²;
R⁷, R^{7'}, R⁸, R^{8'}, R⁹, R^{9'}, R¹⁰, R^{10'}, R¹¹ and R^{11'} independently from each other are hydrogen or C₁₋₇-alkyl;
R¹² is hydrogen or C₁₋₇-alkyl;
p is 0, 1 or 2;
R¹³ is C₁₋₇-alkyl or C₃₋₇-cycloalkyl;
q is 1, 2 or 3; and
R¹⁴ and R¹⁵ independently from each other are C₁₋₇-alkyl;
and pharmaceutically acceptable salts thereof.

2. Compounds of formula I according to claim 1, wherein R¹ is -CO-R³ and
wherein R³ is selected from the group consisting of
C₁₋₇-alkyl,
C₃₋₇-cycloalkyl-C₃₋₇-alkyl, wherein the cycloalkyl ring may be unsubstituted or substituted with one or two groups independently selected from C₃₋₇-alkyl, and phenyl, wherein the phenyl ring may be unsubstituted or substituted with one or two groups independently selected from C₁₋₇-alkyl, cyano, halogen, halogen-C₁₋₇-alkyl and C₁₋₇-alkoxy.

3. Compounds of formula I according to claims 1 or 2, wherein R¹ is -CO-R³ and wherein R³ is C₁₋₇-alkyl or phenyl, wherein the phenyl ring may be unsubstituted or substituted with one or two groups independently selected from C₁₋₇-alkyl, cyano, halogen, halogen-C₁₋₇-alkyl and C₁₋₇-alkoxy.

4. Compounds of formula I according to any one of claims 1 to 3, wherein R¹ is -CO-R³ and wherein R³ is C₁₋₇-alkyl.

5. Compounds of formula I according to any one of claims 1 to 3, wherein R¹ is -CO-R³ and wherein R³ is phenyl, wherein the phenyl ring may be unsubstituted or substituted with one or two groups independently selected from C₁₋₇-alkyl, cyano, halogen, halogen-C₁₋₇-alkyl and C₁₋₇-alkoxy.

6. Compounds of formula I according to any one of claims 1 to 3 or 5, wherein R¹ is -CO-R³ and wherein R³ is phenyl substituted with one or two groups independently selected from halogen, halogen-C₁₋₇-alkyl and C₁₋₇-alkoxy.

7. Compounds of formula I according to claim 1, wherein R¹ is -CO-NR⁴R⁵ and wherein R⁴ and R⁵ independently from each other are selected from the group consisting of
hydrogen, C₁₋₇-alkyl, C₁₋₇-alkoxy-C₁₋₇-alkyl,
phenyl unsubstituted or substituted with one or two groups independently selected from C₁₋₇-alkyl, halogen, C₁₋₇-alkoxy and halogen-C₁₋₇-alkyl, and
phenyl-C₁₋₇-alkyl, wherein phenyl is unsubstituted or substituted with one or two groups independently selected from C₁₋₇-alkyl, halogen, C₁₋₇-alkoxy and halogen-C₁₋₇-alkyl,
or R⁴ and R⁵ together with the nitrogen atom to which they are attached form a 4-, 5-, 6-or 7-membered heterocyclic ring optionally containing a further heteroatom selected from nitrogen, oxygen or sulfur, a sulfinyl group or a sulfonyl group, said heterocyclic ring being unsubstituted or substituted with one or two groups independently selected from lower alkyl, halogen, cyano, lower alkoxy and phenyl, wherein phenyl is unsubstituted or substituted with one or two groups independently selected from C₁₋₇-alkyl, halogen and C₁₋₇-alkoxy.

8. Compounds of formula I according to claims 1 or 7, wherein R¹ is -CO-NR⁴R⁵ and wherein R⁴ and R⁵ independently from each other are hydrogen or C₁₋₇-alkyl.

9. Compounds of formula I according to claims 1 or 7, wherein R¹ is -CO-NR⁴R⁵ and wherein R⁴ and R⁵ together with the nitrogen atom to which they are attached form a 4-, 5-, 6- or 7-membered heterocyclic ring optionally containing a further heteroatom selected from nitrogen, oxygen or sulfur, a sulfinyl group or a sulfonyl group, said heterocyclic ring being unsubstituted or substituted with one or two groups independently selected from C₁₋₇-alkyl, halogen, cyano, C₁₋₇-alkoxy and phenyl, wherein phenyl is unsubstituted or substituted with one or two groups independently selected from C_{1-\7}-alkyl, halogen and C₁₋₇-alkoxy.

10. Compounds of formula I according to any one of claims 1, 7 or 9, wherein R¹ is -CO-NR⁴R⁵ and wherein R⁴ and R⁵ together with the nitrogen atom to which they are attached form a heterocyclic ring selected from the group consisting of pyrrolidinyl, piperidinyl, piperazinyl and morpholinyl, said heterocyclic ring being unsubstituted or substituted with one or two groups independently selected from C₁₋₇-alkyl, halogen, cyano, C₁₋₇-alkoxy and phenyl, wherein phenyl is unsubstituted or substituted with one or two groups independently selected from C₁₋₇-alkyl, halogen and C₁₋₇-alkoxy.

11. Compounds of formula I according to claim 1, wherein R¹ is SO₂-R⁶ and wherein R⁶ is selected from the group consisting of C₁₋₇-alkyl, C₁₋₇-alkoxy-C₁₋₇-alkyl, C₃₋₇-cycloalkyl-C₁₋₇-alkyl, wherein the cycloalkyl ring may be unsubstituted or substituted with one or two groups independently selected from C₁₋₇-alkyl, halogen and C₁₋₇-alkoxy, and
phenyl, wherein the phenyl ring may be unsubstituted or substituted with one or two groups independently selected from C₁₋₇-alkyl, cyano, halogen, halogen-C₁₋₇-alkyl and C₁₋₇-alkoxy.

12. Compounds of formula 1 according to claims 1 or 11, wherein R¹ is SO₂-R⁶ and wherein R⁶ is C₁₋₇-alkyl or phenyl, wherein the phenyl ring may be unsubstituted or substituted with one or two groups selected from C₁₋₇-alkyl and halogen.

13. Compounds of formula I according to any one of claims 1 to 12, wherein R² signifies 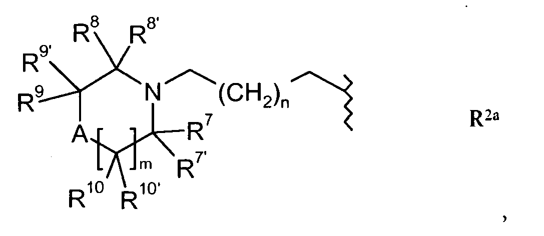 wherein
m is 0 or 1;
n is 1;
A is selected from -CR¹¹R^{11'}-, O, S or -NR¹²;
R⁷, R^{7'}, R⁸, R^{8'}, R⁹, R^{9'}, R¹⁰, R^{10'}, R¹¹ and R^{11'} independently from each other are hydrogen or C₁₋₇-alkyl; and
R¹² is hydrogen or C₁₋₇-alkyl.

14. Compounds of formula I according to any one of claims 1 to 13, wherein A is CR¹¹R^{11'} and R⁷, R^{7'}, R⁸, R^{8'}, R⁹, R^{9'}, R¹⁰, R^{10'}, R¹¹ and R^{11'} independently from each other are hydrogen.

15. Compounds of formula I according to any one of claims 1 to 13, wherein A is O and R⁷, R^{7'}, R⁸, R^{8'}, R⁹, R^{9'}, R¹⁰ and R^{10'} are hydrogen.

16. Compounds of formula 1 according to any one of claims 1 to 12, wherein R² signifies 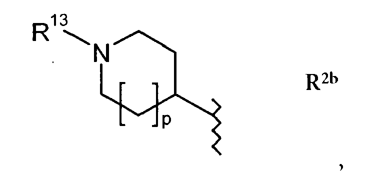 wherein p is 0 or 1; and R¹³ is C₁₋₇-alkyl.

17. Compounds of formula I according to any one of claims 1 to 12 or 16, wherein R¹³ is isopropyl or isobutyl.

18. Compounds of formula I according to any one of claims 1 to 12, wherein R² signifies 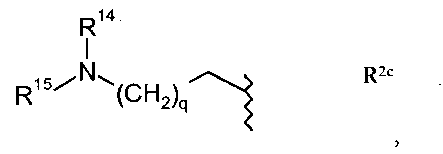 wherein q is 1, 2 or 3; and R¹⁴ and R¹⁵ independently from each other are C₁₋₇-alkyl.

19. Compounds of formula I according to claim 1, selected from the group consisting of
morpholin-4-yl-[2-(3-piperidin-1-yl-propoxy)-4,7-dihydro-5*H*-thieno[*2,3-c*]pyridin-6-yl]-methanone,
piperidin-1-yl-[2-(3-piperidin-1-yl-propoxy)-4,7-dihydro-5*H*-thieno[*2,3-c*]pyndin-6-yl]-methanone,
(4-methyl-piperazin-1-yl)-[2-(3-piperidin-1-yl-propoxy)-4,7-dihydro-5*H*-thieno[*2,3-c*]pyridin-6-yl]-methanone,
[2-(3-piperidin-1-yl-propoxy)-4,7-dihydro-5*H*-thieno[*2,3-c*]pyridin-6-yl]-pyrrolidin-1-yl-methanone,
(4-methoxy-piperidin-1-yl)-[2-(3-piperidin-1-yl-propoxy)-4,7-dihydro-5*H*-thieno[*2,3-c*]pyridin-6-yl]-methanone,
[2-(3-piperidin-1-yl-propoxy)-4,7-dihydro-5*H*-thieno[*2,3-c*]pyridin-6-yl]-(4-o-tolyl-piperazin-1-yl)-methanone,
3-methyl-1-[2-(3-piperidin-1-yl-propoxy)-4,7-dihydro-5*H*-thieno[*2,3-c*]pyridin-6-yl]-butan-1-one,
3,3-dimethyl-1-[2-(3-piperidin-1-yl-propoxy)-4,7-dihydro-5*H*-thieno[*2,3-c*]pyridin-6-yl]-butan-1-one,
(4-methoxy-phenyl)-[2-(3-piperidin-1-yl-propoxy)-4,7-dihydro-5*H*-thieno[*2,3-c*]pyridin-6-yl]-methanone,
(3-fluoro-phenyl)-[2-(3-piperidin-1-yl-propoxy)-4,7-dihydro-5*H*-thieno[*2,3-c*]pyridin-6-yl]-methanone,
(3-chloro-phenyl)-[2-(3-piperidin-1-yl-propoxy)-4,7-dihydro-5*H*-thieno[*2,3-c*]pyridin-6-yl]-methanone,
6-methanesulfonyl-2-(3-piperidin-1-yl-propoxy)-4,5,6,7-tetrahydro-thieno[*2,3-c*]pyridine,
6-(3-fluoro-benzenesulfonyl)-2-(3-piperidin-1-yl-propoxy)-4,5,6,7-tetrahydro-thieno[*2,3-c*]pyridine,
2-(3-piperidin-1-yl-propoxy)-6-(toluene-4-sulfonyl)-4,5,6,7-tetrahydro-thieno[*2,3-c*]pyridine,
2-(3-piperidin-1-yl-propoxy)-6-(toluene-2-sulfonyl)-4,5,6,7-tetrahydro-thieno[*2,3-c*]pyridine,
6-(4-ethyl-benzenesulfonyl)-2-(3-piperidin-1-yl-propoxy)-4,5,6,7-tetrahydro-thieno[*2,3-c*]pyridine,
6-(4-tert-butyl-benzenesulfonyl)-2-(3-piperidin-1-yl-propoxy)-4,5,6,7-tetrahydro-thieno[*2,3-c*]pyridine,
6-benzenesulfonyl-2-(3-piperidin-1-yl-propoxy)-4,5,6,7-tetrahydro-thieno[*2,3-c*]pyridine,
6-(4-isopropyl-benzenesulfonyl)-2-(3-piperidin-1-yl-propoxy)-4,5,6,7-tetrahydro-thieno[*2,3-c*]pyridine,
3,3-dimethyl-1-[2-(3-pyrrolidin-1-yl-propoxy)-4,7-dihydro-5*H*-thieno[*2,3-c*]pyridin-6-yl]-butan-1-one,
(3-chloro-phenyl)-[2-(3-pyrrolidin-1-yl-propoxy)-4,7-dihydro-5*H*-thieno[*2,3-c*]pyridin-6-yl]-methanone,
(2-chloro-phenyl)-[2-(3-pyrrolidin-1-yl-propoxy)-4,7-dihydro-5*H*-thieno[*2,3-c*]pyridin-6-yl]-methanone,
[2-(3-pyrrolidin-1-yl-propoxy)-4,7-dihydro-5*H*-thieno[*2,3-c*]pyridin-6-yl]-(4-trifluoromethyl-phenyl)-methanone,
phenyl-[2-(3-pyrrolidin-1-yl-propoxy)-4,7-dihydro-5*H*-thieno[*2,3-c*]pyridin-6-yl]-methanone,
(3-methoxy-phenyl)-[2-(3-pyrrolidin-1-yl-propoxy)-4,7-dihydro-5*H*-thieno[*2,3-c*]pyridin-6-yl]-methanone,
morpholin-4-yl-[2-(3-pyrrolidin-1-yl-propoxy)-4,7-dihydro-5*H*-thieno[*2,3-c*]pyridin-6-yl]-methanone,
pyrrolidin-1-yl-[2-(3-pyrrolidin-1-yl-propoxy)-4,7-dihydro-5*H*-thieno[*2,3-c*]pyridin-6-yl]-methanone,
piperidin-1-yl-[2-(3-pyrrolidin-1-yl-propoxy)-4,7-dihydro-5*H*-thieno[*2,3-c*]pyridin-6-yl]-methanone,
(4-methyl-piperazin-1-yl)-[2-(3-pyrrolidin-1-yl-propoxy)-4,7-dihydro-5*H*-thieno[*2,3-c*]pyridin-6-yl]-methanone,
2-(3-pyrrolidin-1-yl-propoxy)-4,7-dihydro-5*H*-thieno[*2,3-c*]pyridin-6-yl]-(4-o-tolyl-piperazin-1-yl)-methanone,
6-methanesulfonyl-2-(3-pyrrolidin-1-yl-propoxy)-4,5,6,7-tetrahydro-thieno[*2,3-c*]pyridine,
6-benzenesulfonyl-2-(3-pyrrolidin-1-yl-propoxy)-4,5,6,7-tetrahydro-thieno[*2,3-c*]pyridine,
2-(3-pyrrolidin-1-yl-propoxy)-6-(toluene-4-sulfonyl)-4,5,6,7-tetrahydro-thieno[*2,3-c*]pyridine,
2-(3-pyrrolidin-1-yl-propoxy)-6-(toluene-2-sulfonyl)-4,5,6,7-tetrahydro-thieno[*2,3-c*]pyridine,
2-(3-pyrrolidin-1-yl-propoxy)-6-(toluene-3-sulfonyl)-4,5,6,7-tetrahydro-thieno[*2,3-c*]pyridine,
6-(4-ethyl-benzenesulfonyl)-2-(3-pyrrolidin-1-yl-propoxy)-4,5,6,7-tetrahydro-thieno[*2,3-c*]pyridine,
6-(4-isopropyl-benzenesulfonyl)-2-(3-pyrrolidin-1-yl-propoxy)-4,5,6,7-tetrahydro-thieno[*2,3-c*]pyridine,
6-(4-tert-butyl-benzenesulfonyl)-2-(3-pyrrolidin-1-yl-propoxy)-4,5,6,7-tetrahydro-thieno[*2,3-c*]pyridine,
6-(3-fluoro-benzenesulfonyl)-2-(3-pyrrolidin-1-yl-propoxy)-4,5,6,7-tetrahydro-thieno[*2,3-c*]pyridine,
6-(propane-2-sulfonyl)-2-(3-pyrrolidin-1-yl-propoxy)-4,5,6,7-tetrahydro-thieno[*2,3-c*]pyridine,
[2-(1-isobutyl-piperidin-4-yloxy)-4,7-dihydro-5*H*-thieno[*2,3-c*]pyridin-6-yl]-phenyl-methanone,
1-[2-(1-isobutyl-piperidin-4-yloxy)-4,7-dihydro-5*H*-thieno[*2,3-c*]pyridin-6-yl]-butan-1-one,
1-[2-(1-isobutyl-piperidin-4-yloxy)-4,7-dihydro-5*H*-thieno[*2,3-c*]pyridin-6-yl]-pentan-1-one,
1-[2-(1-isobutyl-piperidin-4-yloxy)-4,7-dihydro-5*H*-thieno[*2,3-c*]pyridin-6-yl]-3,3-dimethyl-butan-1-one,
[2-(1-isobutyl-piperidin-4-yloxy)-4,7-dihydro-5*H*-thieno[*2,3-c*]pyridin-6-yl]-(3-methoxy-phenyl)-methanone,
[2-(1-isobutyl-piperidin-4-yloxy)-4,7-dihydro-5*H*-thieno[*2,3-c*]pyridin-6-yl]-(4-methoxy-phenyl)-methanone,
(4-fluoro-phenyl)-[2-(1-isobutyl-piperidin-4-yloxy)-4,7-dihydro-5*H*-thieno[*2,3-c*]pyridin-6-yl]-methanone,
(3-fluoro-phenyl)-[2-(1-isobutyl-piperidin-4-yloxy)-4,7-dihydro-5*H*-thieno[*2,3-c*]pyridin-6-yl]-methanone,
[2-(1-isobutyl-piperidin-4-yloxy)-4,7-dihydro-5*H*-thieno[*2,3-c*]pyridin-6-yl]-(4-trifluoromethyl-phenyl)-methanone,
(3-chloro-phenyl)-[2-(1-isobutyl-piperidin-4-yloxy)-4,7-dihydro-5*H*-thieno[*2,3-c*]pyridin-6-yl]-methanone,
(2-chloro-phenyl)-[2-(1-isobutyl-piperidin-4-yloxy)-4,7-dihydro-5*H*-thieno[*2,3-c*]pyridin-6-yl]-methanone,
[2-(1-isobutyl-piperidin-4-yloxy)-4,7-dihydro-5*H*-thieno[*2,3-c*]pyridin-6-yl]-morpholin-4-yl-methanone,
2-(1-isobutyl-piperidin-4-yloxy)-4,7-dihydro-5*H*-thieno[*2,3-c*]pyridine-6-carboxylic acid diethylamide,
[2-(1-isobutyl-piperidin-4-yloxy)-4,7-dihydro-5*H*-thieno[*2,3-c*]pyridin-6-yl]-piperidin-1-yl-methanone,
[2-(1-isobutyl-piperidin-4-yloxy)-4,7-dihydro-5*H*-thieno[*2,3-c*]pyridin-6-yl]-pyrrolidin-1-yl-methanone,
2-(1-isobutyl-piperidin-4-yloxy)-6-methanesulfonyl-4,5,6,7-tetrahydro-thieno[*2,3-c*]pyridine,
6-benzenesulfonyl-2-(1-isobutyl-piperidin-4-yloxy)-4,5,6,7-tetrahydro-thieno[*2,3-c*]pyridine,
2-(1-isobutyl-piperidin-4-yloxy)-6-(toluene-4-sulfonyl)-4,5,6,7-tetrahydro-thieno[*2,3-c*]pyridine,
2-(1-isobutyl-piperidin-4-yloxy)-6-(toluene-2-sulfonyl)-4,5,6,7-tetrahydro-thieno[*2,3-c*]pyridine,
2-(1-isobutyl-piperidin-4-yloxy)-6-(toluene-3-sulfonyl)-4,5,6,7-tetrahydro-thieno[*2,3-c*]pyridine,
6-(4-ethyl-benzenesulfonyl)-2-(1-isobutyl-piperidin-4-yloq)-4,5,6,7-tetrahydro-thieno[*2,3-c*]pyridine,
6-(3-fluoro-benzenesulfonyl)-2-(1-isobutyl-piperidin-4-yloxy)-4,5,6,7-tetrahydro-thieno[*2,3-c*]pyridine,
1-[2-(-isopropyl-piperidin-4-yloxy)-4,7-dihydro-5*H*-thieno[*2,3-c*]pyridin-6-yl]-butan-1-one,
1-[2-(1-isopropyl-piperidin-4-yloxy)-4,7-dihydro-5*H*-thieno[*2,3-c*]pyridin-6-yl]-3-methyl-butan-1-one,
1-[2-(1-isopropyl-piperidin-4-yloxy)-4,7-dihydro-5*H*-thieno[*2,3-c*]pyridin-6-yl]-3,3-dimethyl-butan-1-one,
[2-(1-isopropyl-piperidin-4-yloxy)-4,7-dihydro-5*H*-thieno[*2,3-c*]pyridin-6-yl]-(4-trifluoromethyl-phenyl)-methanone,
(3-chloro-phenyl)-[2-(1-isopropyl-piperidin-4-yloxy)-4,7-dihydro-5*H*-thieno[*2,3-c*]pyridin-6-yl]-methanone,
[2-(1-isopropyl-piperidin-4-yloxy)-4,7-dihydro-5*H*-thieno[*2,3-c*]pyridin-6-yl]-(3-methoxy-phenyl)-methanone,
[2-(1-isopropyl-piperidin-4-yloxy)-4,7-dihydro-5*H*-thieno[*2,3-c*]pyridin-6-yl]-(4-methoxy-phenyl)-methanone,
(4-fluoro-phenyl)-[2-(1-isopropyl-piperidin-4-yloxy)-4,7-dihydro-5*H*-thieno[*2,3-c*]pyridin-6-yl]-methanone,
(3-fluoro-phenyl)-[2-(1-isopropyl-piperidin-4-yloxy)-4,7-dihydro-5*H*-thieno[*2,3-c*]pyridin-6-yl]-methanone,
(2-chloro-phenyl)-[2-(1-isopropyl-piperidin-4-yloxy)-4,7-dihydro-5*H*-thieno[*2,3-c*]pyridin-6-yl]-methanone,
[2-(1-isopropyl-piperidin-4-yloxy)-4,7-dihydro-5*H*-thieno[*2,3-c*]pyridin-6-yl]-(2-methoxy-phenyl)-methanone,
(2-fluoro-phenyl)-[2-(1-isopropyl-piperidin-4-yloxy)-4,7-dihydro-5*H*-thieno[*2,3-c*]pyridin-6-yl]-methanone,
[2-(1-isopropyl-piperidin-4-yloxy)-4,7-dihydro-5*H*-thieno[*2,3-c*]pyridin-6-yl]-morpholin-4-yl-methanone,
2-(1-isopropyl-piperidin-4-yloxy)-4,7-dihydro-5*H*-thieno[*2,3-c*]pyridine-6-carboxylic aciddiethylamide,
[2-(1-isopropyl-piperidin-4-yloxy)-4,7-dihydro-5*H*-thieno[*2,3-c*]pyridin-6-yl]-piperidin-1-yl-methanone,
2-(1-isopropyl-piperidin-4-yloxy)-4,7-dihydro-5*H*-thieno[*2,3-c*]pyridine-6-carboxylic acid methylamide,
[2-(1-isopropyl-piperidin-4-yloxy)-4,7-dihydro-5*H*-thieno[*2,3-c*]pyridin-6-yl]-(4-methyl-piperazin-1-yl)-methanone,
2-(1-isopropyl-piperidin-4-yloxy)-4,7-dihydro-5*H*-thieno[*2,3-c*]pyridine-6-carboxylic acid ethylamide,
2-(1-isopropyl-piperidin-4-yloxy)-4,7-dihydro-5*H*-thieno[*2,3-c*]pyridine-6-carboxylic acidisopropylamide,
2-(1-isopropyl-piperidin-4-yloxy)-4,7-dihydro-5*H*-thieno[*2,3-c*]pyridine-6-carboxylic acid butylamide,
[2-(1-isopropyl-piperidin-4-yloxy)-4,7-dihydro-5*H*-thieno[*2,3-c*]pyridin-6-yl]-(4-o-tolyl-piperazin-1-yl)-methanone,
2-(1-isopropyl-piperidin-4-yloxy)-6-methanesulfonyl-4,5,6,7-tetrahydro-thieno[*2,3-c*]pyridine,
6-benzenesulfonyl-2-(1-isopropyl-piperidin-4-yloxy)-4,5,6,7-tetrahydro-thieno[*2,3-c*]pyridine,
2-(1-isopropyl-piperidin-4-yloxy)-6-(toluene-4-sulfonyl)-4,5,6,7-tetrahydro-thieno[*2,3-c*]pyridine,
2-(1-isopropyl-piperidin-4-yloxy)-6-(toluene-2-sulfonyl)-4,5,6,7-tetrahydro-thieno[*2,3-c*]pyridine,
2-(1-isopropyl-piperidin-4-yloxy)-6-(toluene-3-sulfonyl)-4,5,6,7-tetrahydro-thieno[*2,3-c*]pyridine,
6-(4-ethyl-benzenesulfonyl)-2-(1-isopropyl-piperidin-4-yloxy)-4,5,6,7-tetrahydro-thieno[*2,3-c*]pyridine,
6-(4-isopropyl-benzenesulfonyl)-2-(1-isopropyl-piperidin-4-yloxy)-4,5,6,7-tetrahydro-thieno[*2,3-c*]pyridine,
6-(4-tert-butyl-benzenesulfonyl)-2-(1-isopropyl-piperidin-4-yloxy)-4,5,6,7-tetrahydro-thieno[*2,3-c*]pyridine,
6-(3-fluoro-benzenesulfonyl)-2-(1-isopropyl-piperidin-4-yloxy)-4,5,6,7-tetrahydro-thieno[*2,3-c*]pyridine,
2-(1-isopropyl-piperidin-4-yloxy)-6-(propane-2-sulfonyl)-4,5,6,7-tetrahydro-thieno[*2,3-c*]pyridine,
2-(1-isopropyl-pyrrolidin-3-yloxiy)-6-(toluene-4-sulfonyl)-4,5,6,7-tetrahydro-thieno[*2,3-c*]pyridine,
6-(4-tert-butyl-benzenesulfonyl)-2-(1-isopropyl-pyrrolidin-3-yloxy)-4,5,6,7-tetrahydro-thieno[*2,3-c*]pyridine,
6-(4-isopropyl-benzenesulfonyl)-2-(1-isopropyl-pyrrolidin-3-yloxy)-4,5,6,7-tetrahydro-thieno[*2,3-c*]pyridine,
(4-chloro-phenyl)-[2-(3-morpholin-4-yl-propoxy)-4,7-dihydro-5*H*-thieno[*2,3-c*]pyridin-6-yl]-methanone,
(3-chloro-phenyl)-[2-(3-morpholin-4-yl-propoxy)-4,7-dihydro-5*H*-thieno[*2,3-c*]pyridin-6-yl]-methanone,
dimethyl-{2-[6-(toluene-2-sulfonyl)-4,5,6,7-tetrahydro-thieno[*2,3-c*]pyridin-2-yloxy]-ethyl}-amine,
(2-chloro-phenyl)-[2-(2-dimethylamino-ethoxy)-4,7-dihydro-5*H*-thieno[*2,3-c*]pyridin-6-yl]-methanone,
{2-[6-(4-isopropyl-benzenesulfonyl)-4,5,6,7-tetrahydro-thieno[*2,3-c*]pyridin-2-yloxy]-ethyl}-dimethyl-amine,
and pharmaceutically acceptable salts thereof.

20. Compounds of formula 1 according to claim 1, selected from the group consisting of
3-methyl-1-[2-(3-piperidin-1-yl-propoxy)-4,7-dihydro-5*H*-thieno[*2,3-c*]pyridin-6-yl]-butan-1-one,
3,3-dimethyl-1-[2-(3-piperidin-1-yl-propoxy)-4,7-dihydro-5*H*-thieno[*2,3-c*]pyridin-6-yl]-butan-1-one,
6-benzenesulfonyl-2-(3-pyrrolidin-1-yl-propoxy)-4,5,6,7-tetrahydro-thieno[*2,3-c*]pyridine,
1-[2-(1-isopropyl-piperidin-4-yloxy)-4,7-dihydro-5*H*-thieno[*2,3-c*]pyridin-6-yl]-butan-1-one,
1-[2-(1-isopropyl-piperidin-4-yloxy)-4,7-dihydro-5*H*-thieno[*2,3-c*]pyridin-6-yl]-3-methyl-butan-1-one,
1-[2-(1-isopropyl-piperidin-4-yloxy)-4,7-dihydro-5*H*-thieno[*2,3-c*]pyridin-6-yl]-3,3-dimethyl-butan-1-one,
(3-chloro-phenyl)-[2-(1-isopropyl-piperidin-4-yloxy)-4,7-dihydro-5*H*-thieno[*2,3-c*]pyridin-6-yl]-methanone,
[2-(1-isopropyl-piperidin-4-yloxy)-4,7-dihydro-5*H*-thieno[*2,3-c*]pyridin-6-yl]-(3-methoxy-phenyl)-methanone,
(4-fluoro-phenyl)-[2-(1-isopropyl-piperidin-4-yloxy)-4,7-dihydro-5*H*-thieno[*2,3-c*]pyridin-6-yl]-methanone,
(3-fluoro-phenyl)-[2-(1-isopropyl-piperidin-4-yloxy)-4,7-dihydro-5*H*-thieno[*2,3-c*]pyridin-6-yl]-methanone,
(2-chloro-phenyl)-[2-(1-isopropyl-piperidin-4-yloxy)-4,7-dihydro-5*H*-thieno[*2,3-c*]pyridin-6-yl]-methanone,
(2-fluoro-phenyl)-[2-(1-isopropyl-piperidin-4-yloxy)-4,7-dihydro-5*H*-thieno[*2,3-c*]pyridin-6-yl]-methanone,
[2-(-isopropyl-piperidin-4-yloxy)-4,7-dihydro-5*H*-thieno[*2,3-c*]pyridin-6-yl]-morpholin-4-yl-methanone,
[2-(1-isopropyl-piperidin-4-yloxy)-4,7-dihydro-5*H*-thieno[*2,3-c*]pyridin-6-yl]-piperidin-1-yl-methanone,
[2-(1-isopropyl-piperidin-4-yloxy)-4,7-dihydro-5*H*-thieno[*2,3-c*]pyridin-6-yl]-(4-methyl-piperazin-1-yl)-methanone,
2-(1-isopropyl-piperidin-4-yloxy)-6-(toluene-2-sulfonyl)-4,5,6,7-tetrahydro-thieno[*2,3-c*]pyridine,
6-(3-fluoro-benzenesulfonyl)-2-(1-isopropyl-piperidin-4-yloxy)-4,5,6,7-tetrahydro-thieno[*2,3-c*]pyridine,
and pharmaceutically acceptable salts thereof.

21. A process for the manufacture of compounds according to any one of claims 1 to 20, which process comprises
coupling an amine of the formula II 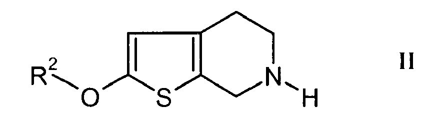 wherein R² is as defined in claim 1, with an halogenide of the formula III
R¹-X III
wherein R¹ is as defined in claim 1 and X is halogen,
in the presence of a base to obtain a compound of the formula I 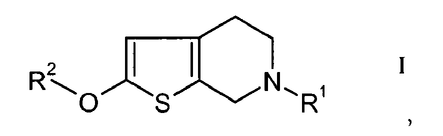 and if desired,
converting the compound obtained into a pharmaceutically acceptable salt.

22. Pharmaceutical compositions comprising a compound according to any one of claims 1 to 20 as well as a pharmaceutically acceptable carrier and/or adjuvant.

23. Pharmaceutical compositions according to claim 22 for the treatment and/or prevention of diseases which are associated with the modulation of H3 receptors.

24. Compounds according to any one of claims 1 to 20 for use as therapeutically active substances.

25. Compounds according to any one of claims 1 to 20 for use as therapeutically active substances for the treatment and/or prevention of diseases which are associated with the modulation of H3 receptors.

26. The use of compounds according to any one of claims 1 to 20 for the preparation of medicaments for the treatment and/or prevention of diseases which are associated with the modulation of H3 receptors.

27. The use according to claim 26 for the treatment and/or prevention of obesity.

## Patentansprüche

1. Verbindungen der allgemeinen Formel 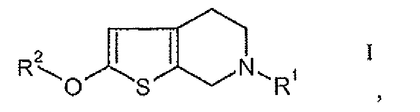 worin
R¹ aus der Gruppe ausgewählt ist, bestehend aus
-CO-R³, wobei
R³ aus der Gruppe ausgewählt ist, bestehend aus
C₁₋₇-Alkyl,
C₃₋₇-Cycloalkyl-C₁₋₇-alkyl, wobei der Cycloalkylring unsubstituiert oder mit einer oder zwei Gruppen, unabhängig voneinander ausgewählt aus C₁₋₇-Alkyl, substitu- iert sein kann, und
Phenyl, wobei der Phenylring unsubstituiert oder mit einer oder zwei Gruppen, un- abhängig voneinander ausgewählt aus C₁₋₇-Alkyl, Cyano, Halogen, Halogen-C₁₋₇- alkyl und C₁₋₇-Alkoxy, substituiert sein kann,
-CO-NR⁴R⁵, wobei
R⁴ und R⁵ unabhängig voneinander aus der Gruppe ausgewählt sind, bestehend aus Wasserstoff, C₁₋₇-Alkyl, C₁₋₇-Alkoxy-C₁₋₇-alkyl, Phenyl, unsubstituiert oder substituiert mit einer oder zwei Gruppen, unabhängig voneinander ausgewählt aus C₁₋₇-Alkyl, Halogen, C₁₋₇-Alkoxy und Halogen-C₁₋₇- alkyl, und
Phenyl-C₁₋₇-alkyl, wobei das Phenyl unsubstituiert oder mit einer oder zwei Grup- pen, unabhängig voneinander ausgewählt aus C₁₋₇-Alkyl, Halogen, C₁₋₇-Alkoxy und Halogen-C₁₋₇-alkyl, substituiert ist,
oder R⁴ und R⁵ zusammen mit dem Stickstoffatom, an das sie gebunden sind, einen 4-, 5-, 6- oder 7gliedrigen heterocyclischen Ring bilden, der gegebenenfalls ein wei- teres Heteroatom, ausgewählt aus Stickstoff, Sauerstoff oder Schwefel, eine Sulfi- nylgruppe oder eine Sulfonylgruppe enthält, wobei der heterocyclische Ring unsub- stituiert oder mit einer oder zwei Gruppen, unabhängig voneinander ausgewählt aus C₁₋₇-Alkyl, Halogen, Cyano, C₁₋₇-Alkoxy und Phenyl, substituiert ist, wobei das Phe- nyl unsubstituiert oder mit einer oder zwei Gruppen, unabhängig voneinander aus- gewählt aus C₁₋₇-Alkyl, Halogen und C₁₋₇-Alkoxy, substituiert ist, und
-SO₂-R⁶, wobei
R⁶ aus der Gruppe ausgewählt ist, bestehend aus
C₁₋₇-Alkyl, C₁₋₇-Alkoxy-C₁₋₇-alkyl,
Cycloalkyl-C₁₋₇-alkyl, wobei der Cycloalkylring unsubstituiert oder mit einer oder zwei Gruppen, unabhängig voneinander ausgewählt aus C₁₋₇-Alkyl, Halogen und C₁₋₇-Alkoxy, substituiert sein kann, und
Phenyl, wobei der Phenylring unsubstituiert oder mit einer oder zwei Gruppen, unab- hängig voneinander ausgewählt aus C₁₋₇-Alkyl, Cyano, Halogen, Halogen-C₁₋₇-al- kyl und C₁₋₇-Alkoxy, substituiert sein kann;
R² eine Gruppe ist, ausgewählt aus 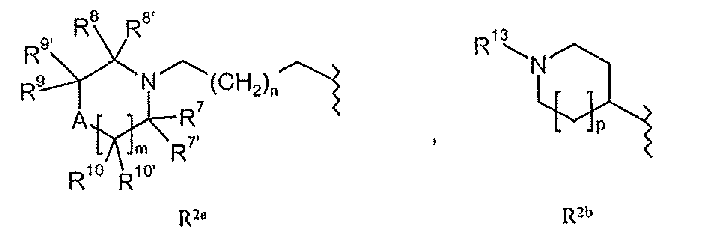 und 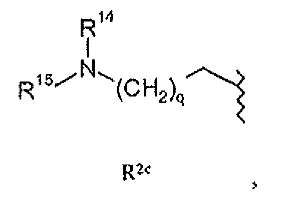
wobei
m 0, 1 oder 2 ist;
n 0, 1 oder 2 ist;
A aus -CR¹¹R^{11'}-, O, S oder -NR¹² ausgewählt ist;
R⁷, R^{7'}, R⁸, R^{8'}, R⁹, R^{9'}, R¹⁰, R^{10'}, R¹¹ und R^{11'} unabhängig voneinander Wasserstoff oder C₁₋₇-Alkyl sind;
R¹² Wasserstoff oder C₁₋₇-Alkyl ist;
p 0, 1 oder 2 ist;
R¹³ C₁₋₇-Alkyl oder C₃₋₇-Cycloalkyl ist;
q 1, 2 oder 3 ist; und
R¹⁴ und R¹⁵ unabhängig voneinander C₁₋₇-Alkyl sind;
und pharmazeutisch akzeptable Salze davon.

2. Verbindungen der Formel I nach Anspruch 1, wobei R¹ -CO-R³ ist und wobei R³ aus der Gruppe ausgewählt ist, bestehend aus
C₁₋₇-Alkyl,
C₃₋₇-Cycloalkyl-C₃₋₇-alkyl, wobei der Cycloalkylring unsubstituiert oder mit einer oder zwei Gruppen, unabhängig voneinander ausgewählt aus C₃₋₇-Alkyl, substituiert sein kann, und Phenyl, wobei der Phenylring unsubstituiert oder mit einer oder zwei Gruppen, unabhängig voneinander ausgewählt aus C₁₋₇-Alkyl, Cyano, Halogen, Halogen-C₁₋₇-alkyl und C₁₋₇-Alkoxy, substituiert sein kann.

3. Verbindungen der Formel I nach den Ansprüchen 1 oder 2, wobei R¹ -CO-R³ ist und wobei R³ C₁₋₇-Alkyl oder Phenyl ist, wobei der Phenylring unsubstituiert oder mit einer oder zwei Gruppen, unabhängig voneinander ausgewählt aus C₁₋₇-Alkyl, Cyano, Halogen, Halogen-C₁₋₇-alkyl und C₁₋₇-Alkoxy, substituiert sein kann.

4. Verbindungen der Formel I nach einem der Ansprüche 1 bis 3, wobei R¹ -CO-R³ ist und wobei R³ C₁₋₇-Alkyl ist.

5. Verbindungen der Formel I nach einem der Ansprüche 1 bis 3, wobei R¹ -CO-R³ ist und wobei R³ Phenyl ist, wobei der Phenylring unsubstituiert oder mit einer oder zwei Gruppen, unabhängig voneinander ausgewählt aus C₁₋₇-Alkyl, Cyano, Halogen, Halogen-C₁₋₇-alkyl und C₁₋₇-Alkoxy, substituiert sein kann.

6. Verbindungen der Formel I nach einem der Ansprüche 1 bis 3 oder 5, wobei R¹ -CO-R³ ist und wobei R³ Phenyl, substituiert mit einer oder zwei Gruppen, unabhängig voneinander ausgewählt aus Halogen, Halogen-C₁₋₇-alkyl und C₁₋₇-Alkoxy, ist.

7. Verbindungen der Formel I nach Anspruch 1, wobei R¹ -CO-NR⁴R⁵ ist und wobei R⁴ und R⁵ unabhängig voneinander aus der Gruppe ausgewählt sind, bestehend aus
Wasserstoff, C₁₋₇-Alkyl, C₁₋₇-Alkoxy-C₁₋₇-alkyl,
Phenyl, unsubstituiert oder substituiert mit einer oder zwei Gruppen, unabhängig voneinander ausgewählt aus C₁₋₇-Alkyl, Halogen, C₁₋₇-Alkoxy und Halogen-C₁₋₇-alkyl, und Phenyl-C₁₋₇-alkyl, wobei das Phenyl unsubstituiert oder mit einer oder zwei Gruppen, unabhängig voneinander ausgewählt aus C₁₋₇-Alkyl, Halogen, C₁₋₇-Alkoxy und Halogen-C₁₋₇-alkyl, substituiert ist,
oder R⁴ und R⁵ zusammen mit dem Stickstoffatom, an das sie gebunden sind, einen 4-, 5-, 6-oder 7gliedrigen heterocyclischen Ring bilden, der gegebenenfalls ein weiteres Heteroatom, ausgewählt aus Stickstoff, Sauerstoff oder Schwefel, eine Sulfinylgruppe oder eine Sulfonylgruppe enthält, wobei der heterocyclische Ring unsubstituiert oder mit einer oder zwei Gruppen, unabhängig voneinander ausgewählt aus Niederalkyl, Halogen, Cyano, Niederalkoxy und Phenyl, substituiert ist, wobei das Phenyl unsubstituiert oder mit einer oder zwei Gruppen, unabhängig voneinander ausgewählt aus C₁₋₇-Alkyl, Halogen und C₁₋₇-Alkoxy, substituiert ist.

8. Verbindungen der Formel I nach den Ansprüchen 1 oder 7, wobei R¹ -CO-NR⁴R⁵ ist und wobei R⁴ und R⁵ unabhängig voneinander Wasserstoff oder C₁₋₇-Alkyl sind.

9. Verbindungen der Formel I nach den Ansprüchen 1 oder 7, wobei R¹ -CO-NR⁴R⁵ ist und wobei R⁴ und R⁵ zusammen mit dem Stickstoffatom, an das sie gebunden sind, einen 4-, 5-, 6- oder 7gliedrigen heterocyclischen Ring bilden, der gegebenenfalls ein weiteres Heteroatom, ausgewählt aus Stickstoff, Sauerstoff oder Schwefel, eine Sulfinylgruppe oder eine Sulfonylgruppe enthält, wobei der heterocyclische Ring unsubstituiert oder mit einer oder zwei Gruppen, unabhängig voneinander ausgewählt aus C₁₋₇-Alkyl, Halogen, Cyano, C₁₋₇-Alkoxy und Phenyl, substituiert ist, wobei das Phenyl unsubstituiert oder mit einer oder zwei Gruppen, unabhängig voneinander ausgewählt aus C₁₋₇-Alkyl, Halogen und C₁₋₇-Alkoxy, substituiert ist.

10. Verbindungen der Formel I nach einem der Ansprüche 1, 7 oder 9, wobei R¹ -CO-NR⁴R⁵ ist und wobei R⁴ und R⁵ zusammen mit dem Stickstoffatom, an das sie gebunden sind, einen heterocyclischen Ring, ausgewählt aus der Gruppe, bestehend aus Pyrrolidinyl, Piperidinyl, Piperazinyl und Morpholinyl, bilden, wobei der heterocyclische Ring unsubstituiert oder mit einer oder zwei Gruppen, unabhängig voneinander ausgewählt aus C₁₋₇-Alkyl, Halogen, Cyano, C₁₋₇-Alkoxy und Phenyl, substituiert ist, wobei das Phenyl unsubstituiert oder mit einer oder zwei Gruppen, unabhängig voneinander ausgewählt aus C₁₋₇-Alkyl, Halogen und C₁₋₇-Alkoxy, substituiert ist.

11. Verbindungen der Formel I nach Anspruch 1, wobei R¹ SO₂-R⁶ ist und wobei R⁶ aus der Gruppe ausgewählt ist, bestehend aus C₁₋₇-Alkyl, C₁₋₇-Alkoxy-C₁₋₇-alkyl, C₃₋₇-Cycloalkyl-C₁₋₇-alkyl, wobei der Cycloalkylring unsubstituiert oder mit einer oder zwei Gruppen, unabhängig voneinander ausgewählt aus C₁₋₇-Alkyl, Halogen und C₁₋₇-Alkoxy, substituiert sein kann, und
Phenyl, wobei der Phenylring unsubstituiert oder mit einer oder zwei Gruppen, unabhängig voneinander ausgewählt aus C₁₋₇-Alkyl, Cyano, Halogen, Halogen-C₁₋₇-alkyl und C₁₋₇-Alkoxy, substituiert sein kann.

12. Verbindungen der Formel I nach den Ansprüchen 1 oder 11, wobei R¹ SO₂-R⁶ ist und wobei R⁶ C₁₋₇-Alkyl oder Phenyl ist, wobei der Phenylring unsubstituiert oder mit einer oder zwei Gruppen, ausgewählt aus C₁₋₇-Alkyl und Halogen, substituiert sein kann.

13. Verbindungen der Formel I nach einem der Ansprüche 1 bis 12, wobei R² 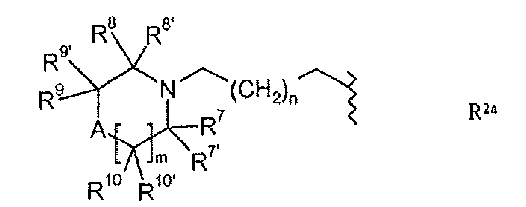 bedeutet, wobei
m 0 oder 1 ist;
n 1 ist;
A ausgewählt ist aus -CR¹¹R^{11'}-, O, S oder -NR¹²;
R⁷, R⁷', R⁸, R^{8'}, R⁹, R^{9'}, R¹⁰, R^{10'}, R¹¹ und R^{11'} unabhängig voneinander Wasserstoff oder C₁₋₇-Alkyl sind und
R¹² Wasserstoff oder C₁₋₇-Alkyl ist.

14. Verbindungen der Formel I nach einem der Ansprüche 1 bis 13, wobei A CR¹¹R^{11'} ist und R⁷, R^{7'}, R⁸, R^{8'}, R⁹, R^{9'}, R¹⁰, R^{10'}, R¹¹ und R^{11'} unabhängig voneinander Wasserstoff sind.

15. Verbindungen der Formel I nach einem der Ansprüche 1 bis 13, wobei A O ist und R⁷, R^{7'}, R⁸, R^{8'}, R⁹, R^{9'}, R¹⁰ und R^{10'} Wasserstoff sind.

16. Verbindungen der Formel I nach einem der Ansprüche 1 bis 12, wobei R² 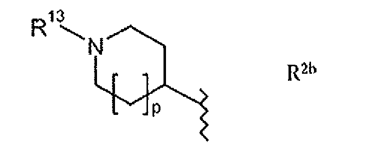 bedeutet, wobei p 0 oder 1 ist und R¹³ C₁₋₇-Alkyl ist.

17. Verbindungen der Formel I nach einem der Ansprüche 1 bis 12 oder 16, wobei R¹³ Isopropyl oder Isobutyl ist.

18. Verbindungen der Formel I nach einem der Ansprüche 1 bis 12, wobei R² 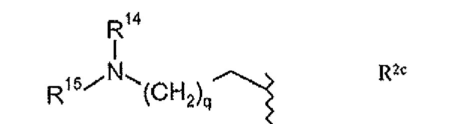 bedeutete, wobei q 1, 2 oder 3 ist und R¹⁴ und R¹⁵ unabhängig voneinander C₁₋₇-Alkyl sind.

19. Verbindungen der Formel I nach Anspruch 1, ausgewählt aus der Gruppe, bestehend aus
Morpliolin-4-yl-[2-(3-piperidin-1-yl-propoxy)-4,7-dihydro-5*H*-thieno[2,3-c]pyridin-6-yl]-methanon,
Piperidin-1-yl-[2-(3-piperidin-1-yl-propoxy)-4,7-dihydro-5*H*-thieno[2,3-c]pyridin-6-yl]-methanon,
(4-Methyl-piperazin-1-yl)-[2-(3-piperidin-1-yl-propoxy)-4,7-dihydro-5*H*-thieno[2,3-c]pyridin-6-yl]-methanon,
[2-(3-Piperidin-1-yl-propoxy)-4,7-dihydro,5*H*-thieno[2,3-c]pyridin-6-yl]-pyrrolidin-1-yl-methanon,
(4-Methoxy-piperidin-1-yl)-[2-(3-piperidin-1-yl-propoxy)-4,7-dihydro-5*H*-thieno[2,3-c]pyridin-6-yl]-methanon,
[2-(3-Piperidin-1-yl-propoxy)-4,7-dihydro-5*H*-thieno[2,3-c]pyridin-6-yl]-(4-o-tolyl-piperazin-1-yl)-methanon,
3-Methyl-1-[2-(3-piperidin-1-yl-propoxy)-4,7-dihydro-5*H*-thieno[2,3-c]pyridin-6-yl]-butan-1-on,
3,3-Dimethyl-1-[2-(3-piperidin-1-yl-propoxy)-4,7-dihydro-5*H*-thieno[2,3-c]pyridin-6-yl]-butan-1-on,
(4-Methoxy-phenyl)-[2-(3-piperidin-1-yl-propoxy)-4,7-dihydro-5*H*-thieno[2,3-c]pyridin-6-yl]-methanon,
(3-Fluor-phenyl)-[2-(3-piperidin-1-yl-propoxy)-4,7-dihydro-5*H*-thieno[2,3-c]pyridin-6-yl]-methanon,
(3-Chlor-phenyl)-[2-(3-piperidin-1-yl-propoxy)-4,7-dihydro-5*H*-thieno[2,3-c]pyridin-6-yl]-methanon,
6-Methansulfonyl-2-(3-piperidin-1-yl-propoxy)-4,5,6,7-tetrahydro-thieno[2,3-c]pyridin, 6-(3-Fluor-benzolsulfonyl)-2-(3-piperidin-1-yl-propoxy)-4,5,6,7-tetrahydro-thieno[2,3-c]-pyridin,
2-(3-Piperidin-1-yl-propoxy)-6-(toluol-4-sulfonyl)-4,5,6,7-tetrahydro-thieno[2,3-c]pyridin, 2-(3-Piperidin-1-yl-propoxy)-6-(toluol-2-sulfonyl)-4,5,6,7-tetraliydro-thieno[2,3-c]pyridin,
6-(4-Ethyl-benzolsulfonyl)-2-(3-piperidin-1-yl-propoxy)-4,5,6,7-tetrahydro-thieno[2,3-c]-pyridin,
6-(4-tert-Butyl-benzolsulfonyl)-2-(3-piperidin-1-yl-propoxy)-4,5,6,7-tetrahydro-thieno[2,3-c]-pyridin,
6-Benzolsulfonyl-2-(3-piperidin-1-yl-propoxy)-4,5,6,7-tetrahydro-thieno[2,3-c]pyridin,
6-(4-Isopropyl-benzolsulfonyl)-2-(3-piperidin-1-yl-propoxy)-4,5,6,7-tetrahydro-thieno[2,3-c]pyridin,
3,3-Dimethyl-1-[2-(3-pyrrolidin-1-yl-propoxy)-4,7-dihydro-5*H*-thieno[2,3-c]pyridin-6-yl]-butan-1-on,
(3-Chlor-phenyl)-[2-(3-pyrrolidin-1-yl-propoxy)-4,7-dihydro-5*H*-thieno[2,3-c]pyridin-6-yl]-methanon,
(2-Chlor-phenyl)-[2-(3-pyrrolidin-1-yl-propoxy)-4,7-dihydro-5*H*-thieno[2,3-c]pyridin-6-yl]-methanon,
[2-(3-Pyrrolidin-1-yl-propoxy)-4,7-dihydro-5*H*-thieno[2,3-c]pyridin-6-yl]-(4-trifluormethylphenyl)-methanon,
Phenyl-[2-(3-pyrrolidin-1-yl-piopoxy)-4,7-diliydro-5*H*-thieno[2,3-c]pyridin-6-yl]-methanon,
(3-Methoxy-phenyl)-[2-(3-pyrrolidin-1-yl-propoxy)-4,7-dihydro-5*H*-thieno[2,3-c]pyridin-6-yl]-methanon,
Morpholin-4-yl-[2-(3-pyrrolidin-1-yl-propoxy)-4,7-dihydro-5*H*-thieno[2,3-c]pyridin-6-yl]-methanon,
Pyrrolidin-1-yl-[2-(3-pyrrolidin-1-yl-propoxy)-4,7-dihydro-5*H*-thieno[2,3-c]pyridin-6-yl]-methanon,
Piperidin-1-yl-[2-(3-pyrrolidin-1-yl-propoxy)-4,7-dihydro-5*H*-thieno[2,3-c]pyridin-6-yl]-methanon,
(4-Methyl-piperazin-1-yl)-[2-(3-pyrrolidin-1-yl-propoxy)-4,7-dihydro-5*H*-thieno[2,3-c]pyridin-6-yl]-methanon,
2-(3-Pyrrolidin-1-yl-propoxy)-4,7-dihydro-5*H*-thieno[2,3-c]pyridin-6-yl]-(4-o-tolyl-piperazin-1-yl)-methanon,
6-Methansulfonyl-2-(3-pyrrolidin-1-yl-propoxy)-4,5,6,7-tetrahydro-thieno[2,3-c]pyridin,
6-Benzolsulfonyl-2-(3-pyrrolidin-1-yl-propoxy)-4,5,6,7-tetrahydro-thieno[2,3-c]pyridin,
2-(3-Pyrrolidin-1-yl-propoxy)-6-(toluol-4-sulfonyl)-4,5,6,7-tetrahydro-thieno[2,3-c]pyridin,
2-(3-Pyrralidin-1-yl-propoxy)-6-(toluol-2-sulfonyl)-4,5,6,7-tetrahydro-thieno[2,3-c]pyridin,
2-(3-Pyrrolidin-1-yl-propoxy)-6-(toluol-3-sulfonyl)-4,5,6,7-tetrahydro-thieno[2,3-c]pyiidin,
6-(4-Ethyl-benzolsulfonyl)-2-(3-pyrrolidin-1-yl-propoxy)-4,5,6,7-tetrahydro-thieno[2,3-c]pyridin,
6-(4-Isopropyl-benzolsulfonyl)-2-(3-pyrrolidin-1-yl-propoxy)-4,5,6,7-tetrahydro-thieno[2,3-c]pyridin,
6-(4-tert-Butyl-benzolsulfonyl)-2-(3-pyrrolidin-1-yl-propoxy)-4,5,6,7-tetrahydro-thieno[2,3-c]pyridin,
6-(3-Fluor-benzolsulfonyl)-2-(3-pyrrolidin-1-yl-propoxy)-4,5,6,7-tetrahydro-thieno[2,3-c]-pyridin,
6-(Propan-2-sulfonyl)-2-(3-pyrrolidin-1-yl-propoxy)-4,5,6,7-tetrahydro-thieno[2,3-c]pyridin,
[2-(1-Isobutyl-piperidin-4-yloxy)-4,7-dihydra-5*H*-thieno[2,3-c]pyridin-6-yl]-phenyl-methanon,
1-[2-(1-Isobutyl-piperidin-4-yloxy)-4,7-dihydro-5*H*-thieno[2,3-c]pyridin-6-yl]-butan-1-on,
1-[2-(1-Isobutyl-piperidin-4-yloxy)-4,7-dihydro-5*H*-thieno[2,3-c]pyridin-6-yl]-pentan-1-on,
1-[2-(1-Isobutyl-piperidin-d-ylaxy)-4,7-dihydro-5*H*-thieno[2,3-c]pyridin-6-yl]-3,3-dimethylbutan-1-on,
[2-(1-Isobutyl-piperidin-4-yloxy)-4,7-dihydro-5*H*-thieno[2,3-c]pyridin-6-yl]-(3-methoxyphenyl)-methanon,
[2-(1-Isobutyl-piperidin-4-yloxy)-4,7-dihydro-5*H*-thieno[2,3-c]pyridin-6-yl]-(4-methoxyphenyl)-methanon,
(4-Fluor-phenyl)-[2-(1-isobutyl-piperidin-4-yloxy)-4,7-dihydro-5*H*-thieno[2,3-c]pyridin-6-yl]-methanon,
(3-Fluor-phenyl)-[2-(1-isobutyl-piperidin-4-yloxy)-4,7-dihydro-5H-thieno[2,3-c]pyridin-6-yl]-methanon,
[2-(1-Isobutyl-piperidin-4-yloxy)-4,7-dihydro-5*H*-thieno[2,3-c]pyridin-6-yl]-(4-trifluormethyl-phenyl)-methanon,
(3-Chlor-phenyl)-[2-(1-isobutyl-piperidin-4-yloxy)-4,7-dihydro-5*H*-thieno[2,3-c]pyridin-6-yl]-methanon,
(2-Chlor-phenyl)-[2-(1-isobutyl-piperidin-4-yloxy)-4,7-dihydro-5*H*-thieno[2,3-c]pyridin-6-yl]-methanon,
[2-(1-Isobutyl-piperidin-4-yloxy)-4,7-dihydio-5*H*-thieno[2,3-c]pyridin-6-yl]-morpholin-4-yl-methanon,
2-(1-Isobutyl-piperidin-4-yloxy)-4,7-dihydro-5*H*-thieno[2,3-c]pyridin-6-carbonsäure-diethylamid,
[2-(1-Isobutyl-piperidin-4-yloxy)-4,7-dihydro-5*H*-thieno[2,3-c]pyridin-6-yl]-piperidin-1-yl-methanon,
[2-(1-Isobutyl-piperidin-4-yloxy)-4,7-dihydro-5*H*-thieno[2,3-c]pyridin-6-yl]-pyrrolidin-1-yl-methanon,
2-(1-Isobutyl-piperidin-4-yloxy)-6-methansulfonyl-4,5,6,7-tetrahydro-thieno[2,3-c]pyridin, 6-Benzolsu]fonyl-2-(1-isobutyl-piperidm-4-yloxy)-4,5,6,7-tetrahydro-thieno[2,3-c]pyridin,
2-(1-Isobutyl-piperidin-4-yloxy)-6-(toluol-4-sufonyl)-4,5,6,7-tetrahydro-thieno[2,3-c]pyridin,
2-(1-Isobutyl-piperidin-4-yloxy)-6-(toluol-2-sulfonyl)-4,5,6,7-tetrahydro-thieno[2,3-c]pyridin,
2-(1-Isobutyl-piperidin-4-yloxy)-6-(toluol-3-sulfonyl)-4,5,6,7-tetrahydro-thieno[2,3-c]pyridin,
6-(4-Ethyl-benzolsulfonyl)-2-(1-isobutyl-piperidin-4-yloxy)-4,5,6,7-tetrahydro-thieno[2,3-c]pyridin,
6-(3-Fluor-benzolsulfonyl)-2-(1-isobutyl-piperidin-4-yloxy)-4,5,6,7-tetrahydro-thieno[2,3-c]-pyridin,
1-[2-(1-Isopropyl-piperidin-4-yloxy)-4,7-dihydro-5*H*-thieno[2,3-c]pyridin-6-yl]-butan-1-on,
1-[2-(1-Isopropyl-piperidin-4-yloxy)-4,7-dihydro-5*H*-thieno[2,3-c]pyridin-6-yl]-3-methylbutan-1-on,
1-[2-(1-Isopropyl-piperidin-4-yloxy)-4,7-dihydro-5*H*-thieno[2,3-c]pyridin-6-yl]-3,3-dimethyl-butan-1-on,
[2-(1-Isopropyl-piperidin-4-yloxy)-4,7-dihydro-5*H*-thieno[2,3-c]pyridin-6-yl]-(4-trifluor methyl-phenyl)-methanon,
(3-Chlor-phenyl)-[2-(1-isopropyl-piperidin-4-yloxy)-4,7-dihydro-5*H*-thieno[2,3-c]pyridin-6-yl]-methanon,
[2-(1-Isopropyl-piperidin-4-yloxy)-4,7-dihydro-5*H*-thieno[2,3-c]pyridin-6-yl]-(3-methoxyphenyl)-methanon,
[2-(1-Isopropyl-piperidin-4-yloxy)-4,7-dihydro-5*H*-thieno[2,3-c]pyridin-6-yl]-(4-methoxyphenyl)-methanon,
(4-Fluor-phenyl)-[2-(1-isopropyl-piperidin-4-yloxy)-4,7-dihydro-5*H*-thieno[2,3-c]pyridin-6-yl]-methanon,
(3-Fluor-phenyl)-[2-(1-isopropyl-piperidin-4-yloxy)-4,7-dihydro-5*H*-thieno[2,3-c]pyridin-6-yl]-methanon,
(2-Chlor-phenyl)-[2-(1-isopropyl-piperidin-4-yloxy)-4,7-dihydro-5*H*-thieno[2,3-c]pyridin-6-yl]-methanon,
[2-(1-Isopropyl-piperidin-4-yloxy)-4,7-dihydro-5*H*-thieno[2,3-c]pyridin-6-yl]-(2-methoxyphenyl)-methanon,
(2-Fluor-phenyl)-[2-(1-isopropyl-piperidin-4-yloxy)-4,7-dihydro-5*H*-thieno[2,3-c]pyridin-6-yl]-methanon,
[2-(1-Isopiopyl-piperidin-4-yloxy)-4,7-dihydro-5*H*-thieno[2,3-c]pyridin-6-yl]-moipholin-4-yl-methanon,
2-(1-Isopiopyl-piperidin-4-yloxy)-4,7-dihydro-5*H*-thieno[2,3-c]pyridin-6-carbonsäurediethyl-amid,
[2-(1-Isopropyl-piperidin-4-yloxy)-4,7-dihydro-5*H*-thieno[2,3-c]pyridin-6-yl]-piperidin-1-yl-methanon,
2-(1-Isopropyl-piperidin-4-yloxy)-4,7-dihydro-5*H*-thieno[2,3-c]pyridin-6-carbonsäuremethyl-amid,
[2-(1-Isopropyl-piperidin-4-yloxy)-4,7-dihydro-5*H*-thieno[2,3-c]pyridin-6-yl]-(4-methylpiperazin-1-yl)-methanon,
2-(1-Isopropyl-piperidin-4-yloxy)-4,7-dihydro-5*H*-thieno[2,3-c]pyridin-6-carbonsäureethyl-amid,
2-(1-Isopropyl-piperidin-4-yloxy)-4,7-dihydro-5*H*-thieno[2,3-c]pyridin-6-carbonsäureisopropylamid,
2-(1-Isopropyl-piperidin-4-yloxy)-4,7-dihydro-5*H*-tliieno[2,3-c]pyridin-6-carbonsäurebutylamid,
[2-(1-Isopropyl-piperidin-4-yloxy)-4,7-dihydro-5*H*-thieno[2,3-c]pyridin-6-yl]-(4-o-tolylpiperazin-1-yl)-methanon,
2-(1-Isopropyl-piperidin-4-yloxy)-6-methansulfonyl-4,5,6,7-tetrahydro-thieno[2,3-c]pyridin,
6-Benzolsulfonyl-2-(1-isopropyl-piperidin-4-yloxy)-4,5,6,7-tetrahydro-thieno[2,3-c]pyridin,
2-(1-Isopropyl-piperidin-4-yloxy)-6-(toluol-4-sulfonyl)-4,5,6,7-tetrahydro-thieno[2,3-c]pyridin,
2-(1-Isopropyl-piperidin-4-yloxy)-6-(toluol-2-sulfonyl)-4,5,6,7-tetrahydro-thieno[2,3-c]pyridin,
2-(1-Isopropyl-piperidin-4-yloxy)-6-(toluol-3-sulfonyl)-4,5,6,7-tetrahydro-thieno[2,3-c]pyridin,
6-(4-Ethyl-benzolsulfonyl)-2-(1-isopropyl-piperidin-4-yloxy)-4,5,6,7-tetrahydro-thieno[2,3-c]pyridin,
6-(4-Isopropyl-benzolsulfonyl)-2-(1-isopropyl-piperidin-4-ylaxy)-4,5,6,7-tetrahydro-thieno[2,3-c]pyridin,
6-(4-tert-Butyl-benzolsulfonyl)-2-(1-isopropyl-piperidin-4-yloxy)-4,5,6,7-tetrahydro-thieno[2,3-e]pyridin,
6-(3-Fluor-benzolsulfonyl)-2-(1-isopropyl-piporidin-4-yloxy)-4,5,6,7-tetrahydro-thieno[2,3-c]pyridin,
2-(1-Isopropyl-piperidin-4-yloxy)-6-(propan-2-sulfonyl)-4,5,6,7-tetrahydro-thieno[2,3-c]pyridin,
2-(1-Isopropyl-pyrrolidin-3-yloxy)-6-(toluol-4-sulfonyl)-4,5,6,7-tetrahydro-thieno[2,3-c]pyridin,
6-(4-tert-Butyl-benzolsulfonyl)-2-(1-isopropyl-pyrrolidin-3-yloxy)-4,5,6,7-tetrahydro-thieno[2,3-c]pyridin,
6-(4-Isopropyl-benzolsulfonyl)-2-(1-isopropyl-pyrrolidin-3-yloxy)-4,5,6,7-tetrahydro-thieno[2,3-c]pyridin,
(4-Chlor-phenyl)-[2-(3-morpholin-4-yl-propoxy)-4,7-dihydro-5*H*-thieno[2,3-c]pyridin-6-yl]-methanon,
(3-Chlor-phenyl)-[2-(3-morpholin-4-yl-propoxy)-4,7-dihydro-5*H*-thieno[2,3-c]pyridin-6-yl]-methanon,
Dimethyl-{2-[6-(toluol-2-sulfonyl)-4,5,6,7-tetrahydro-thieno[2,3-c]pyridin-2-yloxy]-ethyl}-amin,
(2-Chlor-phenyl)-[2-(2-dimethylamino-ethoxy)-4,7-dihydro-5*H*-thieno[2,3-c]pyridin-6-yl]-methanon,
{2-[6-(4-Isopropyl-benzolsulfonyl)-4,5,6,7-tetrahydro-thieno[2,3-c]pyridin-2-yloxy]-ethyl}-dimethyl-amin
und pharmazeutisch akzeptablen Salzen davon.

20. Verbindungen der Formel I nach Anspruch 1, ausgewählt aus der Gruppe, bestehend aus
3-Metliyl-1-[2-(3-piperidin-1-yl-propoxy)-4,7-dihydro-5*H*-thieno[2,3-c]pyridin-6-yl]-butan-1-on,
3,3-Dimethyl-1-[2-(3-piperidin-1-yl-propoxy)-4,7-dihydro-5*H*-thieno[2,3-c]pyridin-6-yl]-butan-1-on,
6-Benzolsulfonyl-2-(3-pyrrolidin-1-yl-propoxy)-4,5,6,7-tetrahydro-thieno[2,3-c]pyridin,
1-[2-(1-Isopropyl-piperidin-4-yloxy)-4,7-dihydro-5H-thieno[2,3-c]pyridin-6-yl]-butan-1-on,
1-[2-(1-Isopropyl-piperidin-4-yloxy)-4,7-dihydro-5H-thieno[2,3-c]pyridin-6-yl]-3-methylbutan-1-on,
1-[2-(1-Isopropyl-piperidin-4-yloxy)-4,7-dihydro-5*H*-thieno[2,3-c]pyridin-6-yl]-3,3-dimethyl-butan-1-on,
(3-Chlor-phenyl)-[2-(1-isopropyl-piperidin-4-yloxy)-4,7-dihydro-5*H*-thieno[2,3-c]pyridin-6-yl]-methanon,
[2-(1-Isopropyl-piperidin-4-yloxy)-4,7-dihydro-5*H*-thieno[2,3-c]pyridin-6-yl]-(3-methoxyphenyl)-methanon,
(4-Fluol-phenyl)-[2-(1-isopropyl-piperidin-4-yloxy)-4,7-dihydro-5*H*-thieno[2,3-c]pyridin-6-yl]-methanon,
(3-Fluor-phenyl)-[2-(1-isopropyl-piperidin-4-yloxy)-4,7-dihydro-5*H-*thieno[2,3-c]pyridin-6-yl]-methanon,
(2-Chlor-phenyl)-[2-(1-isopropyl-piperidin-4-yloxy)-4,7-dihydro-5*H*-thieno[2,3-c]pyridin-6-yl]-methanon,
(2-Fluor-phenyl)-[2-(1-isopropyl-piperidin-4-yloxy)-4,7-dihydro-5*H*-thieno[2,3-c]pyridin-6-yl]-methanon,
[2-(1-Isopropyl-piperidin-4-yloxy)-4,7-dihydro-5*H*-thieno[2,3-c]pyridin-6-yl]-morpholin-4-yl-methanon,
[2-(1-Isopropyl-piperidin-4-yloxy)-4,7-dihydro-5*H*-thieno[2,3-c]pyridin-6-yl]-piperidin-1-yl-methanon,
[2-(1-Isopropyl-piperidin-4-yloxy)-4,7-dihydro-5*H*-thieno[2,3-c]pyridin-6-yl]-(4-methylpiperazin-1-yl)-methanon,
2-(1-Isopropyl-piperidin-4-yloxy)-6-(toluol-2-sulfonyl)-4,5,6,7-tetrahydro-thieno[2,3-c]pyridin,
6-(3-Fluor-benzolsulfonyl)-2-(1-isopropyl-piperidin-4-yloxy)-4,5,6,7-tetrahydro-thieno[2,3-c]pyridin
und pharmazeutisch akzeptablen Salzen davon.

21. Verfahren zur Herstellung von Verbindungen nach einem der Ansprüche 1 bis 20, wobei das Verfahren
das Verknüpfen eines Amins der Formel II 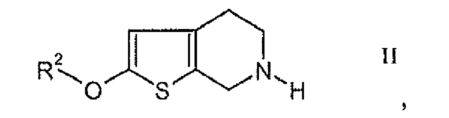 wobei R² wie in Anspruch 1 definiert ist, mit einem Halogenid der Formel III
R¹-X III
wobei R¹ wie in Anspruch 1 definiert ist und X Halogen ist,
in Gegenwart einer Base unter Erhalt einer Verbindung der Formel I 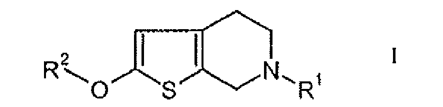 und, wenn gewünscht,
das Umwandeln der erhaltenen Verbindung in ein pharmazeutisch akzeptables Salz umfasst.

22. Pharmazeutische Zusammensetzungen, umfassend eine Verbindung nach einem der Ansprüche 1 bis 20 sowie einen pharmazeutisch akzeptablen Träger und/oder ein pharmazeutisch akzeptables Adjuvans.

23. Pharmazeutische Zusammensetzungen nach Anspruch 22 zur Behandlung und/oder Vorbeugung von Erkrankungen, die mit der Modulation von H3-Rezeptoren in Verbindung stehen.

24. Verbindungen nach einem der Ansprüche 1 bis 20 zur Verwendung als therapeutisch aktive Substanzen.

25. Verbindungen nach einem der Ansprüche 1 bis 20 zur Verwendung als therapeutisch aktive Substanzen für die Behandlung und/oder Vorbeugung von Erkrankungen, die mit der Modulation von H3-Rezeptoren in Verbindung stehen.

26. Verwendung von Verbindungen nach einem der Ansprüche 1 bis 20 zur Herstellung von Medikamenten für die Behandlung und/oder Vorbeugung von Erkrankungen, die mit der Modulation von H3-Rezeptoren in Verbindung stehen.

27. Verwendung nach Anspruch 26 zur Behandlung und/oder Vorbeugung von Fettleibigkeit.

## Revendications

1. Composés de formule générale 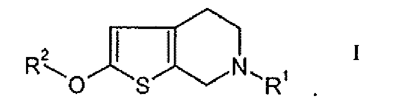 dans laquelle
R¹ est choisi dans le groupe consistant en
un groupe -CO-R³, dans lequel
R³ est choisi dans le groupe consistant en des groupes
alkyle en C₁ à C₇,
(cycloalkyle en C₃ à C₇) (alkyle en C₁ à C₇), dans lequel le noyau cycloalkyle peut être non substitué ou substitué avec un ou deux groupes choisis indépendamment parmi des groupes alkyle en C₁ à C₇, et
phényle, le noyau phényle pouvant être non substitué ou substitué avec un ou deux groupes choisis indépendamment entre des groupes alkyle en C₁ à C₇, cyano, halogéno, halogénoalkyle en C₁ à C₇ et alkoxy en C₁ à C₇,
un groupe -CO-NR⁴R⁵, dans lequel
R⁴ et R⁵, indépendamment l'un de l'autre, sont choisis dans le groupe consistant en un atome d'hydrogène, des groupes alkyle en C₁ à C₇, (alkoxy en C₁ à C₇)(alkyle en C₁ à C₇), phényle non substitué ou substitué avec un ou deux groupes choisis indépendamment entre des groupes alkyle en C₁ à C₇, halogéno, alkoxy en C₁ à C₇ et halogénoalkyle en C₁ à C₇, et phényl(alkyle en C₁ à C₇), dans lequel le groupe phényle est non substitué ou substitué avec un ou deux groupes choisis indépendamment entre des groupes alkyle en C₁ à C₇, halogéno, alkoxy en C₁ à C₇ et halogéno- alkyle en C₁ à C₇,
ou bien R⁴ et R⁵, conjointement avec l'atome d'azote auquel ils sont fixés, forment un noyau hétérocyclique tétra-, penta-, hexa- ou heptagonal, contenant facultativement un hétéroatome supplémentaire choisi entre l'azote, l'oxygène ou le soufre, un groupe sulfinyle ou un groupe sulfonyle, ledit noyau hétéro- acyclique étant non substitué ou substitué avec un ou deux groupes choisis indépendamment entre des groupes alkyle en C₁ à C₇, halogéno, cyano, alkoxy en C₁ à C₇ et phényle, le groupe phényle étant non substitué ou substitué avec un ou deux groupes choisis indépendamment entre des groupes alkyle en C₁ à C₇, halogéno et alkoxy en C₁ à C₇, et
un groupe -SO₂-R⁶, dans lequel
R⁶ est choisi dans le groupe consistant en des groupes
alkyle en C₁ à C₇, (alkoxy en C₁ à C₇) (alkyle en C₁ à C₇),
cycloalkyl(alkyle en C₁ à C₇), dans lequel le noyau cycloalkyle peut être non substitué ou substitué avec un ou deux groupes choisis indépendamment entre des groupes alkyle en C₁ à C₇, halogéno et alkoxy en C₁ à C₇, et
phényle, ledit noyau phényle pouvant être non substitué ou substitué avec un ou deux groupes choisis indépendamment entre des groupes alkyle en C₁ à C₇, cyano, halogéno, halogénoalkyle en C₁ à C₇ et alkoxy en C₁ à C₇ ;
R² représente un groupe choisi entre des groupes 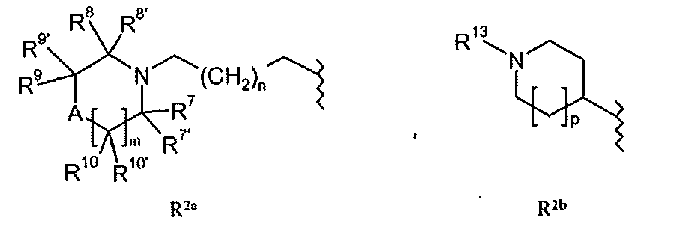 et 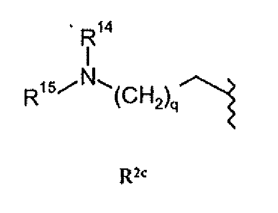
dans lesquels
m est égal à 0, 1 ou 2 ;
n est égal à 0, 1 ou 2 ;
A est choisi entre des groupes -CR¹¹R^{11'}, O, S ou -NR¹² ;
R⁷, R⁷', R⁸, R^{8'}, R⁹, R^{9'}, R¹⁰, R^{10'}, R¹¹ et R^{11'}, indépendamment les uns des autres, représentent un atome d'hydrogène ou un groupe alkyle en C₁ à C₇ ;
R¹² représente un atome d'hydrogène ou un groupe alkyle en C₁ à C₇ ;
p est égal à 0, 1 ou 2 ;
R¹³ représente un groupe alkyle en C₁ à C₇ ou cycloalkyle en C₃ à C₇ ;
q est égal à 1, 2 ou 3 ; et
R¹⁴ et R¹⁵, indépendamment l'un de l'autre, représentent un groupe alkyle en C₁ à C₇ ;
et leurs sels pharmaceutiquement acceptables.

2. Composés de formule I suivant la revendication 1, dans lesquels R¹ représente un groupe -CO-R³ et dans lesquels R³ est choisi dans le groupe consistant en des groupes
alkyle en C₁ à C₇,
(cycloalkyle en C₃ à C₇) (alkyle en C₃ à C₇), dans lequel le noyau cycloalkyle peut être non substitué ou substitué avec un ou deux groupes choisis indépendamment parmi des groupes alkyle en C₃ à C₇, et
phényle, le noyau phényle pouvant être non substitué ou substitué avec un ou deux groupes choisis indépendamment entre des groupes alkyle en C₁ à C₇, cyano, halogéno, halogénoalkyle en C₁ à C₇ et alkoxy en C₁ à C₇.

3. Composés de formule I suivant la revendication 1 ou 2, dans lesquels R¹ représente un groupe -CO-R³ et dans lesquels R³ représente un groupe alkyle en C₁ à C₇ ou phényle, le noyau phényle pouvant être non substitué ou substitué avec un ou deux groupes choisis indépendamment entre des groupes alkyle en C₁ à C₇, cyano, halogéno, halogénoalkyle en C₁ à C₇ et alkoxy en C₁ à C₇.

4. Composés de formule I suivant l'une quelconque des revendications 1 à 3, dans lesquels R¹ représente un groupe -CO-R³ et dans lesquels R³ représente un groupe alkyle en C₁ à C₇.

5. Composés de formule I suivant l'une quelconque des revendications 1 à 3, dans lesquels R¹ représente un groupe -CO-R³ et dans lesquels R³ représente un groupe phényle, le noyau phényle pouvant être non substitué ou substitué avec un ou deux groupes choisis indépendamment entre des groupes alkyle en C₁ à C₇, cyano, halogéno, halogénoalkyle en C₁ à C₇ et alkoxy en C₁ à C₇.

6. Composés de formule I suivant l'une quelconque des revendications 1 à 3 et 5, dans lesquels R¹ représente un groupe -CO-R³ et dans lesquels R³ représente un groupe phényle substitué avec un ou deux groupes choisis indépendamment entre des groupes halogéno, halogénoalkyle en C₁ à C₇ et alkoxy en C₁ à C₇.

7. Composés de formule I suivant la revendication 1, dans lesquels R¹ représente un groupe -CO-NR⁴R⁵ et dans lesquels R⁴ et R⁵, indépendamment l'un de l'autre, sont choisis dans le groupe consistant en
un atome d'hydrogène, des groupes alkyle en C₁ à C₇, (alkoxy, en C₁ à C₇) (alkyle en C₁ à C₇)
phényle non substitué ou substitué avec un ou deux groupes choisis indépendamment entre des groupes alkyle en Ci à C₇, halogéno, alkoxy en C₁ à C₇ et halogénoalkyle en C₁ à C₇, et phényl(alkyle en C₁ à C₇), dans lequel le groupe phényle est non substitué ou substitué avec un ou deux groupes choisis indépendamment entre des groupes alkyle en C₁ à C₇, halogéno, alkoxy en C₁ à C₇ et halogénoalkyle en C₁ à C₇,
ou bien R⁴ et R⁵, conjointement avec l'atome d'azote auquel ils sont fixés, forment un noyau hétérocyclique tétra-, penta-, hexa- ou heptagonal contenant facultativement un hétéroatome supplémentaire choisi entre l'azote, l'oxygène et le soufre, un groupe sulfinyle ou un groupe sulfonyle, ledit noyau hétérocyclique étant non substitué ou substitué avec un ou deux groupes choisis indépendamment entre des groupes alkyle inférieur, halogéno, cyano, alkoxy inférieur et phényle, le groupe phényle étant non substitué ou substitué avec un ou deux groupes choisis indépendamment entre des groupes alkyle en C₁ à C₇, halogéno et alkoxy en C₁ à C₇.

8. Composés de formule I suivant la revendication 1 ou 7, dans lesquels R¹ représente un groupe -CO-NR⁴R⁵ et dans lesquels R⁴ et R⁵, indépendamment l'un de l'autre, représentent un atome d'hydrogène ou un groupe alkyle en C₁ à C₇.

9. Composés de formule I suivant la revendication 1 ou 7, dans lesquels R¹ représente un groupe -CO-NR⁴R⁵ et dans lesquels R⁴ et R⁵, conjointement avec l'atome d'azote auquel ils sont fixés, forment un noyau hétérocyclique tétra-, penta, hexa- ou heptagonal contenant facultativement un hétéroatome supplémentaire choisi entre l'azote, l'oxygène et le soufre, un groupe sulfinyle ou un groupe sulfonyle, ledit noyau hétérocyclique étant non substitué ou substitué avec un ou deux groupes choisis indépendamment entre des groupes alkyle en C₁ à C₇, halogéno, cyano, alkoxy en C₁ à C₇ et phényle, le groupe phényle étant non substitué ou substitué avec un ou deux groupes choisis indépendamment entre des groupes alkyle en C₁ à C₇, halogéno et alkoxy en C₁ à C₇.

10. Composés de formule I suivant l'une quelconque des revendications 1, 7 et 9 dans lesquels R¹ représente un groupe -CO-NR⁴R⁵ et dans lesquels R⁴ et R⁵, conjointement avec l'atome d'azote auquel ils sont fixés, forment un noyau hétérocyclique choisi dans le groupe consistant en les noyaux pyrrolidinyle, pipéridinyle, pipérazinyle et morpholinyle, ledit noyau hétérocyclique étant non substitué ou substitué avec un ou deux groupes choisis indépendamment entre des groupes alkyle en C₁ à C₇, halogéno, cyano, alkoxy en C₁ à C₇ et phényle, le groupe phényle étant non substitué ou substitué avec un ou deux groupes choisis indépendamment entre des groupes alkyle en C₁ à C₇, halogéno et alkoxy en C₁ à C₇.

11. Composés de formule I suivant la revendication 1, dans lesquels R¹ représente un groupe -SO₂-R⁶ et dans lesquels R⁶ est choisi dans le groupe consistant en des groupes alkyle en C₁ à C₇, (alkoxy en C₁ à C₇) (alkyle en C₁ à C₇),
(cycloalkyle en C₃ à C₇) (alkyle en C₁ à C₇), dans lequel le noyau cycloalkyle peut être non substitué ou substitué avec un ou deux groupes choisis indépendamment entre des groupes alkyle en C₁ à C₇, halogéno et alkoxy en C₁ à C₇, et
phényle, le noyau phényle pouvant être non substitué ou substitué avec un ou deux groupes choisis indépendamment entre des groupes alkyle en C₁ à C₇, cyano, halogéno, halogénoalkyle en C₁à C₇ et alkoxy en C₁ à C₇.

12. Composés de formule 1 suivant la revendication 1 ou 11, dans lesquels R¹ représente un groupe -SO₂-R⁶ et dans lesquels R⁶ représente un groupe alkyle en C₁ à C₇ ou phényle, le noyau phényle pouvant être non substitué ou substitué avec un ou deux groupes choisis entre des groupes alkyle en C₁ à C₇ et halogéno.

13. Composés de formule 1 suivant l'une quelconque des revendications 1 à 12, dans lesquels R² représente un groupe 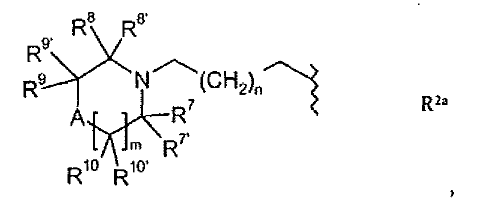 dans lequel
m est égal à 0 ou 1 ;
n est égal à 1 ;
A est choisi entre des groupes -CR¹¹R^{11'}-, O, S ou -NR¹² ;
R⁷, R^{7'}, R⁸, R^{8'}, R⁹, R^{9'}, R¹⁰, R^{10'}, R¹¹ et R^{11'}, indépendamment les uns des autres, représentent un atome d'hydrogène ou un groupe alkyle en C₁ à C₇ ; et
R¹² représente un atome d'hydrogène ou un groupe alkyle en C₁ à C₇.

14. Composés de formule I suivant l'une quelconque des revendications 1 à 13, dans lesquels A représente un groupe CR¹¹R^{11'} et R⁷, R^{7'}, R⁸, R^{8'}, R⁹, R^{9'}, R¹⁰, R^{10'}, R¹¹ et R^{11'}, indépendamment les uns des autres, représentent un atome d'hydrogène.

15. Composés de formule I suivant l'une quelconque des revendications 1 à 13, dans lesquels A représente un groupe O et R⁷, R^{7'}, R⁸, R^{8'}, R⁹, R^{9'}, R¹⁰ et R^{10'} représentent des atomes d'hydrogène.

16. Composés de formule I suivant l'une quelconque des revendications 1 à 12, dans lesquels R² représente un groupe 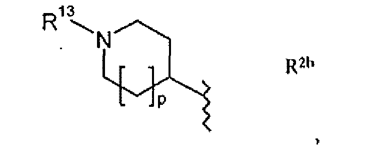 dans lequel p est égal à 0 ou 1 ; et R¹³ représente un groupe alkyle en C₁ à C₇.

17. Composés de formule I suivant l'une quelconque des revendications 1 à 12 et 16, dans lesquels R¹³ représente un groupe isopropyle ou isobutyle.

18. Composés de formule I suivant l'une quelconque des revendications 1 à 12, dans lesquels R² représente un groupe 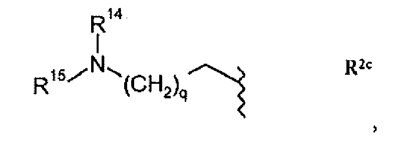 dans lequel q est égal à 1, 2 ou 3 ; et R¹⁴ et R¹⁵, indépendamment l'un de l'autre, représentent un groupe alkyle en C₁ à C₇.

19. Composés de formule I suivant la revendication 1, choisis dans le groupe consistant en
la morpholine-4-yl-[2-(3-pipéridine-1-yl-propoxy)-4,7-dihydro-5*H*-thiéno[*2,3-c*]pyridine-6-yl]-méthanone,
la pipéridine-1-yl-[2-(3-pipéridine-1-yl-propoxy)-4,7-dihydro-5*H*-thiéno[*2,3-c*]pyridine-6-y]-méthanone,
la (4-méthyl-pipérazine-1-yl)-[2-(3-pipéridine-1-yl-propoxy)-4,7-dihydro-5*H*-thiéno[*2,3-c*]pyridine-6-yl]-méthanone,
la [2-(3-pipéridine-1-yl-propoxy)-4,7-dihydro-5*H*-thiéno-[2,3-*c*]pyridine-6-yl]-pyrrolidine-l-yl-méthanone,
la (4-méthoxy-pipéridine-1-yl)-[2-(3-pipéridine-1-yl-propoxy)-4,7-dihydro-5*H*-thiéno[*2,3-c*]pyridine-6-yl]-méthanone,
la [2-(3-pipéridine-1-yl-propoxy)-4,7-dihydro-5*H*-thiéno-[*2,3-c*]pyridine-6-yl]-(4-o-tolyl-pipérazine-1-yl)-méthanone,
la 3-méthyl-1-[2-(3-pipéridine-1-yl-propoxy)-4,7-dihydro-5*H*-thiéno[2,3-*c*]pyridine-6-yl]-butane-1-one,
la 3,3-diméthyl-1-[2-(3-pipéridine-1-yl-propoxy)-4,7-dihydro-5*H*-thiéno[2,3-*c*]pyridine-6-yl]-butane-1-one,
la (4-méthoxy-phényl)-[2-(3-pipéridine-1-yl-propoxy)-4,7-dihydro-5*H*-thiéno[*2,3-c*]pyridine-6-yl]-méthanone,
la (3-fluorophényl)-[2-(3-pipéridine-1-yl-propoxy)-4,7-dihydro-5*H*-thiéno[*2,3-c*]pyridine-6-yl]-méthanone,
la (3-chlorophényl)-[2-(3-pipéridine-1-yl-propoxy)-4,7-dihydro-5*H*-thiéno[*2,3-c*]pyridine-6-yl]-méthanone,
la 5-méthanesulfonyl-2-(3-pipéridine-1-yl-propoxy)-4,5,6,7-tétrahydrothiéno[*2,3-c*]pyridine,
la 6-(3-fluorobenzènesulfonyl)-2-(3-pipéridine-1-yl-prapaxy)-4,5,6,7-tétxahydrothiéno[*2,3-c*]pyridine,
la 2-(3-pipéridine-1-yl-propoxy)-6-(toluène-4-sulfonyl)-4,5,6,7-tétrahydrothiéno[*2,3-c*]pyridine,
la 2-(3-pipéridine-1-yl-propoxy)-6-(toluène-2-sulfonyl)-4,5,6,7-tétrahydrothiéno[*2,3-c*]pyridine,
la 6-(4-éthyl-benzênesulfanyl)-2-(3-pipéridine-1-yl-propoxy)-4,5,6,7-tétrahydrothiéno[*2,3-c*]pyridine,
la 6-(4-tertiobutyl-benzènesulfonyl)-2-(3-pipéridine-2-yl-propoxy)-4,5,6,7-tétrahyàrothiéno[*2,3-c*]pyridine,
la 5-benzènesulfonyl-2-(3-pipéridine-1-yl-propoxy)-4,5,6,7-tétrahydrothiéno[*2,3-c*]pyridine,
la 6-(4-isopropyl-benzènesulfonyl)-2-(3-pipéridine-1-yl-propoxy)-4,5,6,7-tétrahydrothiéno[*2,3-c*]pyridine,
la 3,3-diméthyl-l-[2-(3-pyrrolidine-1-yl-propoxy)-4,7-dihydro-5*H*-thiéno[*2,3-c*]pyridine-6-yl]-butane-1-one,
la (3-chlorophényl)-[2-(3-pyrrolidine-1-yl-propoxy)-4,7-dihydro-5*H*-thiéno[*2,3-c*]pyridine-6-yl]-méthanone,
la (2-chlorophényl)-[2-(3-pyrrolidine-1-yl-propoxy)-4,7-dihydro-5*H*-thiéno[*2,3-c*]pyridine-6-yl]-méthanone,
la [2-(3-pyrrolidine-1-yl-propoxy)-4,7-dihydro-5*H*-thiéno-[*2,3-c*]pyridine-6-yl]-(4-trifluorométhyl-phényl)-méthanone,
la phényl-[2-(3-pyrrolidine-1-yl-propoxy)-4,7-dihydro-5*H*-thiéno[2,3-*c*]pyridine-6-yl]-méthanone,
la (3-méthoxyphényl)-[2-(3-pyrrolidine-1-yl-propoxy)-4,7-dihydro-5*H*-thiéno[*2,3-c*]pyridine-6-yl]-méthanone,
la morpholine-4-yl-[2-(3-pyrrolidine-1-yl-propoxy)-4,7-dihydro-5*H*-thiéno[*2,3-c*]pyridine-6-yl]-méthanone,
la pyrrolidine-1-yl-[2-(3-pyrrolidine-1-yl-propoxy)-4,7-dihydro-5*H*-thiéno[*2,3-c*]pyridine-6-yl]-méthanone,
la pipéridine-1-yl-[2-(3-pyrrolidine-1-yl-propoxy)-4,7-dihydro-5*H*-thiéno[2,3*-c*]pyridine-6-yl]-méthanone,
la (4-méthyl-pipérazine-1-yl)-[2-(3-pyrrolidine-1-yl-propoxy)-4,7-dihydro-5*H*-thiéno[*2,3-c*]pyridine-6-yl]-méthanone,
la 2-(3-pyrrolidine-1-yl-propoxy)-4,7-dihydro-5*H*-thiéno-[*2,3-c*]pyridine-6-yl]-(4-o-tolyl-pipérazine-1-yl)-méthanone,
la 6-méthanesulfonyl-2-(3-pyrrolidine-1-yl-propoxy)-4,5,6,7-tétrahydrothiéno[*2,3-c*]pyridine,
la 6-benzènesulfonyl-2-(3-pyrrolidine-1-yl-propoxy)-4,5,6,7-tétrahydrothiéno[*2,3-c*]pyridine,
la 2-(3-pyrrolidine-1-yl-propoxy)-6-(toluène-4-sulfonyl)-4,5,6,7-tétrahydrothiéno[*2,3-c*]pyridine,
la 2-(3-pyrrolidine-1-yl-propoxy)-6-(toluène-2-sulfonyl)-4,5,6,7-tétrahydrothiéno[*2,3-c*]pyridine,
la 2-(3-pyrrolidine-1-yl-propoxy)-6-(toluène-3-sulfonyl)-4,5,6,7-tétrahydrothiéno[*2,3-c*]pyridine,
la 6-(4-éthyl-benzènesulfonyl)-2-(3-pyrrolidine-1-yl-propoxy)-4,5,6,7-tétrahydrothiéno[*2,3-c*]pyridine,
la 6-(4-isopropyl-benzènesulfonyl)-2-(3-pyrrolidine-1-yl-propoxy)-4,5,6,7-tétrahydrothiéno[*2,3-c*]pyridine,
la 6-(4-tertiobutyl-benzènesulfonyl)-2-(3-pyrrolidine-1-yl-propoxy)-4,5,6,7-tétrahydrothiéno[*2,3-c*]pyridine,
la 6-(3-fluoro-benzènesulfonyl)-2-(3-pyrrolidine-1-yl-propoxy)-4,5,6,7-tétrahydrothiéno[*2,3-c*]pyridine,
la 6-(propane-2-sulfonyl)-2-(3-pyrrolidine-1-yl-propoxy)-4,5,6,7-tétrahydrothiéno[*2,3-c*]pyridine,
la [2-(1-isobutyl-pipéridine-4-yloxy)-4,7-dihydro-5*H-*thiéno[*2,3-c*]pyridine-6-yl]-phényl-méthanone,
la 1-[2-(2-isobutyl-pipéridine-4-yloxy)-4,7-dihydro-5*H-*thiéno[*2,3-c*]pyridine-6-yl]-butane-1-one,
la 1-[2-(1-isobutyl-pipéridine-4-yloxy)-4,7-dihydro-5*H-*thiéno[*2,3-c*]pyridine-6-yl]-pentane-1-one,
la 1-[2-(1-isobutyl-pipéridine-4-yloxy)-4,7-dihydro-5*H-*thiéno[*2,3-c*]pyridine-6-yl]-3,3-diméthyl-butane-1-one,
la [2-(1-isobutyl-pipéridine-4-yloxy)-4,7-dihydro-5*H-*thiéno[*2,3-c*]pyridine-6-yl]-(3-méthoxy-phényl)-méthanone,
la [2-(1-isobutyl-pipéridine-4-yloxy)-4,7-dihydro-5H-thiéno[*2,3-c*]pyridine-6-yl]-(4-méthoxy-phényl)-méthanone,
la (4-fluorophényl)-[2-(1-isobutyl-pipéridine-4-yloxy)-4,7-dihydro-5*H*-thiéno[*2,3-c*]pyridine-6-yl]-méthanone,
la (3-fluorophényl)-[2-(1-isobutyl-pipéridine-4-yloxy)-4,7-dihydro-5H-thiéno[*2,3-c*]pyridine-6-yl]-méthanone,
la [2-(1-isobutyl-pipéridine-4-yloxy)-4,7-dihydro-5*H-*thiéno[*2,3-c*]pyridine-6-yl]-(4-trifluorométhyl-phényl)-méthanone,
la (3-chlorophényl)-[2-(1-isobutyl-pipéridine-4-yloxy)-4,7-dihydro-5*H*-thiéno[*2,3-c*]pyridine-6-yl]-méthanone,
la (2-chlorophényl)-[2-(1-isobutyl-pipéridine-4-yloxy)-4,7-dihydro-5*H*-thiéno[*2,3-c*]pyridine-6-yl]-méthanone,
la [2-(1-isobutyl-pipéridine-4-yloxy)-4,7-dihydro-5*H-*thiéno[*2,3-c*]pyridine-6-yl]-morpholine-4-yl-méthanone,
le diéthylamide d'acide 2-(1-isobutyl-pipéridine-4-yloxy)-4,7-dihydro-5*H*-thiéno[*2,3-c*]pyridine-6-carboxylique,
la [2-(1-isobutyl-pipéridine-4-yloxy)-4,7-dihydro-5*H-*thiéno[*2,3-c*]pyridine-6-yl]-pipéridine-1-yl-méthanone,
la [2-(1-isobutyl-pipéridine-4-yloxy)-4,7-dihydro-5*H-*thiéno[*2,3-c*]pyridine-6-yl]-pyrrolidine-1-yl-méthanone,
la 2-(1-isobutyl-pipéridine-4-yloxy)-6-méthanesulfonyl-4,5,6,7-tétrahydrothiéno[*2,3-c*]pyridine,
la 6-benzènesulfonyl-2-(1-isobutyl-pipéridine-4-yloxy)-4,5,6,7-tétrahydrothiéno[*2,3-c*]pyridine,
la 2-(1-isobutyl-pipéridine-4-yloxy)-6-(toluène-4-sulfonyl)-4,5,6,7-tétrahydrothiéno[*2,3-c*]pyridine,
la 2-(1-isobutyl-pipéridine-4-yloxy)-6-(toluène-2-sulfonyl)-4,5,6,7-tétrahydrothiéno[*2,3-c*]pyridine,
la 2-(1-isobutyl-pipéridine-4-yloxy)-6-(toluène-3-sulfonyl)-4,5,6,7-tétrahydrothiéno[*2,3-c*]pyridine,
la 6-(4-éthyl-benzènesulfonyl)-2-(1-isobutyl-pipéridine-4-yloxy)-4,5,6,7-tétrahydrothiéno[*2,3-c*]pyridine,
la 6-(3-fluoro-benzénesulfonyl)-2-(1-isobutyl-pipéridine-4-yloxy)-4,5,6,7-tétrahydrothiéno[*2,3-c*]pyridine,
la 1-[2-(1-isopropyl-pipéridine-4-yloxy)-4,7-dihydro-5*H*-thiéno[*2,3-c*]pyridine-6-yl]-butane-1-one,
la 1-[2-(1-isopropyl-pipéridine-4-yloxy)-4,7-dihydro-5*H*-thiéno[*2,3-c*]pyridine-6-yl]-3-méthyl-butane-1-one,
la 1-[2-(1-isopropyl-pipéridine-4-yloxy)-4,7-dihydro-5*H*-thiéno[*2,3-c*]pyridine-6-yl]-3,3-diméthyl-butane-1-one,
la [2-(1-isopropyl-pipéridine-4-yloxy)-4,7-dihydro-5*H-*thiéno[2,3-*c*]pyridine-6-yl]-(4-trifluorométhyl-phényl)-méthanone,
la (3-chlorophényl)-[2-(I-isopropyl-pipéridine-4-yloxy)-4,7-dihydro-5*H*-thiéno[*2,3-c*]pyridine-6-yl]-méthanone,
la [2-(1-isopropyl-pipéridine-4-yloxy)-4,7-dihydro-5*H-*thiéno[*2,3-c*]pyridine-6-yl]-(3-méthoxy-phényl)-méthanone,
la [2-(2-isopropyl-pipéridine-4-yloxy)-4,7-dihydro-5*H-*thiéno[*2,3-c*]pyridine-6-yl]-(4-méthoxy-phényl)-méthanone,
la (4-fluorophényl)-[2-(1-isopropyl-pipéridine-4-yloxy)-4,7-dihydro-5*H*-thiéno[*2,3-c*]pyridine-6-yl]-méthanone,
la (3-fluorophényl)-[2-(1-isopropyl-pipéridine-4-yloxy)-4,7-dihydro-5*H*-thiéno[*2,3-c*]pyridine-6-yl]-méthanone,
la (2-chlorophényl)-[2-(1-isopropyl-pipéridine-4-yloxy)-4,7-dihydro-5H-thiéno[*2,3-c*]pyridine-6-yl]-méthanone,
la [2-(1-isopropyl-pipéridine-4-yloxy)-4,7-dihydro-5*H-*thiéno[*2,3-c*]pyridine-6-yl]-(2-méthoxy-phényl)-méthanone,
la (2-fluorophényl)-[2-(1-isopropyl-pipéridine-4-yloxy)-4,7-dihydro-5*H*-thiéno[*2,3-c*]pyridine-6-yl]-méthanone,
la [2-(1-isopropyl-pipéridine-4-yloxy)-4,7-dihydro-5*H-*thiéno[*2,3-c*]pyridine-6-yl]morpholine-4-yl-méthanone,
le diéthylamide d'acide 2-(1-isopropyl-pipéridine-4-yloxy)-4,7-dihydro-5*H*-thiéno[*2,3-c*]pyridine-6-carboxylique,
la [2-(1-isopropyl-pipéridine-4-yloxy)-4,7-dihydro-5*H-*thiéno[*2,3-c*]pyridine-6-yl]-pipéridine-1-yl-méthanone,
le méthylamide d'acide 2-(1-isopropyl-pipéridine-4-yloxy)-4,7-dihydro-5*H*-thiéno[*2,3-c*]pyridine-6-carboxylique,
la [2-(1-isopropyl-pipéridine-4-yloxy)-4,7-dihydro-5*H-*thiéno[*2,3-c*]pyridine-6-yl]-(4-méthyl-pipérazine-1-yl)-méthanone,
l'êthylamide d'acide 2-(1-isopropyl-pipéridine-4-yloxy)-4,7-dihydro-5*H*-thiéno[*2,3-c*]pyridine-6-carboxylique,
l'isopropylamide d'acide 2-(1-isopropyl-pipéridine-4-yloxy)-4,7-dihydro-5*H*-thiéno[*2,3-c*]pyridine-6-carboxylique,
le butylamide d'acide 2-(1-isopropyl-pipéridine-4-yloxy)-4,7-dihydro-5H-thiéno[*2,3-c*]pyridine-6-carboxylique,
la [2-(1-isopropyl-pipéridine-4-yloxy)-4,7-dihydro-5*H-*thiéno[*2,3-c*]pyridine-6-yl]-(4-o-tolyl-pipérazine-1-yl)-méthanone,
la 2-(1-isopropyl-pipéridine-4-yloxy)-6-méthanesulfonyl-4,5,6,7-tétrahydrothiéno[*2,3-c*]pyridine,
la 6-benzènesulfonyl-2-(1-isopropyl-pipéridine-4-yloxy)-4,5,6,7-tétrahydrothiéno[*2,3-c*]pyridine,
la 2-(1-isopropyl-pipéridine-4-yloxy)-6-(toluène-4-sulfonyl)-4,5,6,7-tétrahydrothiéno[*2,3-c*]pyridine,
la 2-(1-isopropyl-pipéridine-4-yloxy)-6-(toluène-2-sulfonyl)-4,5,6,7-tétrahydrothiéno[*2,3-c*]pyridine,
la 2-(1-isopropyl-pipéridine-4-yloxy)-6-(toluène-3-sulfonyl)-4,5,6,7-tétrahydrothiéno[*2,3-c*]pyridine,
la 5-(4-éthyl-benzènesulfonyl)-2-(1-isopropyl-pipéridine-4-yloxy)-4,5,6,7-tétrahydrothiéno[*2,3-c*]pyridine,
la 6-(4-isopropyl-benzènesulfonyl)-2-(1-isopropyl-pipéridine-4-yloxy)-4,5,6,7-tétrahydrothiéno[*2,3-c*]pyridine,
la 6-(4-tertiobutyl-benzènesulfonyl)-2-(1-isopropyl-pipéridine-4-yloxy)-4,5,6,7-tétrahydrothiéno[*2,3-c*]pyridine,
la 6-(3-fluoro-benzènesulfonyl)-2-(1-isopropyl-pipéridine-4-yloxy)-4,5,6,7-tétrahydrothiéno[*2,3-c*]pyridine,
la 2-(1-isopropyl-pipéridine-4-yloxy)-6-(propane-2-sulfonyl)-4,5,6,7-tétrahydrothiéno[*2,3-c*]pyridine,
la 2-(1-isopropyl-pyrrolidine-3-yloxy)-6-(toluène-4-sulfonyl)-4,5,6,7-tétrahydrothiéno[*2,3-c*]pyridine,
la 6-(4-tertiobutyl-benzènesulfonyl)-2-(1-isopropyl-pyrrolidine-3-yloxy)-4,5,6,7-tétrahydrothiéno[*2,3-c*]pyridine,
la 6-(4-isopropyl-benzènesulfonyl)-2-(1-isopropyl-pyrrolidine-3-yloxy)-4,5,6,7-tétrahydrothiéno[*2,3-c*]pyridine,
la (4-chlorophényl)-[2-(3-morpholine-4-yl-propoxy)-4,7-dihydro-5*H*-thiéno[*2,3-c*]pyridine-6-yl]-méthanone,
la (3-chlorophényl)-[2-(3-morpholine-4-yl-propoxy)-4,7-dihydro-5*H*-thiéno[*2,3-c*]pyridine-6-yl]-méthanone,
la diméthyl-{2-[6-(toluène-2-sulfonyl)-4,5,6,7-tétrahydro-thiéno[*2,3-c*]pyridine-2-yloxy]-éthyl}-amine,
la (2-chlorophényl)-[2-(2-diméthylamino-éthoxy)-4,7-dihydro-5*H*-thiéno[*2,3-c*]pyridine-6-yl]-méthanone,
la {2-[6-(4-isopropyl-benzènesulfonyl)-4,5,6,7-tétrahydro-thiéno[2,3-*c*]pyridine-2-yloxy]-éthyl}-diméthylamine,
et leurs sels pharmaceutiquement acceptables.

20. Composés de formule I suivant la revendication 1, choisis dans le groupe consistant en
la 3-méthyl-1-[2-(3-pipéridine-1-yl-propoxy)-4,7-dihydro-5*H*-thiéno[2,3-*c*]pyridine-6-yl]-butane-1-one,
la 3,3-diméthyl-1-[2-(3-pipéridine-1-yl-propoxy)-4,7-dihydro-5*H*-thiéno[*2,3-c*]pyridine-6-yl]-butane-1-one,
la 6-benzènesulfonyl-2-(3-pyrrolidine-1-yl-propoxy)-4,5,6,7-tétrahydrothiéno[*2,3-c*]pyridine,
la 1-[2-(1-isopropyl-pipéridine-4-yloxy)-4,7-dihydro-5*H*-thiéno[*2,3-c*]pyridine-6-yl]-butane-1-one,
la 1-[2-(1-isopropyl-pipéridine-4-yloxy)-4,7-dihydro-5*H*-thiéno[*2,3-c*]pyridine-6-yl]-3-méthyl-butane-1-one,
la 1-[2-(1-isopropyl-pipéridine-4-yloxy)-4,7-dihydro-5*H*-thiéno[*2,3-c*]pyridine-6-yl]-3,3-diméthyl-butane-1-one,
la (3-chlorophényl)-[2-(1-isopropyl-pipéridine-4-yloxy)-4,7-dihydro-5*H*-thiéno[*2,3-c*]pyridine-6-yl]-méthanone,
la [2-(1-isopropyl-pipéridine-4-yloxy)-4,7-dihydro-5*H-*thiéno[*2,3-c*]pyridine-6-yl]-(3-méthoxy-phényl)-méthanone,
la (4-fluorophényl)-[2-(1-isopropyl-pipéridine-4-yloxy)-4,7-dihydro-5H-thiéno[*2,3-c*]pyridine-6-yl]-méthanone,
la (3-fluorophényl)-[2-(1-isopropyl-pipéridine-4-yloxy)-4,7-dihydro-5*H*-thiéno[*2,3-c*]pyridine-6-yl]-méthanone,
la (2-chlorophényl)-[2-(1-isopropyl-pipéridine-4-yloxy-4,7-dihydro-5*H*-thiéno[*2,3-c*]pyridine-6-yl]-méthanone,
la (2-fluorophényl)-[2-(1-isopropyl-pipéridine-4-yloxy)-4,7-dihydro-5*H*-thiéno[*2,3-c*]pyridine-6-yl]-méthanone,
la [2-(1-isopropyl-pipéridine-4-yloxy)-4,7-dihydro-5*H-*thiéno[*2,3-c*]pyridine-6-y]-morpholine-4-yl-méthanone,
la [2-(1-isopropyl-pipéridine-4-yloxy)-4,7-dihydro-5*H-*thiéno[*2,3-c*]pyridine-6-yl]-pipéridine-1-yl-méthanone,
la [2-(1-isopropyl-pipéridine-4-yloxy)-4,7-dihydro-5*H-*thiéno[*2,3-c*]pyridine-6-yl]-(4-méthyl-pipérazine-1-yl)-méthanone,
la 2-(1-isopropyl-pipéridine-4-yloxy)-6-(toluène-2-sulfonyl)-4,5,6,7-tétrahydrothiéno[*2,3-c*]pyridine,
la 6-(3-fluoro-benzènesulfonyl)-2-(1-isopropyl-pipéridine-4-yloxy)-4,5,6,7-tétrahydrothiéno[2,3-*c*]pyridine,
et leurs sels pharmaceutiquement acceptables.

21. Procédé pour la production de composés suivant l'une quelconque des revendications 1 à 20, procédé qui comprend
le couplage d'une amine de formule II 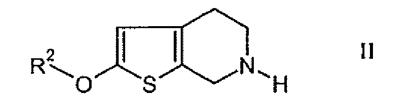 dans laquelle R² est tel que défini dans la revendication 1, avec un halogénure de formule III
R¹-X III
dans laquelle R¹ est tel que défini dans la revendication 1 et X représente un atome d'halogène,
en présence d'une base, pour obtenir un composé de formule I 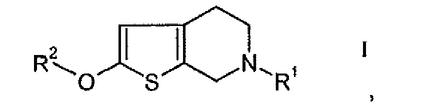 et, si cela est désiré
la conversion du composé obtenu en un sel pharmaceutiquement acceptable.

22. Compositions pharmaceutiques comprenant un composé suivant l'une quelconque des revendications 1 à 20 ainsi qu'un support et/ou adjuvant pharmaceutiquement acceptable.

23. Compositions pharmaceutiques suivant la revendication 22, pour le traitement et/ou la prévention de maladies qui sont associées à la modulation des récepteurs H3.

24. Composés suivant l'une quelconque des revendications 1 à 20, destinés à être utilisés comme substances thérapeutiquement actives.

25. Composés suivant l'une quelconque des revendications 1 à 20, destinés à être utilisés comme substances thérapeutiquement actives pour le traitement et/ou la prévention de maladies qui sont associées à la modulation des récepteurs H3.

26. Utilisation de composés suivant l'une quelconque des revendications 1 à 20, pour la préparation de médicaments destinés au traitement et/ou à la prévention de maladies qui sont associées à la modulation des récepteurs H3.

27. Utilisation suivant la revendication 26, pour le traitement et/ou la prévention de l'obésité.
